# FASCICULE DE BREVET EUROPEEN

(11) **EP 3 240 789 B1**
(45) Date de publication et mention de la délivrance du brevet: **29.07.2020**
(21) Numéro de dépôt: 15820182.2
(22) Date de dépôt: 30.12.2015
(51) Int. Cl.: C07D 487/04, A61K 31/4985, A61P 35/00

(54) **NOUVEAUX IMIDAZO[1,2-A]QUINOXALINES ET DÉRIVÉS POUR LE TRAITEMENT DES CANCERS**
NEUE IMIDAZO[1,2-A]CHINOXALINE UND DERIVATE DAVON ZUR BEHANDLUNG VON KREBS
NEW IMIDAZO[1,2-A]QUINOXALINES AND DERIVATES THEREOF FOR THE TREATMENT OF CANCER

(30) Priorité: 31.12.2014 FR 1463480
(43) Date de publication de la demande: 08.11.2017
(73) Titulaire: Université de Montpellier, 34090 Montpellier (FR); Centre National de la Recherche Scientifique (C.N.R.S.), 75016 Paris (FR); AxLR, SATT du Languedoc Roussillon, 34095 Montpellier (FR)
(72) Inventeur: DELEUZE-MASQUEFA, Carine, 34430 Saint Jean De Vedas (FR); BONNET, Pierre-Antoine, 34080 Montpellier (FR); CUQ, Pierre, 34970 Maurin (FR); PATINOTE, Cindy, 34090 Montpellier (FR)
(74) Mandataire: Regimbeau
(86) Numéro de dépôt international: PCT/EP2015/081397
(87) Numéro de publication internationale: WO 2016/107895

(56) Documents cités:
- WO-A1-2010/124826
- FR-A1- 2 921 927

## Description

La présente invention concerne des composés imidazo[1,2-*a*]quinoxaline tels que définis dans les revendications pour le traitement des cancers ainsi que les compositions pharmaceutiques comprenant ces composés et leurs utilisations en thérapie. L'invention a également pour objet l'utilisation de composés dérivés d'imidazo[1,2-*a*]quinoxaline tels que définis dans les revendications pour la préparation de médicaments pour le traitement des cancers et en particulier pour le traitement des mélanomes et des lymphomes à cellules T.

**Contenu de l'invention**

| | |
|---|---|
| INTRODUCTION..... | 3 |
| | |
| RESUME DE L'INVENTION ET DE LA DESCRIPTION..... | 7 |
| Définitions..... | 9 |
| | |
| | |
| DESCRIPTION DETAILLEE DE L'INVENTION ET LA DESCRIPTION..... | 12 |
| Figures..... | 21 |
| Exemples..... | 22 |
| Synthèse générale des composés imidazo[1,2-*a*]quinoxalines..... | 22 |
| Partie expérimentale..... | 23 |
| Chimie..... | 23 |
| Procédure pour l'addition en position 4..... | 24 |
| Procédure pour l'addition de l'ammoniac..... | 24 |
| Procédure pour l'addition de la méthylamine..... | 24 |
| Procédure générale de bromation..... | 28 |
| Procédure générale pour la réaction de couplage croisé de Suzuki..... | 31 |
| Procédure générale pour la réaction de déprotection..... | 35 |
| Biologie : Etude de l'activité des composés sur les 15 lignées tumorales, Etude de cytotoxicité in vitro..... | 39 |
| Protocole..... | 39 |
| Matériels et méthodes..... | 48 |
| Etude de l'effet des composés sur la polymérisation de la tubuline purifiée..... | 48 |
| | |
| REVENDICATIONS..... | 49 |

### INTRODUCTION

Face à la faible efficacité d'une grande majorité d'anticancéreux classiques dans le traitement de cancers tels que la prostate, le colon, le sein, le mélanome, et encore le lymphome, les recherches s'orientent vers de nouvelles stratégies thérapeutiques. En effet, réussir à contourner les problèmes de résistances et de métastases, rencontrés de plus en plus souvent dans ce type de maladie, représente un défi important pour la recherche.

De grands espoirs ont été placés dans l'immunothérapie qui contrairement aux autres thérapies, permet de traiter l'organisme dans sa globalité et peut éliminer les cellules tumorales disséminées dans l'ensemble du corps. Parmi les différentes approches d'immunothérapies, la découverte de l'Imiquimod (Aldara®), premier anticancéreux immunomodulateur, efficace notamment contre certains cancers de la peau tel que le mélanome, a permis de faire un pas en avant dans cette nouvelle voie. L'Imiquimod est une molécule tricyclique azotée, de la famille des imidazoquinolines (WO 2006/070408, US 4,689,338). Elle est tout d'abord connue pour son activité antivirale contre certains virus tels que l'herpès simplex II, le sendaï virus et le papilloma virus. Les dernières publications parues sur cette molécule mettent aussi en évidence une activité antitumorale immunomodulatrice importante sur les cancers de la peau, tels que les carcinomes basocellulaires, les kératoses actiniques et les mélanomes. Des études plus récentes ont montré également une efficacité contre les métastases cutanées et les tumeurs vasculaires. L'Imiquimod est ainsi le premier d'une nouvelle classe de médicaments anticancéreux appelés modificateurs de la réponse immunitaire innée et acquise dont le mécanisme d'action diffère de tous les anticancéreux connus comme les moutardes à l'azote, les nitroso-urées, les agents alkylants, les organo-platines, etc...

Or, un certain nombre de documents de l'art antérieur divulguent d'autres dérivés de quinoxalines pour diverses applications thérapeutiques.

Ainsi, Deleuze-Masquéfa et al. (Bioorganic & Medicinal Chemistry 12, 1129-1139, 2004) ont décrit des dérivés imidazo[1,2-*a*]quinoxalines comme inhibiteurs des PDE4 (phosphodiestérases 4). Contrairement à l'Imiquimod, ces molécules inhibent la production et les effets du TNF-α *in vitro* et elles semblent donc avoir un mode d'action différent de l'Imiquimod. Ces dérivés pourraient avoir un intérêt pour leurs propriétés anti-inflammatoires (Mojaria et al. International Journal of Immunopathology and Pharmacology, 19, 2, 77-90, 2006). Une activité anticancéreuse, et à fortiori une activité sur les mélanomes ou les lymphomes, n'est ni décrite ni suggérée dans ces documents. En effet, il est décrit que les propriétés cytoprotectrices des composés ne peuvent pas être corrélées avec leur activité IPDE4. Les composés agiraient en activant la voie p38MAPK et en inhibant la voie PI3K. Ainsi ils inhiberaient l'action et la production de TNF-α faisant de ces drogues des composés anti-inflammatoires potentiels. L'effet des composés concerne la protection de la mort cellulaire induite par TNF-α sur des cellules immunitaires. Cela ne permet pas d'envisager *a priori* les effets des composés concernant leur activité cytotoxique directe sur des cellules cancéreuses.

Bonnard et al. (RICT 2005, Paris) ont décrit la synthèse et l'évaluation pour leur activité antitumorale de dérivés imidazo[1,2-*a*]quinoxalines. La structure des composés testés n'est cependant pas décrite.

US 2003/0022898 décrit des dérivés ayant également une activité anti-inflammatoire dont un composé 4-(2'-aminoéthyl)-amino-1,8-diméthylimidazo(1,2-*a*)quinoxaline. Ce composé est également décrit comme ayant une activité anti-mélanome dans le document US 2006/0025419.

Colotta et al. (Eur. J. Med. Chem, 30, 133-139, 1995) décrit différents composés dont des imidazo[1,2-*a*]quinoxalines. Des triazoloquinoxalines sont décrites comme se liant au récepteur adénosine. Des applications particulières des imidazo[1,2-*a*]quinoxalines ne sont pas décrites.

Catarzi et al. (J. Med. Chem., 37, 2846-2850, 1994) décrit des triazoloquinoxalines ainsi que des imidazoquinoxalines se liant au récepteur de la benzodiazépine. Les molécules se liant au récepteur de la benzodiazépine sont en général reconnues pour leurs activités anxiolytiques. Ce document ne décrit pas d'applications dans le cancer.

WO 93/04066 décrit des composés imidazoquinoxalinols se liant spécifiquement au récepteurs GABAa. Seules des utilisations thérapeutiques comme sédatifs, anxiolytiques, anticonvulsifs, etc. sont envisagées. Des applications dans d'autres domaines thérapeutiques et notamment pour le traitement des cancers ne sont ni décrites ni suggérées par ce document.

Zurbonsen et al. (European Journal of Pharmacology, 320, 215-221, 1997 et Biochemical Pharmacology, 54, 365-371, 1997) décrivent des dérivés d'imidazo[1,2-*a*]pyrazines ayant une activité inhibitrice sur les phosphodiéstérases et capables d'induire l'apoptose d'une lignée cellulaire leucémique. Il est cependant à noter que tous les inhibiteurs des phosphodiestérases ne présentent pas une activité anticancéreuse. En outre, les composés de la présente invention se distinguent par une activité élevée démontrée dans des tests *in vitro* et *in vivo* aussi bien sur lymphome que sur le mélanome.

WO 2007/109813 concerne des imidazoquinoxalines ayant une activité immunomodulatrice. Seules des applications dans le domaine des adjuvants et des vaccins sont décrites. Ce document envisage des applications potentielles dans de nombreux domaines thérapeutiques dont le cancer mais aucune donnée *in vitro* ou *in vivo* vient confirmer une quelconque activité anticancéreuse.

WO 2007/087250 décrit des inhibiteurs de la 5-LO (lipoxygénase). Des applications dans le domaine du cancer ne sont pas décrites.

WO2010/124826 décrit des anticancéreux inhibiteurs de Mps-1 kinase (monopolar spindle 1 kinase), et cite notamment, parmi 330 composés, le composé 1-25 qui est un 1-(3,4-dimethoxyphenyl)-N-(2-methylpropyl) imidazo [1,2-a] quinoxalin-4-amine. Ce composé diffère des composés de l'invention tels que définis dans les revendications en ce qu'il présente un groupement phényl substitué par des groupes methoxy et une efficacité inférieure à celle des composés de l'invention.

Les premières imidazo[1,2-*a*]quinoxalines objet de FR 2,921,927 (équivalent à WO 2009/043934 déposé un an plus tard) présentent une activité intéressante, nouvelle et inattendue en 2007, sur différents cancers tels que mélanome et lymphome à cellules T. Les activités mesurées sur différentes lignées cellulaires représentatives de ces cancers montrent des activités (IC₅₀) de l'ordre de la micromole. Ainsi, une activité cytotoxique directe avec des IC₅₀ de l'ordre de la micromole a été mise en évidence *in vitro* sur différentes lignées cellulaires tumorales humaines pour les composés divulgués dans cette demande de brevet: mélanome, sein, colon, lymphome B et leucémies /lymphomes T de l'adulte (ATL) (Table 1) (Moarbess et al.), Bioorg. Med. Chem. 16, 6601-6610, 2008).

Plusieurs résultats concernant l'activité anticancéreuse *in vivo* des molécules directement issues de FR 2,921,927 ont été publiés (Moarbess et al., Bioorg. Med. Chem. 16, 6601-6610, 2008 ; Khier et al., J. Separation Sci. 2009, 32, 1363-1373 ; Khier et al., Drug Metab Dispos 2010, 38(10), 1836-1847 ; Khier et al., Eur. J. Pharm. Sci. 2010, 39(1-3):23-29 ; Lafaille et al., Anal Chem. 2012, 84(22):9865-72 ; Lafaille F. et al., J. Pharm. Biomed. Anal. 2014, 88, 429-440).

En outre, un problème majeur posé par le développement d'anticancéreux concerne leur toxicité, liée à leur manque de spécificité. En effet de nombreux anticancéreux agissent en perturbant des mécanismes cellulaires physiologiques généraux tels que par exemple l'inhibition de la tubuline. La tubuline est en effet présente dans toutes les cellules et est essentielle au processus de multiplication cellulaire, qu'il soit physiologique ou pathologique dans le cas par exemple des cancers. L'altération des processus physiologiques de multiplication cellulaire peut générer des effets secondaires importants et doit être évité. Les premières imidazo[1,2-*a*]quinoxalines synthétisées présentent en effet cet effet délétère d'inhibition de la tubuline.

Ainsi, des études d'inhibition de polymérisation de la tubuline sur fractions de tubuline purifiée ont montré une inhibition puissante par certain des composés divulgués dans FR 2,921,927 (Gattacceca et al., Faseb J., 2013, 27, Ib584 ; Saliba et al., Anti-Cancer Drugs, 2014, 25, 624-632.).

Or, les composés de la présente invention tels que définis dans les revendications ont une efficacité d'un tout autre niveau que les premiers dérivés d'imidazo[1,2-*a*]quinoxaline spécifiquement décrits dans FR 2,921,927, avec des valeurs d'IC₅₀ de l'ordre de la dizaine de nanomole, voire nanomolaire pour certains. De plus, ces nouvelles molécules ne présentent plus d'effet significatif sur la tubuline. Les molécules selon la présente invention tels que définis dans les revendications permettent en effet de ne pas altérer la polymérisation de la tubuline et donc d'éviter les effets secondaires y associés. En effet l'activité microtubulaire risquant d'être aussi bloquée dans le cas de composés inhibiteurs de polymérisation de la tubuline, tout autre activité cellulaire la nécessitant sera aussi inhibée telle que le transport axonal intra-neuronal, la phagocytose ou chimiotactisme leucocytaire par exemple. En outre, les composés de la présente invention ont démontré une activité significative sur les tumeurs testées *in vitro.* Ces effets sont donc tout à fait surprenants et permettent d'envisager de nouvelles thérapies, anticancéreuses, en particulier.

Les nouvelles propriétés apparaissent liées à une famille de molécules présentant des caractéristiques précises en terme de groupements sur le cycle phényle aromatique fixé en position 1 ou 2 de l'imidazole de l'hétérocycle imidazo[1,2-*a*]quinoxaline.

Ainsi, pour remédier aux inconvénients de l'état de la technique, la présente invention propose de nouveaux composés dérivés d'imidazo[1,2-*a*]quinoxaline tels que définis dans les revendications pour le traitement des cancers sans distinction spécifique de lieux de localisation de la ou des tumeurs. Ces composés peuvent être utilisés pour la préparation de médicaments pour le traitement des cancers.

### RESUME DE L'INVENTION ET DE LA DESCRIPTION

La présente invention concerne l'objet des revendications.

La présente description concerne également un composé de formule générale (I) : 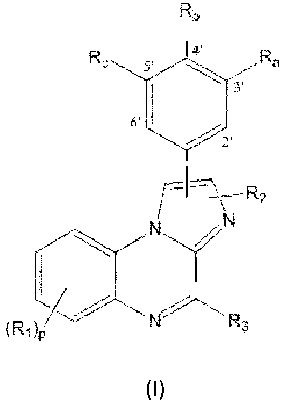 dans laquelle :
- les différents R₁ représentent indépendamment les uns des autres un atome d'hydrogène, d'halogène ou un groupe choisi parmi les groupes hydroxy, C₁-C₄ alkyle, C₁-C₄ alcoxy, amino, C₁-C₄ alkylamino, C₁-C₄ dialkylamino, cyano, -CF₃, -OCF₃, -(CH₂)ₙNR₄R₅, - NH-(CH₂)ₙNR₄R₅, -(CH₂)ₙCOR₄, -(CH₂)ₙCO-NR₄R₅, -(CH₂)ₙSO₂-NR₄R₅, et -(CH₂)ₙCO₂R₄ ;
- R₂ représente un atome d'hydrogène, ou un groupe choisi parmi les groupes hydroxy et C₁-C₂ alkyle, préférentiellement un atome d'hydrogène;
- R₃ représente un halogène ou un groupe choisi parmi les groupes amino, C₁-C₃ alkylamino, C₁-C₃ dialkylamino, amine-(C₁-C₆ alkyl)-amino, N-(tert-butyloxycarbonyl)amino-(C₁-C₆ alkyl)-amino, pipéridyle, N-(tert-butyloxycarbonyl)pipéridyle, un groupe aminé d'un acide α-aminé ou un groupe aminé de la chaîne latérale d'un acide α-aminé,
- Rₐ, R_{b} et R_{c} représentent indépendamment les uns des autres un atome d'hydrogène ou un groupe choisi parmi les groupes hydroxy, C₁-C₄ alcoxy, amino, C₁-C₄ alkylamino et C₁-C₄ dialkylamino;
- n étant 0, 1, 2, 3 ou 4, préférentiellement, n étant 0 ;
- p étant 1, 2, 3 ou 4 ;
- R₄ et R₅ représentent indépendamment l'un de l'autre un atome d'hydrogène ou un groupe C₁-C₄ alkyle, préférentiellement linéaire ou ramifié;
à la condition qu'au moins 2 des résidus Rₐ, R_{b} et R_{c} sont différents de H,
ou un sel pharmaceutiquement acceptable de celui-ci.

R3 est préférentiellement un halogène ou un groupe choisi parmi les groupes amino, particulièrement un groupe méthylamino, diméthylamino, éthylamino, diéthylamino, amineméthylamino, amineéthylamino, aminepropylamino et aminehexylamino, et en particulier un groupe méthylamino.

L'acide aminé peut être tout acide aminé naturel, tout acide aminé non naturel ou tout acide aminé dont la chaîne latérale est modifiée. De manière avantageuse l'acide aminé peut être choisi dans le groupe constitué par Gly, Ala, Val, Leu, Phe, Tyr, Ser, Lys, Orn, Arg, Glu, His, Trp, Pro, Thr, Cys, Met, Asn et Gln.

La présente description concerne en outre un procédé de fabrication d'un composé de l'invention caractérisé en ce que ledit procédé comprend :
- une étape de couplage du composé de formule (V) : 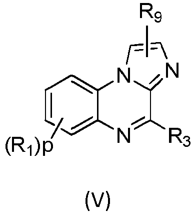 dans laquelle R₁, R₃ et p sont tels que définis présentement ; et
   R₉ est un atome d'halogène ;
   avec un acide arylboronique approprié (e.g. de type [Ph(Rₐ,R_{b},R_{c})-B(OH)₂]), diversement substitué en position méta et/ou para, par des groupements Rₐ, R_{b} et/ou R_{c} tels que définis présentement, dans les conditions de Suzuki; et
      - récupération, extraction et/ou purification du composé obtenu selon la présente invention.

La présente description concerne également une composition pharmaceutique comprenant au moins un composé selon la présente description et optionnellement un véhicule pharmaceutiquement approprié.

La présente description concerne donc un composé selon la présente description en tant que médicament.

La présente description concerne enfin un composé selon la présente description pour son utilisation dans le traitement d'au moins un cancer.

### Définitions

Par « halogène » on entend en particulier selon la présente description les halogènes suivants: F, Cl, Br et I.

Par « alkyle » on entend en particulier selon la présente description les radicaux alkyles linéraires ou ramifiés, en particulier les radicaux alkyles en C1, C2, C3, C4, C5 ou C6, lorsque la longueur de chaîne n'est pas précisée, en particulier les radicaux méthyle, éthyle, *n*-propyle, *i-*propyle, *n*-butyle, *i*-butyle ou *t*-butyle. Cette définition s'applique également aux parties alkyles des radicaux cycloalkyl, alcoxy, acyle, aralkyle, alkylamino, thioalkyle. Ainsi par « C₁-C₄ dialkylamino », il est entendu que 2 groupements alkyles en C₁-C₄ sont reliés à une même amine. Lorsque la stéréochimie d'un groupement alkyle n'est pas précisée (*e.g*. aminopropylamine), préférentiellement, il s'agit de l'alkyle linéaire (« n-alkyle », *i.e.* amino-(*n*-propyl)-amine) dans le cas de figure précédent).

Par « alkényle » on entend de préférence selon la description une chaîne hydrocarbonée, monovalente, insaturée et comprenant au moins une double liaison, linéaire ou ramifiée, comportant de 2 à 6 atomes de carbone lorsque la longueur de chaîne n'est pas précisée, dont des éléments représentatifs sont par exemple les groupes vinyle, 1-propényle, 2-propényle, isoprényle, butényle, pentényle, hexényle.

Par « cycloalkyle » on entend avantageusement selon la description les cycloalkyles en C3-C7 lorsque le nombre de carbone compris dans le cycle n'est pas précisé, plus particulièrement les radicaux cyclopropyle, cyclobutyle, cyclopentyle, cyclohexyle et cycloheptyle.

Par « aryle » on entend de préférence selon la description un ou plusieurs cycles aromatiques ayant 6 à 10 atomes de carbone lorsque le nombre de carbone compris dans le cycle n'est pas précisé, pouvant être accolés ou fusionnés, en particulier le phényle. Cette définition s'applique également à la partie aryle des radicaux aralkyles. Le groupe aralkyle est de préférence (CH₂)ₘ-phényle dans lequel m est compris entre 0 et 4.

Par « amino » ou « amine » on entend une amine primaire, secondaire ou tertiaire, préférentiellement -NH₂ ou -NH₃⁺ s'il s'agit d'un sel.

Par « méthylamino » ou « alkylamino » on entend les radicaux -NH-CH3 ou -NH-alkyle.

Par « aminealkylamino » on entend les radicaux-NH-alkyl-NH2.

Par « hétérocycle » on entend avantageusement selon la description un cycle en C3-C7, lorsque le nombre de carbone compris dans le cycle n'est pas précisé, contenant au moins un hétéroatome choisi parmi l'azote, l'oxygène ou le soufre, en particulier les hétérocycles sont choisis parmi thiényle, furyle, quinolinyle, indolyle, pyrazole, pyrrole, pyridine, pyrimidine, imidazole.

Par « cancer » on entend toutes les formations néoplasiques malignes, quelle qu'en soit la nature histologique. Il existe deux grandes catégories de tumeurs malignes : les carcinomes, d'origine épithéliale, et les sarcomes, d'origine conjonctive. Les tumeurs malignes sont formées de cellules atypiques, envahissantes ou disséminantes, caractérisées généralement par un pouvoir d'accroissement autonome, une délimitation imprécise, une capacité d'envahissement des tissus et vaisseaux voisins et une tendance à disséminer par la production de métastases. On citera notamment les cancers du sein, de la prostate, du pancréas, des poumons, de l'œsophage, de la peau, de la vessie, de l'estomac, du foie, de l'utérus, du côlon et du rectum.

Par « cancer solide » on entend les cancers à tumeur(s) de type carcinome ou sarcome. Ces cancers se développent dans n'importe quel tissu.

Par « cancer liquide » on entend les cancers de type leucémies ou lymphomes. Les leucémies sont des cancers du sang et de la moelle osseuse, alors que les lymphomes sont des cancers du système lymphatique. Ces cancers du sang résultent donc d'une prolifération tumorale des cellules sanguines. Il existe ainsi des leucémies aiguës et chroniques des cancers du système lymphatique (lymphomes, maladie de Hodgkin et myélome multiple). Par exemple, dans le cas de la leucémie, celle-ci apparaît quand des cellules sanguines (telles que les lymphocytes ou les leucocytes) encore immatures (dites «précurseurs») deviennent cancéreuses et ne peuvent plus mûrir ou se spécialiser normalement.

Par « cancer métastatique », ou encore dit « disséminé », on entend des tumeurs produisant des cellules cancéreuses migrantes à travers le corps, par la voie des vaisseaux sanguins ou lymphatiques et ayant colonisés au moins un autre tissu (et pouvant ainsi générer au moins une nouvelle tumeur).

Par « cancer secondaire » on entend les cancers consécutifs à un traitement anticancéreux, censé être thérapeutique.

Par « mélanome » on entend une tumeur maligne qui se développe aux dépens des tissus pigmentés, ceux de la peau ou de l'œil plus spécialement.

Par « lymphome » on entend toute tumeur, généralement maligne, due à une prolifération des cellules du tissu lymphoïde, se développant surtout au niveau de la rate ou des ganglions.

Par « sel pharmaceutiquement acceptable » on entend de préférence selon l'invention un sel d'acide pharmaceutiquement acceptable, c'est-à-dire avec tout acide non toxique, y compris les acides organiques et inorganiques. De tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoïque, pantothénique, phosphorique, succinique, sulfurique, tartrique et paratoluènesulfonique.

### DESCRIPTION DETAILLEE

### DE L'INVENTION ET LA DESCRIPTION

Le/les groupements R₁ selon la présente description représentent de manière préférée et indépendamment les uns des autres, un atome d'hydrogène, un halogène ou un groupe choisi parmi les groupes méthyle, éthyle, propyle, isopropyle, butyle, isobutyle, tertiobutyle, et -(CH₂)_{n'-} (CH=CH)-(CH₂)_{n"}-CH₃ avec n' et n" compris indépendamment entre 0 et 4, COOR₄, NR₄R₅ et OR₄, R₄ et R₅ sont définis ci-dessus.

De préférence, p= 0, 1, 2 ou 3. Préférentiellement, p= 0.

De préférence, R₂ est un atome d'hydrogène.

En particulier selon la présente description, Rₐ représente un atome d'hydrogène ou un groupe choisi parmi les groupes hydroxy et C₁-C₄ alcoxy. Selon la présente invention, Rₐ représente un groupe hydroxy.
En particulier selon la présente description, R_{b} représente un atome d'hydrogène ou un groupe choisi parmi les groupes hydroxy et C₁-C₄ alcoxy. Selon la présente invention, R_{b} représente un groupe hydroxy.

En particulier selon la présente description, R_{c} représente un atome d'hydrogène ou un groupe choisi parmi les groupes hydroxy et C₁-C₄ alcoxy. Selon la présente invention, R_{c} représente un atome d'hydrogène ou un groupe hydroxy.

De préférence, R₃ représente un halogène ou un groupe choisi parmi les groupes, C₁-C₃ alkylamino, C₁-C₃ dialkylamino, ou amine-(C₁-C₆ alkyl)-amino.

De manière plus avantageuse, R₃ représente un halogène ou un groupe choisi parmi les groupes amino, C₁-C₂ alkylamino, C₁-C₂ dialkylamino, amine-(C₁-C₃ alkyl)-amino ou aminehexylamino.

De manière plus avantageuse, R₃ représente un halogène ou un groupe choisi parmi les groupes C₁-C₂ alkylamino, C₁-C₂ dialkylamino, amine-(C₁-C₃ alkyl)-amino ou aminehexylamino.

De manière plus préférée, R₃ est un groupe chloro, amino, méthylamino ou amineéthylamino et plus préférentiellement méthylamino ou amineéthylamino.

De manière avantageuse, R3 est un groupe aminé d'un acide α-aminé ou un groupe aminé de la chaîne latérale d'un acide α-aminé.

Dans le cas ici évoqué ou R3 est un acide aminé, il est lié au cycle via l'azote de la fonction aminée en α de l'acide α-aminé ou via l'azote de la fonction aminée de la chaîne latérale de l'acide α-aminé comme par exemple dans le cas d'une lysine.

En particulier selon la présente description, Rₐ est choisi parmi H, hydroxy et méthoxy. Selon la présente invention, Rₐ représente un groupe hydroxy.

En particulier selon la présente description, R_{b} est choisi parmi H, hydroxy et méthoxy. Selon la présente invention, R_{b} représente un groupe hydroxy.

En particulier selon la présente description, R_{c} est choisi parmi H, hydroxy et méthoxy. Selon la présente invention, R_{c} représente un atome d'hydrogène ou un groupe hydroxy.

La présente description concerne particulièrement, un composé caractérisé en ce qu'il réponde à la formule (II) : 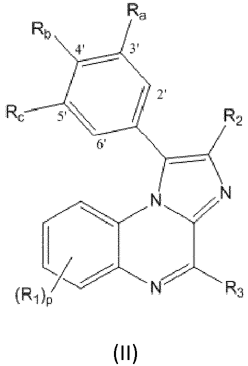
- dans laquelle les différents groupements R₁, R₂, R₃, Rₐ, R_{b}, R_{c} et p sont tels que définis ci-dessus,
ou un sel pharmaceutiquement acceptable de celui-ci.

Plus particulièrement, la présente description concerne un composé selon les formule (I) ou (II) caractérisé en ce que :
- Rₐ, R_{b} et R_{c} représentent indépendamment les uns des autres un atome d'hydrogène ou un groupe choisi parmi les groupes hydroxy, C₁-C₄ alcoxy, préférentiellement un atome d'hydrogène ou un groupe hydroxy,
ou un sel pharmaceutiquement acceptable de celui-ci.

Un aspect de la présente description concerne un composé caractérisé en ce qu'il réponde à la formule (III) : 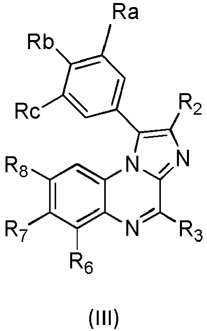
- dans laquelle les différents groupements R₂, R₃, Rₐ, R_{b} et R_{c} sont tels que définis ci-dessus,
- R₆, R₇ et R₈ représentent indépendamment l'un de l'autre un atome d'hydrogène, d'halogène ou un groupe choisi parmi les groupes hydroxy, C₁-C₄ alkyle, C₁-C₄ alcoxy, amino, C₁-C₄ alkylamino, C₁-C₄ dialkylamino, cyano, CF₃, OCF₃, -(CH₂)ₙNR₄R₅,-NH-(CH₂)ₙNR₄R₅, -(CH₂)ₙCOR₄, -(CH₂)ₙCO-NR₄R₅, -(CH₂)ₙSO₂-NR₄R₅, et -(CH₂)ₙCO₂R₄;
- n étant 0, 1, 2, 3 ou 4, préférentiellement, n étant 0 ; et
- les différents R₄ et R₅ représentent indépendamment les uns des autres un atome d'hydrogène ou un groupe C₁-C₄ alkyle, préférentiellement linéaire ou ramifié ;
ou un sel pharmaceutiquement acceptable de celui-ci.

Avantageusement, le composé de formule (III) est caractérisé en ce que :
- R₆, R₇ et R₈ représentent indépendamment l'un de l'autre un atome d'hydrogène, d'halogène ou un groupe choisi parmi les groupes hydroxy, C₁-C₄ alkyle, C₁-C₄ alcoxy, OCF₃, préférentiellement hydrogène, methoxy, OCF₃ ou méthyle ;
ou un sel pharmaceutiquement acceptable de celui-ci.

Dans un mode de réalisation particulier, la présente description concerne un composé caractérisé en ce qu'il réponde à la formule (IV) : 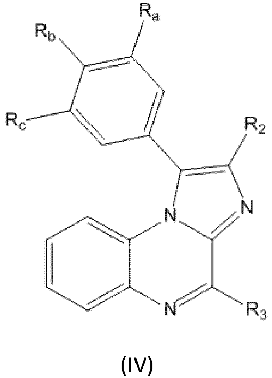
- dans laquelle les différents groupements R₂, R₃, Rₐ, R_{b} et R_{c} sont tels que définis ci-dessus;
ou un sel pharmaceutiquement acceptable de celui-ci.

Dans un mode particulier de réalisation, la présente invention vise un composé selon (I) ou (II) dans lequel
R1 = H
R2 = H
R3 = NH2 ou NHCH3
Ra et Rb = OH et Rc=H

Dans un autre mode particulier de réalisation, la description vise également un composé selon (I) ou (II) dans lequel
R1 = H
R2 = H
R3 = NH2 ou NHCH3
Ra et Rb = OCH3 et Rc = H

Les composés selon la présente invention sont choisis parmi les composés listés dans la revendication 6.

Les composés selon la présente description sont choisis parmi les composés suivants :
1. 1-(3,4-diméthoxyphényl)-N-méthylimidazo[1,2-a]quinoxalin-4-amine
2. 1-(3,5-diméthoxyphényl)-N-méthylimidazo[1,2-a]quinoxalin-4-amine
3. 1-(3,4-diméthoxyphényl)imidazo[1,2-a]quinoxalin-4-amine
4. N-méthyl-1-(3,4,5-triméthoxyphényl)imidazo[1,2-a]quinoxalin-4-amine
5. 1-(3,4-dihydroxyphényl)imidazo[1,2-a]quinoxalin-4-amine
6. 1-(3,4-dihydroxyphényl)-N-méthylimidazo[1,2-a]quinoxalin-4-amine
7. 1-(3,5-dihydroxyphényl)-N-méthylimidazo[1,2-a]quinoxalin-4-amine
8. 4-(4-amino-6-(trifluorométhyl)imidazo[1,2-a]quinoxalin-1-yl)benzène-1,2-diol
9. 4-(4-(méthylamino)-6-(trifluorométhyl)imidazo[1,2-a]quinoxalin-1-yl)benzène-1,2-diol
10. 4-(4-((2-aminoéthyl)amino)-6-(trifluorométhyl)imidazo[1,2-a]quinoxalin-1-yl)benzène-1,2-diol
11. 4-(4-((3-aminopropyl)amino)-6-(trifluorométhyl)imidazo[1,2-a]quinoxalin-1-yl)benzène-1,2-diol
12. 4-(4-((6-aminohexyl)amino)-6-(trifluorométhyl)imidazo[1,2-a]quinoxalin-1-yl)benzène-1,2-diol
13. 4-amino-1-(3,4-dihydroxyphényl)imidazo[l,2-a]quinoxaline-6-carbonitrile
14. 1-(3,4-dihydroxyphényl)-4-(méthylamino)imidazo[1,2-a]quinoxaline-6-carbonitrile
15. 4-((2-aminoéthyl)amino)-1-(3,4-dihydroxyphényl)imidazo[1,2-a]quinoxaline-6-carbonitrile
16. 4-((3-aminopropyl)amino)-1-(3,4-dihydroxyphényl)imidazo[1,2-a]quinoxaline-6-carbonitrile
17. 4-((6-aminohexyl)amino)-1-(3,4-dihydroxyphényl)imidazo[1,2-a]quinoxaline-6-carbonitrile
18. 4-amino-1-(3,4-dihydroxyphényl)imidazo[1,2-a]quinoxaline-6-carboxylate de méthyle
19. 1-(3,4-dihydroxyphényl)-4-(méthylamino)imidazo[1,2-a]quinoxaline-6-carboxylate de méthyle
20. 4-((3-aminopropyl)amino)-1-(3,4-dihydroxyphényl)imidazo[1,2-a]quinoxaline-6-carboxylate de méthyle
21. 4-((6-aminohexyl)amino)-1-(3,4-dihydroxyphényl)imidazo[1,2-a]quinoxaline-6-carboxylate de méthyle
22. Acide 4-amino-1-(3,4-dihydroxyphényl)imidazo[1,2-a]quinoxaline-6-carboxylique
23. Acide 1-(3,4-dihydroxyphényl)-4-(méthylamino)imidazo[1,2-a]quinoxaline-6-carboxylique
24. Acide 4-((2-aminoéthyl)amino)-1-(3,4-dihydroxyphényl)imidazo[1,2-a]quinoxaline-6-carboxylique
25. Acide 4-((3-aminopropyl)amino)-1-(3,4-dihydroxyphényl)imidazo[1,2-a]quinoxaline-6-carboxylique
26. Acide 4-((6-aminohexyl)amino)-1-(3,4-dihydroxyphényl)imidazo[1,2-a]quinoxaline-6-carboxylique
27. 4-(4,6-diaminoimidazo[1,2-a]quinoxalin-1-yl)benzène-1,2-diol
28. 4-(6-amino-4-(méthylamino)imidazo[1,2-a]quinoxalin-1-yl)benzène-1,2-diol
29. 4-(6-amino-4-((2-aminoéthyl)amino)imidazo[1,2-a]quinoxalin-1-yl)benzène-1,2-diol
30. 4-(6-amino-4-((3-aminopropyl)amino)imidazo[1,2-a]quinoxalin-1-yl)benzène-1,2-diol
31. 4-(6-amino-4-((6-aminohexyl)amino)imidazo[1,2-a]quinoxalin-1-yl)benzène-1,2-diol
32. 4-(4-amino-7-(trifluorométhyl)imidazo[1,2-a]quinoxalin-1-yl)benzène-1,2-diol
33. 4-(4-(méthylamino)-7-(trifluorométhyl)imidazo[1,2-a]quinoxalin-1-yl)benzène-1,2-diol
34. 4-(4-((2-aminoéthyl)amino)-7-(trifluorométhyl)imidazo[1,2-a]quinoxalin-1-yl)benzène-1,2-diol
35. 4-(4-((3-aminopropyl)amino)-7-(trifluorométhyl)imidazo[1,2-a]quinoxalin-1-yl)benzène-1,2-diol
36. 4-(4-((6-aminohexyl)amino)-7-(trifluorométhyl)imidazo[1,2-a]quinoxalin-1-yl)benzène-1,2-diol
37. 4-amino-1-(3,4-dihydroxyphényl)imidazo[1,2-a]quinoxaline-7-carbonitrile
38. 1-(3,4-dihydroxyphényl)-4-(méthylamino)imidazo[1,2-a]quinoxaline-7-carbonitrile
39. 4-((2-aminoéthyl)amino)-1-(3,4-dihydroxyphényl)imidazo[1,2-a]quinoxaline-7-carbonitrile
40. 4-((3-aminopropyl)amino)-1-(3,4-dihydroxyphényl)imidazo[1,2-a]quinoxaline-7-carbonitrile
41. 4-((6-aminohexyl)amino)-1-(3,4-dihydroxyphényl)imidazo[1,2-a]quinoxaline-7-carbonitrile
42. Acide 4-amino-1-(3,4-dihydroxyphényl)imidazo[1,2-a]quinoxaline-7-carboxylique
43. Acide 1-(3,4-dihydroxyphényl)-4-(méthylamino)imidazo[1,2-a]quinoxaline-7-carboxylique
44. Acide 4-((2-aminoéthyl)amino)-1-(3,4-dihydroxyphényl)imidazo[1,2-a]quinoxaline-7-carboxylique
45. Acide 4-((3-aminopropyl)amino)-1-(3,4-dihydroxyphényl)imidazo[1,2-a]quinoxaline-7-carboxylique
46. Acide 4-((6-aminohexyl)amino)-1-(3,4-dihydroxyphényl)imidazo[1,2-a]quinoxaline-7-carboxylique
47. 4-(4,7-diaminoimidazo[1,2-a]quinoxalin-1-yl)benzène-1,2-diol
48. 4-(7-amino-4-(méthylamino)imidazo[1,2-a]quinoxalin-1-yl)benzène-1,2-diol
49. 4-(7-amino-4-((2-aminoéthyl)amino)imidazo[1,2-a]quinoxalin-1-yl)benzène-1,2-diol
50. 4-(7-amino-4-((3-aminopropyl)amino)imidazo[1,2-a]quinoxalin-1-yl)benzène-1,2-diol
51. 4-(7-amino-4-((6-aminohexyl)amino)imidazo[1,2-a]quinoxalin-1-yl)benzène-1,2-diol
52. 4-(4-amino-8-(trifluorométhyl)imidazo[1,2-a]quinoxalin-1-yl)benzène-1,2-diol
53. 4-(4-(méthylamino)-8-(trifluorométhyl)imidazo[1,2-a]quinoxalin-1-yl)benzène-1,2-diol
54. 4-(4-((2-aminoéthyl)amino)-8-(trifluorométhyl)imidazo[1,2-a]quinoxalin-1-yl)benzène-1,2-diol
55. 4-(4-((3-aminopropyl)amino)-8-(trifluorométhyl)imidazo[1,2-a]quinoxalin-1-yl)benzène-1,2-diol
56. 4-(4-((6-aminohexyl)amino)-8-(trifluorométhyl)imidazo[1,2-a]quinoxalin-1-yl)benzène-1,2-diol
57. 4-amino-1-(3,4-dihydroxyphényl)imidazo[1,2-a]quinoxaline-8-carbonitrile
58. 1-(3,4-dihydroxyphényl)-4-(méthylamino)imidazo[1,2-a]quinoxaline-8-carbonitrile
59. 4-((2-aminoéthyl)amino)-1-(3,4-dihydroxyphényl)imidazo[1,2-a]quinoxaline-8-carbonitrile
60. 4-((3-aminopropyl)amino)-1-(3,4-dihydroxyphényl)imidazo[1,2-a]quinoxaline-8-carbonitrile
61. 4-((6-aminohexyl)amino)-1-(3,4-dihydroxyphényl)imidazo[1,2-a]quinoxaline-8-carbonitrile
62. 4-amino-1-(3,4-dihydroxyphényl)imidazo[1,2-a]quinoxaline-8-carboxylate de méthyle
63. 1-(3,4-dihydroxyphényl)-4-(méthylamino)imidazo[1,2-a]quinoxaline-8-carboxylate de méthyle
64. 4-((2-aminoéthyl)amino)-1-(3,4-dihydroxyphényl)imidazo[1,2-a]quinoxaline-8-carboxylate de méthyle
65. 4-((3-aminopropyl)amino)-1-(3,4-dihydroxyphényl)imidazo[1,2-a]quinoxaline-8-carboxylate de méthyle
66. 4-((6-aminohexyl)amino)-1-(3,4-dihydroxyphényl)imidazo[1,2-a]quinoxaline-8-carboxylate de méthyle
67. Acide 4-amino-1-(3,4-dihydroxyphényl)imidazo[1,2-a]quinoxaline-8-carboxylique
68. Acide 1-(3,4-dihydroxyphényl)-4-(méthylamino)imidazo[1,2-a]quinoxaline-8-carboxylique
69. Acide 4-((2-aminoéthyl)amino)-1-(3,4-dihydroxyphényl)imidazo[1,2-a]quinoxaline-8-carboxylique
70. Acide 4-((3-aminopropyl)amino)-1-(3,4-dihydroxyphényl)imidazo[1,2-a]quinoxaline-8-carboxylique
71. Acide 4-((6-aminohexyl)amino)-1-(3,4-dihydroxyphényl)imidazo[1,2-a]quinoxaline-8-carboxylique
72. 4-(4,8-diaminoimidazo[1,2-a]quinoxalin-1-yl)benzène-1,2-diol
73. 4-(8-amino-4-(méthylamino)imidazo[1,2-a]quinoxalin-1-yl)benzène-1,2-diol
74. 4-(8-amino-4-((2-aminoéthyl)amino)imidazo[1,2-a]quinoxalin-1-yl)benzène-1,2-diol
75. 4-(8-amino-4-((3-aminopropyl)amino)imidazo[1,2-a]quinoxalin-1-yl)benzène-1,2-diol
76. 4-(8-amino-4-((6-aminohexyl)amino)imidazo[1,2-a]quinoxalin-1-yl)benzène-1,2-diol
77. tert-butyl (2-((1-(3,4-diméthoxyphényl)imidazo[1,2-a]quinoxalin-4-yl)amino)éthyl)carbamate
78. tert-butyl (6-((1-(3,4-diméthoxyphényl)imidazo[1,2-a]quinoxalin-4-yl)amino)hexyl)carbamate
79. tert-butyl 4-(1-(3,4-diméthoxyphényl)imidazo[1,2-a]quinoxalin-4-yl)pipérazine-1-carboxylate
80. tert-butyl (3-((1-(3,4-diméthoxyphényl)imidazo[1,2-a]quinoxalin-4-yl)amino)propyl)carbamate
81. N-(1,3-dihydroxy-2-(hydroxyméthyl)propan-2-yl)-2-((1-(3,4-diméthoxyphényl)imidazo[1,2-a]quinoxalin-4-yl)amino)acétamide
82. 1-(3,4-diméthoxyphényl)-N-méthyl-7-(trifluorométhyl)imidazo[1,2-a]quinoxalin-4-amine
83. 1-(3,4-diméthoxyphényl)-N-méthyl-7-(trifluorométhoxy)imidazo[1,2-a]quinoxalin-4-amine
84. 1-(3,4-diméthoxyphényl)-N-méthyl-7-(carbonitrile)imidazo[1,2-a]quinoxalin-4-amine
85. 1-(3,4-diméthoxyphényl)-N-méthyl-8-(trifluorométhyl)imidazo[1,2-a]quinoxalin-4-amine
86. 1-(3,4-dihydroxyphényl)imidazo[1,2-a]quinoxalin-4-amine
87. 1-(3,4-dihydroxyphényl)-N-méthylimidazo[1,2-a]quinoxalin-4-amine
88. 1-(3,5-dihydroxyphényl)-N-méthylimidazo[1,2-a]quinoxalin-4-amine
89. 4-(4-((2-aminoéthyl)amino)imidazo[1,2-a]quinoxalin-1-yl)benzène-1,2-diol
90. Chlorure de 2-((1-(3,4-dihydroxyphényl)imidazo[1,2-a]quinoxalin-4-yl)amino)éthylammonium
91. 4-(4-((6-aminohexyl)amino)imidazo[1,2-a]quinoxalin-1-yl)benzène-1,2-diol
92. Chlorure de 6-((1-(3,4-dihydroxyphényl)imidazo[1,2-a]quinoxalin-4-yl)amino)hexan-1-ammonium
93. 4-(4-(pipérazin-1-yl)imidazo[1,2-a]quinoxalin-1-yl)benzène-1,2-diol
94. 4-(4-((3-aminopropyl)amino)imidazo[1,2-a]quinoxalin-1-yl)benzène-1,2-diol
95. N-(1,3-dihydroxy-2-(hydroxyméthyl)propan-2-yl)-2-((1-(3,4-dihydroxyphényl)imidazo[1,2-a]quinoxalin-4-yl)amino)acétamide
96. 4-(4-(méthylamino)-7-(trifluorométhoxy)imidazo[1,2-a]quinoxalin-1-yl)benzène-1,2-diol
97. 4-(4-(méthylamino)-8-(trifluorométhyl)imidazo[1,2-a]quinoxalin-1-yl)benzène-1,2-diol
98. Acide (2S)-2-((1-(3,4-diméthoxyphényl)imidazo[1,2-a]quinoxalin-4-yl)amino)propanoïque
99. Acide (2S)-2-((1-(3,4-diméthoxyphényl)imidazo[1,2-a]quinoxalin-4-yl)amino)-3-méthylbutanoïque
100. Acide (2S)-2-amino-5-((1-(3,4-dihydroxyphényl)imidazo[1,2-a]quinoxalin-4-yl)amino)pentanoique

Avantageusement, selon la présente invention, le composé de formule (I), (II), (III) ou (IV) est caractérisé en ce que :
R₂ et R_{c} sont des atomes d'hydrogène ;
R₃ est un groupe méthylamino ou amino et
   - Rₐ et R_{b} sont des groupes hydroxy ;
ou un sel pharmaceutiquement acceptable de celui-ci.

En particulier, le composé selon la présente description peut être choisi dans le groupe consistant en 1-(3,4-diméthoxyphényl)-N-méthylimidazo[1,2-a]quinoxalin-4-amine, 1-(3,5-diméthoxyphényl)-N-méthylimidazo[1,2-a]quinoxalin-4-amine, 1-(3,4-diméthoxyphényl)imidazo[1,2-a]quinoxalin-4-amine, 1-(3,5-diméthoxyphényl)imidazo[1,2-a]quinoxalin-4-amine, 1-(3,4,5-triméthoxyphényl)-N-méthylimidazo[1,2-a]quinoxalin-4-amine, 1-(3,4-dihydroxyphényl)-N-méthylimidazo[1,2-a]quinoxalin-4-amine, 1-(3,4-dihydroxyphényl)imidazo[1,2-a]quinoxalin-4-amine, 1-(3,5-dihydroxyphényl)-N-méthylimidazo[1,2-a]quinoxalin-4-amine et 1-(3,5-dihydroxyphényl)imidazo[1,2-a]quinoxalin-4-amine. Avantageusement, le composé selon la présente invention peut être choisi dans le groupe consistant en 1-(3,4-dihydroxyphényl)-N-méthylimidazo[1,2-a]quinoxalin-4-amine et 1-(3,4-dihydroxyphényl)imidazo[1,2-a]quinoxalin-4-amine.

En outre la composition selon la présente invention peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

L'objet de la présente invention concerne également un composé tel que décrit présentement pour son utilisation dans le traitement d'un cancer solide, d'un cancer liquide, éventuellement métastatique ou secondaire.

De manière avantageuse, le composé pour son utilisation selon la présente invention est caractérisé en ce que le cancer est un cancer du sein, de la prostate, du pancréas, des poumons, de l'œsophage, de la peau, de la vessie, de l'estomac, du foie, de l'utérus, du côlon ou du rectum.

Dans un mode particulier, le composé pour son utilisation selon la présente invention est caractérisé en ce que le cancer est un cancer de la prostate métastatique. Les cancers de la prostate métastatiques sont des cancers qui présentent des métastases c'est-à-dire des tumeurs formées à partir de cellules cancéreuses qui se sont détachées d'une première tumeur et qui ont migré par les vaisseaux lymphatiques ou les vaisseaux sanguins dans une autre partie du corps où elles se sont installées et développées.

De manière également avantageuse, le composé pour son utilisation selon la présente invention est caractérisé en ce que le cancer est un cancer du sang ou de certaines cellules du sang telles que lymphocytes ou leucocytes.

En outre, le composé pour son utilisation selon la présente invention est caractérisé en ce que le cancer est un mélanome, un cancer du pancréas, du colon ou de la prostate, un lymphome, une leucémie ou un myélome.

### Figures

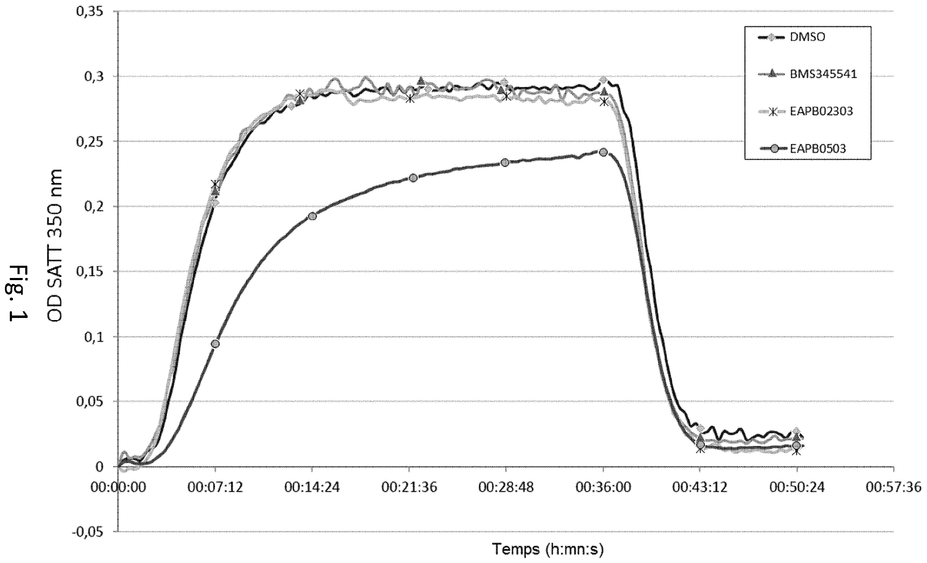

**Figure 1****. Etude de l'effet innovant des composés du brevet de sélection sur la polymérisation de la tubuline purifiée.** Il s'agit d'une étude comparée de l'effet sur la polymérisation de la tubuline purifiée des composés du brevet de sélection par rapport à celui des composés du brevet FR 2,921,927. La tubuline polymérisée absorbe à 350 nm. EAPB0503 diminue le niveau d'absorbance contrairement à EAPB02303 qui ne le modifie pas.

### Exemples

Les exemples qui suivent ne limitent en rien l'objet de la présente invention et sont fournis à titre d'illustration de la présente invention.

### Synthèse générale des composés imidazo[1,2-a]quinoxalines

Les dérivés de l'imidazoquinoxaline non substitués sur la position 1 sont synthétisés selon une stratégie de synthèse décrite précédemment (Moarbess et al. 2008 Bioorganic & Medicinal Chemistry 16, 6601-6610, Masquefa et al. 2009 European Journal of Medicinal Chemistry 44, 3406-3411). L'avantage de ce schéma de synthèse est l'obtention directe des composés de structure imidazoquinoxaliniques sans purification intermédiaire ou finale. Cette stratégie de synthèse permet l'obtention du composé 3 avec de bons rendements et une pureté suffisante pour la poursuite de la synthèse (schéma 1). L'obtention des dérivés substitués en position 4 par un atome de chlore ou une fonction amine peut bénéficier de l'assistance par micro-ondes, bien qu'un chauffage conventionnel soit toujours possible. L'utilisation des micro-ondes permet de diminuer les temps de réaction et d'optimiser les rendements de réaction. La réaction de couplage croisé de type Suzuki peut aussi être réalisée sous assistance micro-ondes et permet l'obtention d'une grande diversité de nouveaux dérivés substitués en position 1 de l'hétérocycle. Le schéma 1 de rétrosynthèse illustre la méthode de cyclisation préférentiellement utilisée permettant l'obtention d'un dimère carbonyl-imidazole 1 par condensation bimoléculaire de l'acide 2-imidazole carboxylique. Le dimère carbonyl-imidazole 1 est couplé à une *ortho-*fluoroaniline pour donner un intermédiaire 2, qui est ensuite cyclisé pour donner accès aux composés tricycliques 3. Les composés chlorés sont obtenus par réaction de l'oxychlorure de phosphore en présence de *N,N*-diéthylaniline dans un flacon scellé sous assistance micro-ondes puis purifiés par chromatographie liquide sur colonne de gel de silice.

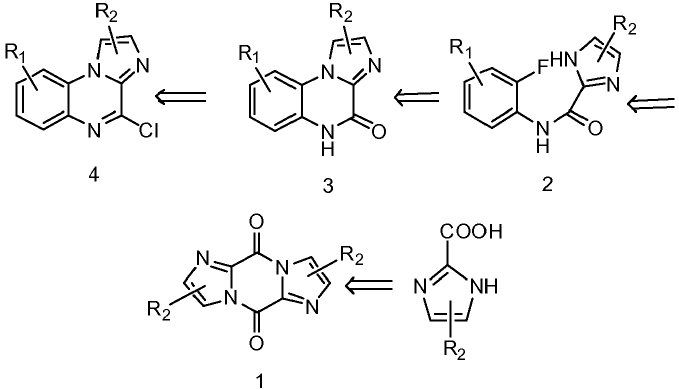

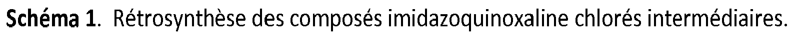

L'atome de chlore en position 4 est substitué par un groupement approprié (R₃). Les composés ainsi substitués en position 4 de l'hétérocycle sont traités par *N*-bromosuccinimide sous assistance micro-ondes pour obtenir les nouveaux dérivés substitués en position 1 ou 2 par un atome de brome.

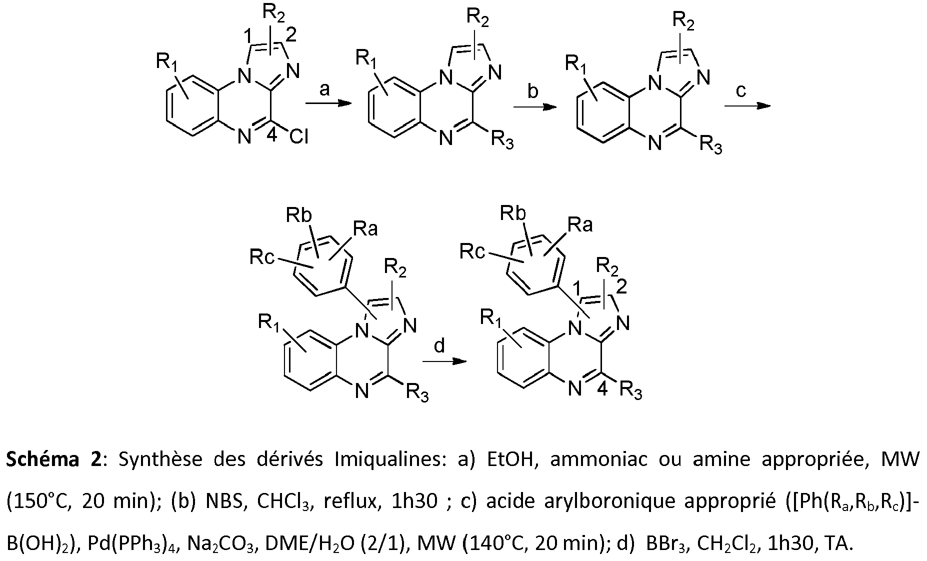

Les acides arylboroniques sont couplés par réaction de Suzuki sous catalyse palladium aux dérivés imidazoquinoxaliniques bromés en position 1 ou 2 pour obtenir les composés aryles substitués avec de bons rendements. Si des composés aryles méthoxylés sont utilisés pour cette étape de couplage, la déméthylation permet de régénérer les fonctions phénols sous l'action du tribromure de bore.

### Partie expérimentale

### Chimie

Les solvants et réactifs sont obtenus à partir de sources commerciales et utilisés sans purification complémentaire. Les spectres ¹H and ¹³C NMR sont enregistrés sur un spectromètre Brüker AC 400. Les déplacements chimiques sont exprimés en partie par million (ppm) avec pour référence la résonance du tétraméthylsilane dans le solvant deutéré d'analyse. Les constantes de couplage sont exprimées en Hertz (Hz), avec les caractéristiques de couplage suivantes: (s) singulet, (br s) singulet large, (d) doublet, (dd) doublet de doublets (t), triplet, (td) triplet de doublets, (m) multiplet. Les chromatographies sur colonne de silice sont réalisées avec un gel de silice Aldrich silica gel 60 Å (230-400 mesh). Les analyses élémentaires sont réalisées au Département Central de Microanalyse (Montpellier, France). Les analyses de spectrométrie de masse sont obtenues grâce à un spectromètre Micromass-QTOF (Waters) équipé d'une source ESI. Les données sont enregistrées en mode positif entre 50 et 1500 Da. Les tensions capillaires et de cone sont respectivement de 3000V et 20V. L'assistance par micro-ondes est réalisée sous appareil Biotage initiator (France).

### Procédure pour l'addition en position 4

### Procédure pour l'addition de l'ammoniac

### Imidazo[1,2-a]quinoxalin-4-amine

Une solution aqueuse d'ammoniaque à 33% (2.7 mL, 19.7 mmol) est ajoutée à une solution de 4-chloroimidazo[1,2-*a*]quinoxaline (0.500 g, 2.5 mmol) dans l'acétonitrile (5 mL). Le mélange est chauffé par microondes à 140°C pendant 4h. Le solvant est évaporé sous pression réduite et le résidu est dissous dans 20 mL de dichlorométhane. Cette solution est successivement lavée avec une solution saturée en NaCl (15 mL) et de l'eau (15 mL), séchée (Na₂SO₄), et concentrée sous pression réduite. Le produit obtenu (rendement 94 %) est utilisé sans purification supplémentaire. ¹H NMR (300 MHz, DMSO-d₆) *δ* : 7.92 (d, 1H), 7.79 (d, 1H), 7.72 (m, 2H), 7.65 (m, 2H), 6.66 (br s, 2H); ¹³ C (300 MHz, DMSO-d₆) *δ* : 142.49, 131.92, 129.26, 128.88, 127.58, 125.81, 125.26, 124.72, 114.26, 111.75.

### Procédure pour l'addition de la méthylamine

Le 4-chloroimidazo[1,2-*a*]quinoxaline est mis en solution dans l'éthanol dans un vial adapté pour les réactions sous micro-ondes et équipé d'un barreau aimanté. Une solution de méthylamine à 33% dans l'éthanol est ajoutée (20 éq.). Le mélange est scellé puis homogénéisé aux ultrasons avant d'être placé aux micro-ondes durant 20 min à 150°C. La puissance délivrée s'ajuste automatiquement à 50 Watts environ. L'éthanol est évaporé sous pression réduite. Le milieu réactionnel est repris dans du dichlorométhane, lavé trois fois par une solution saturée de bicarbonate de sodium, séché sur sulfate de magnésium, filtré et concentré. Le brut réactionnel est purifié par chromatographie sur colonne de gel de silice afin d'obtenir les produits attendus.

### N-méthylimidazo[1,2-a]quinoxalin-4-amine (PED26)

Addition de la méthylamine sur la 4-chloroimidazo[1,2-*a*]quinoxaline. Rdt 88%. Mw : 198.22 g/mol. 1H-RMN δ (ppm, 400 MHz, DMSO d6) 3.25 (d, 3H, NH*CH3*), 6.15 (s, 1H), 7.25 (t, 1H), 7.40 (t, 1H), 7.52 (s, 1H), 7.65 (dd, 1H), 7.75 (dd, 1H), 7.92 (s, 1H). ¹³ C (300 MHz, DMSO-d₆) *δ* : 29.20, 113.89, 114.14, 124.34, 125.14, 127.20, 128.17, 129.40, 131.99, 139.17, 142.25

### N-méthyl-7-(trifluorométhyl)imidazo[1,2-a]quinoxalin-4-amine

Addition de la méthylamine sur la 4-chloro-7-(trifluorométhyl)imidazo[1,2-a]quinoxaline. Rdt : 94%. Mw : 266.22 g/mol. 1H-RMN δ (ppm, 400 MHz, DMSO d6) 3.04 (d, 3H, *CH3*NH, J=8 Hz), 7.60 (d, 1H, CH 8, J=8Hz), 7.67 (d, 1H, CH 2, J=4Hz), 7.85 (s, 1H, CH 6), 8.06 (d, 1H, *NH*CH3, J=4Hz), 8.31 (d, 1H, CH 9, J=8Hz), 8.69 (d, 1H, CH 1, J=4Hz). 13C-RMN δ (ppm, 100 MHz, DMSO d6) 27.73 (CH3NH), 115.71 (CH 1), 117.15 (CH 9), 118.92 (CH 8), 123.22 (CH 6), 127.26 (Cq 5'), 132.76 (CH 2), 132.87 (Cq 3'), 137.63 (Cq 9'), 149.11 (Cq 4). 19F-RMN δ (ppm, 400 MHz, DMSO d6) -60.42. MS (ESI +, QTof, m/z) : 267.10 [M+H]⁺

### N-méthyl-7-(trifluorométhoxy)imidazo[1,2-a]quinoxalin-4-amine

Addition de la méthylamine sur la 4-chloro-7-(trifluorométhoxy)imidazo[1,2-a]quinoxaline. Rdt : 70%. Mw : 282.22 g/mol. 1H-RMN δ (ppm, 400 MHz, DMSO d6) 3.04 (d, 3H, *CH3*NH, J=8 Hz), 7.28 (d, 1H, CH 8, J=8Hz), 7.49 (s, 1H, CH 6), 7.64 (d, 1H, CH 2, J=4Hz), 8.02 (d, 1H, *NH*CH3, J=4Hz), 8.21 (d, 1H, CH 9, J=8Hz), 8.62 (d, 1H, CH 1, J=4Hz). 13C-RMN δ (ppm, 100 MHz, DMSO d6) 27.73 (*CH3*NH), 115.51 (CH 1, CH 8), 117.49 (CH 9), 117.99 (CH 6), 123.79 (Cq 5'), 132.51 (CH 2), 132.84 (Cq 3'), 138.75 (Cq 9'), 146.71 (Cq 7), 149.06 (Cq 4). 19F-RMN δ (ppm, 100 MHz, DMSO d6) -56.81 (OCF3). MS (ESI +, QTof, m/z) : 282.9 [M+H]⁺

### N-méthyl-7-(carbonitrile)imidazo[1,2-a]quinoxalin-4-amine

Addition de la méthylamine sur la 4-chloro-7-(carbonitrile)imidazo[1,2-a]quinoxaline. Rdt : 60%. Mw : 223.23 g/mol. 1H-RMN δ (ppm, 400 MHz, DMSO d6) 3.04 (d, 3H, C*H3*NH), 7.68 (s, 1H, CH 2), 7.70 (d, 1H, CH 8, J=8Hz), 8.00 (s, 1H, CH 6), 8.11 (m, 1H, *NH*CH3), 8.28 (d, 1H, CH 9, J=8Hz), 8.68 (s, 1H, CH 1). 13C-RMN δ (ppm, 100 MHz, DMSO d6) 27.77 (CH3), 109.23 (Cq 7), 115.83 (CH1), 117.40 (CH 9), 119.25 (Cq CN), 125.76 (CH 8), 127.94 (Cq 5'), 130.39 (CH 6), 132.93 (CH 2), 133.13 (Cq 3'), 137.79 (Cq 9'), 149.10 (Cq 4). MS (ESI +, QTof, m/z) : 224.0 [M+H]⁺

### N-méthyl-8-(trifluorométhyl)imidazo[1,2-a]quinoxalin-4-amine

Addition de la méthylamine sur la 4-chloro-8-(trifluorométhyl)imidazo[1,2-a]quinoxaline. Rdt : 82%. Mw: 266.22 g/mol. 1H-RMN δ (ppm, 400 MHz, DMSO d6) 3.06 (d, 3H, *CH3*NH, J=4Hz), 7.65 (d, 1H, CH 2, 4Hz), 7.67 (d, 1H, CH 7, J=8Hz), 7.72 (d, 1H, CH 6, J=8Hz), 8.15 (m, 1H, NH), 8.55 (s, 1H, CH 9), 8.81 (s, 1H, CH 1). 13C-RMN δ (ppm, 100 MHz, DMSO d6) 27.75 (CH3NH), 113.72 (CH 9), 115.88 (CH 1), 122.46 (CH 7), 124.90 (Cq 5'), 127.08 (CH 6), 132.57 (CH 2), 132.95 (Cq 3'), 140.53 (Cq 9'), 149.49 (Cq 4). 19F-RMN δ (ppm, 100 MHz, DMSO d6) -59.36 (CF3). MS (ESI +, QTof, m/z) : 267.00 [M+H]⁺

### Procédure pour l'addition d'une autre amine primaire ou secondaire

Le 4-chloroimidazo[1,2-*a*]quinoxaline et l'amine primaire (ou secondaire) (2 éq.) sont solubilisés dans l'acétonitrile (12 mL) dans un vial adapté pour les réactions sous micro-ondes et équipé d'un barreau aimanté. On ajoute 4 éq. de DIEA. Le mélange est scellé puis homogénéisé aux ultrasons avant d'être placé aux micro-ondes durant 20 min à 150°C. La puissance délivrée s'ajuste automatiquement à 45 Watts environ. L'acétonitrile est évaporé sous pression réduite. Le milieu réactionnel est repris dans de l'acétate d'éthyle, lavé par une solution saturée de chlorure d'ammonium, puis par de l'eau distillée, et enfin par une solution saturée de chlorure de sodium. La phase organique est séchée sur sulfate de magnésium, filtrée et concentrée. Le brut réactionnel est purifié par chromatographie sur colonne de gel de silice afin d'obtenir le composé d'addition attendu.

### tert-butyl (2-(imidazo[1,2-a]quinoxalin-4-ylamino)éthyl)carbamate

Addition de la *N-Boc*-éthylènediamine sur la 4-chloro-imidazo[1,2-a]quinoxaline. Rdt : 81%. Mw : 327.38 g/mol. 1H-RMN δ (ppm, 400 MHz, DMSO d6) 1.36 (s, 9H, 3 x CH3 tBu), 3.26 (qd, 2H, *CH2*NHBoc, J=4Hz), 3.61 (qd, 2H, *CH2*NH, J=4Hz), 7.01 (t, 1H, *NH*Boc, J=4Hz), 7.28 (t, 1H, CH 7, J=8Hz), 7.30 (t, 1H, CH 8, J=8Hz), 7.58 (d, 1H, CH 9, J=8Hz), 7.62 (d, 1H, CH 2, J=4Hz), 7.68 (t, 1H, NH, J=4Hz), 8.11 (d, 1H, CH 6, J=8Hz), 8.60 (d, 1H, CH 1, J=4Hz). 13C-RMN δ (ppm, 100 MHz, DMSO d6) 28.68 (CH3 tBu), 40.00 (CH2NHBoc, *CH2*NH), 78.07 (Cq tBu), 115.03 (CH 1), 115.83 (CH 6), 123.17 (CH 7), 124.77 (Cq 5'), 126.62 (CH 9), 126.78 (CH 8), 132.26 (CH 2), 132.45 (Cq 3'), 137.17 (Cq 9'), 147.79 (Cq 4), 156.23 (C=O carbamate). MS (ESI +, QTof, m/z) : 328.18 [M+H]⁺

### tert-butyl (6-(imidazo[1,2-a]quinoxalin-4-ylamino)hexyl)carbamate

Addition de la *N-Boc*-1,6-hexanediamine sur la 4-chloro-imidazo[1,2-a]quinoxaline. Rdt : 89%. Mw : 383.49 g/mol. 1H-RMN δ (ppm, 400 MHz, DMSO d6) 1.29 to 1.40 (m, 15H, 3 x CH3 tBu, *CH2*CH2CH2NH, *CH2*CH2CH2NHBoc, *CH2*CH2NHBoc), 1.64 (qt, 2H, *CH2*CH2NH, J=8Hz), 2.91 (qd, 2H, *CH2*NHBoc, J=4Hz), 3.56 (qd, 2H, *CH2*NH, J=4Hz), 6.76 (t, 1H, *NH*Boc, J=4Hz), 7.27 (t, 1H, CH 7, J=8Hz), 7.37 (t, 1H, CH 8, J=8Hz), 7.57 (d, 1H, CH 9, J=8Hz), 7.61 (d, 1H, CH 2, J=4Hz), 7.68 (t, 1H, NH, J=4Hz), 8.07 (d, 1H, CH 6, J=8Hz), 8.58 (d, 1H, CH 1, J=4Hz). 13C-RMN δ (ppm, 100 MHz, DMSO d6) 26.55 and 26.73 (*CH2*CH2CH2NH, *CH2*CH2CH2NHBoc), 28.73 (CH3 tBu), 29.24 (*CH2*CH2NH), 29.95 (*CH2*CH2NHBoc), 40.00 (*CH2*NHBoc, *CH2*NH), 77.73 (Cq tBu), 114.96 (CH 1), 115.78 (CH 6), 122.88 (CH 7), 124.66 (Cq 5'), 126.59 (CH 9), 126.74 (CH 8), 132.15 (CH 2), 132.93 (Cq 3'), 137.42 (Cq 9'), 147.74 (Cq 4), 156.04 (C=O carbamate). MS (ESI +, QTof, m/z) : 384.24 [M+H]⁺

### tert-butyl 4-(imidazo[1,2-a]quinoxalin-4-yl)pipérazine-1-carboxylate

Addition de la 1-*Boc*-pipérazine sur la 4-chloro-imidazo[1,2-a]quinoxaline. Rdt : 63%. Mw : 353.42 g/mol. 1H-RMN δ (ppm, 400 MHz, DMSO d6) 1.44 (s, 9H, 3 x CH3 tBu), 3.51 (m, 4H, 2 x *CH2*NBoc), 4.30 (m, 4H, 2 x *CH2*NC=N), 7.33 (t, 1H, CH 7, J=8Hz), 7.44 (t, 1H, CH 8, J=8Hz), 7.60 (d, 1H, CH 9, J=8Hz), 7.69 (d, 1H, CH 2, J=4Hz), 8.14 (d, 1H, CH 6, J=8Hz), 8.68 (d, 1H, CH 1, J=4Hz). 13C-RMN δ (ppm, 100 MHz, DMSO d6) 28.54 (CH3 tBu), 44.5 (*CH2*NBoc), 46.06 (*CH2*NC=N), 79.52 (Cq tBu), 114.82 (CH 1), 115.67 (CH 6), 124.01 (CH 7), 124.98 (Cq 5'), 126.97 (CH 9, CH 8), 136.03 (CH 2), 132.52 (Cq 3'), 136.03 (Cq 9'), 147.53 (Cq 4), 154.44 (C=O carbamate). MS (ESI +, QTof, m/z) : 354.19 [M+H]⁺

### tert-butyl (3-(imidazo[1,2-a]quinoxalin-4-ylamino)propyl)carbamate

Addition de la *N-Boc*-1,3-diaminopropane sur la 4-chloro-imidazo[1,2-a]quinoxaline. Rdt : 93%. Mw : 341.41 g/mol. 1H-RMN δ (ppm, 400 MHz, DMSO d6) 1.37 (s, 9H, 3 x CH3 tBu), 1.74 (m, 2H, *CH2*CH2NHBoc), 3.02 (qd, 2H, *CH2*NHBoc, J=4Hz), 3.56 (qd, 2H, *CH2*NH, J=4Hz), 6.93 (t, 1H, *NH*Boc, J=4Hz), 7.26 (t, 1H, CH 7, J=8Hz), 7.37 (t, 1H, CH 8, J=8Hz), 7.58 (d, 1H, CH 9, J=8Hz), 7.60 (s, 1H, CH 2, J=4Hz), 7.70 (t, 1H, NH, J=4Hz), 8.08 (d, 1H, CH 6, J=8Hz), 8.59 (s, 1H, CH 1, J=4Hz). 13C-RMN δ (ppm, 100 MHz, DMSO d6) 28.72 (CH3 tBu), 29.77 (*CH*2CH2NHBoc), 37.76 (CH2NH), 37.94 (*CH*2NHBoc), 77.92 (Cq tBu), 115.04 (CH 1), 115.83 (CH 6), 123.03 (CH 7), 124.70 (Cq 5'), 126.50 (CH 9), 126.76 (CH 8), 132.23 (CH 2), 132.53 (Cq 3'), 137.26 (Cq 9'), 147.79 (Cq 4), 156.11 (C=O carbamate). MS (ESI +, QTof, m/z) : 342.19 [M+H]⁺

### 2-(acétoxyméthyl)-2-(2-(imidazo[1,2-a]quinoxalin-4-ylamino)acétamido)propane-1,3-diyl diacétate

Addition du 2-(acétoxyméthyl)-2-(2-aminoacétamido)propane-1,3-diyl diacétate sur la 4-chloro-imidazo[1,2-a]quinoxaline. Rdt : 44%. Mw : 471.46 g/mol. 1H-RMN δ (ppm, 400 MHz, DMSO d6) 1.95 (s, 6H, 3 x *CH2*OAc), 4.14 (d, 2H, CH2 Gly, J=8Hz), 4.30 (s, 9H, 3 x CO*CH3*), 7.32 (t, 1H, CH 7, J=8Hz), 7.42 (t, 1H, CH 8, J=8Hz), 7.56 (d, 1H, CH 9, J=8Hz), 7.60 (d, 1H, CH 2, J=4Hz), 7.66 (t, 1H, NH, J=4Hz), 8.00 (s, 1H, NH), 8.12 (d, 1H, CH 6, J=8Hz), 8.64 (s, 1H, CH 1, J=4Hz). 13C-RMN δ (ppm, 100 MHz, DMSO d6) 20.92 (*CH2*OAc), 44.15 (CH2 Gly), 57.41 (Cq Tris), 62.35 (CO*CH3*), 115.18 (CH 1), 115.94 (CH 6), 123.64 (CH 7), 124.98 (Cq 5'), 126.66 (CH 9), 126.86 (CH 8), 132.53 (CH 2), 132.63 (Cq 3'), 136.84 (Cq 9'), 147.46 (Cq 4), 170.24 (C=O amide), 170.46 (3 x C=O ester). MS (ESI +, QTof, m/z) : 472.18 [M+H]⁺

### Procédure pour l'addition d'une fonction amine d'un acide α-aminé.

Le 4-chloroimidazo[1,2-*a*]quinoxaline, l'acide aminé (4 éq.) la diisopropyléthylamine (4 éq.) sont solubilisés dans le diméthylformamide (12 mL) dans un vial adapté pour les réactions sous micro-ondes et équipé d'un barreau aimanté. Le mélange est scellé puis homogénéisé aux ultrasons avant d'être placé aux micro-ondes durant 20 min à 150°C. La puissance délivrée s'ajuste automatiquement à 45 Watts environ. Le diméthylformamide est évaporé sous pression réduite. Le milieu réactionnel est repris dans de l'acétate d'éthyle, lavé par une solution saturée de chlorure d'ammonium, puis par de l'eau distillée, et enfin par une solution saturée de chlorure de sodium. La phase organique est séchée sur sulfate de magnésium, filtrée et concentrée. Le brut réactionnel est purifié par chromatographie sur colonne de gel de silice afin d'obtenir le composé d'addition attendu.

### tert-butyl 2-(imidazo[1,2-a]quinoxalin-4-ylamino)acétate

Rdt : 42%. Mw : 298.34 g/mol. 1H-RMN δ (ppm, 400 MHz, DMSO d6) 1.42 (s, 9H, 3 x CH3 tBu), 4.12 (d, 2H, CH2 Gly, J=8Hz), 7.31 (t, 1H, CH 7, J=8Hz), 7.35 (t, 1H, CH 8, J=8Hz), 7.52 (t, 1H, CH 9, J=8Hz), 7.66 (s, 1H, CH 2), 7.95 (t, 1H, NH, J=8Hz), 8.14 (d, 1H, CH 6, J=8Hz), 8.64 (s, 1H, CH 1). MS (ESI +, QTof, m/z): 299.0 [M+H]⁺

### Procédure générale de bromation

Le composé issu de l'addition d'une amine en position 4 et le N-bromosuccinimide (1.3 éq.) sont solubilisés dans le chloroforme à température ambiante et sous agitation. Le milieu réactionnel est porté à reflux durant 1h30. Après être revenu à température ambiante, une solution aqueuse de bicarbonate de sodium à 5% est ajoutée au milieu réactionnel. La phase aqueuse est extraite trois fois par du dichlorométhane. Les phases organiques sont réunies, séchées sur sulfate de sodium, filtrées et concentrées sous pression réduite. Le produit obtenu est utilisé sans purification supplémentaire, la réaction est quantitative.

### 1-Bromo-imidazo[1,2-a]quinoxalin-4-amine

Mw : 263.09 g/mol. ¹H NMR (300 MHz, DMSO-d₆) *δ* : 7.95 (d, 1H), 7.75 (m, 2H), 7.71 (m, 1H), 7.68 (m, 1H), 6.65 (br s, 2H); ¹³ C (300 MHz, DMSO-d₆) δ : 142.20, 138.78, 129.66, 128.16, 127.17, 126.95, 126.18, 125.51, 113.98, 99.27.

### 1-Bromo-N-méthylimidazo[1,2-a]quinoxalin-4-amine (CPA8)

Mw : 277.12 g/mol. ¹H NMR (300 MHz, DMSO-d₆) *δ* : 3.25 (d, 3H), 6.15 (s, 1H), 7.25 (t, 1H), 7.40 (t, 1H), 7.65 (dd, 1H), 7.75 (dd, 1H), 9.10 (s, 1H). ¹³ C (300 MHz, DMSO-d₆) *δ* : 29.20, 101.40, 113.86, 125.13, 126.03, 127.09, 127.78, 128.96, 138.85, 140.54, 141.97.

### tert-butyl (2-((1-bromoimidazo[1,2-a]quinoxalin-4-yl)amino)éthyl)carbamate

Mw : 406.28 g/mol. 1H-RMN δ (ppm, 400 MHz, DMSO d6) 1.36 (s, 9H, 3 x CH3 tBu), 3.26 (m, 2H, *CH2*NHBoc), 3.59 (m, 2H, *CH2*NH), 6.98 (m, 1H, *NH*Boc), 7.30 (t, 1H, CH 7, J=8Hz), 7.35 (t, 1H, CH 8, J=8Hz), 7.61 (d, 1H, CH 9, J=8Hz), 7.72 (m, 2H, CH 2, NH), 8.96 (d, 1H, CH 6, J=8Hz). 13C-RMN δ (ppm, 100 MHz, DMSO d6) 28.68 (CH3 tBu), 40.00 (*CH2*NHBoc, *CH2*NH), 78.09 (Cq tBu), 99.35 (Cq 1), 114.98 (CH 6), 122.66 (CH 7), 125.91 (Cq 5'), 127.34 (CH 8, CH 9), 134.20 (CH 2), 134.35 (Cq 3'), 138.03 (Cq 9'), 147.35 (Cq 4), 156.24 (C=O carbamate). MS (ESI +, QTof, m/z) : 406.08 [M+H]⁺

### tert-butyl (6-((1-bromoimidazo[1,2-a]quinoxalin-4-yl)amino)hexyl)carbamate

Mw : 462.38 g/mol. 1H-RMN δ (ppm, 400 MHz, DMSO d6) 1.29 to 1.40 (m, 15H, 3 x CH3 tBu, *CH2*CH2CH2NH, *CH2*CH2CH2NHBoc, *CH2*CH2NHBoc), 1.63 (m, 2H, *CH2*CH2NH), 2.87 (qd, 2H, *CH2*NHBoc, J=4Hz), 3.51 (qd, 2H, *CH2*NH, J=4Hz), 6.76 (t, 1H, *NH*Boc, J=4Hz), 7.28 (t, 1H, CH 7, J=8Hz), 7.42 (t, 1H, CH 8, J=8Hz), 7.58 (d, 1H, CH 9, J=8Hz), 7.63 (s, 1H, CH 2), 7.74 (m, 1H, NH), 8.94 (d, 1H, CH 6, J=8Hz). 13C-RMN δ (ppm, 100 MHz, DMSO d6) 26.53 and 26.69 (*CH2*CH2CH2NH, *CH2*CH2CH2NHBoc), 28.73 (CH3 tBu), 29.14 (*CH2*CH2NH), 29.93 (*CH2*CH2NHBoc), 40.00 (*CH2*NHBoc, *CH2*NH), 77.73 (Cq tBu), 99.39 (Cq 1), 114.96 (CH 6), 122.45 (CH 7), 125.73 (Cq 5'), 127.31 (CH 8, CH 9), 134.20 (Cq 3'), 134.50 (CH 2), 134.64 (Cq 9'), 149.14 (Cq 4), 156.03 (C=O carbamate). MS (ESI +, QTof, m/z) : 462.15 [M+H]⁺

### tert-butyl 4-(1-bromoimidazo[1,2-a]quinoxalin-4-yl)pipérazine-1-carboxylate

Mw : 432.31 g/mol. 1H-RMN δ (ppm, 400 MHz, DMSO d6) 1.38 (s, 9H, 3 x CH3 tBu), 3.45 (m, 4H, 2 x *CH2*NBoc), 4.15 (m, 4H, 2 x *CH2*NC=N), 7.28 (t, 1H, CH 7, J=8Hz), 7.44 (t, 1H, CH 8, J=8Hz), 7.54 (d, 1H, CH 9, J=8Hz), 7.70 (d, 1H, CH 2, J=4Hz), 8.93 (d, 1H, CH 6, J=8Hz). 13C-RMN δ (ppm, 100 MHz, DMSO d6) 28.53 (CH3 tBu), 42.5 (*CH2*NBoc), 46.42 (*CH2*NC=N), 79.54 (Cq tBu), 99.64 (Cq 1), 114.97 (CH 6), 123.49 (CH 7), 126.11 (Cq 5'), 127.47 (CH 9, CH 8), 134.29 (CH 2), 134.66 (Cq 3'), 136.85 (Cq 9'), 147.33 (Cq 4), 154.43 (C=O carbamate). MS (ESI +, QTof, m/z) : 432.10 [M+H]⁺

### tert-butyl (3-((1-bromoimidazo[1,2-a]quinoxalin-4-yl)amino)propyl)carbamate

Mw : 420.30 g/mol. 1H-RMN δ (ppm, 400 MHz, DMSO d6) 1.38 (s, 9H, 3 x CH3 tBu), 1.74 (m, 2H, *CH2*CH2NHBoc), 3.00 (qd, 2H, *CH2*NHBoc, J=4Hz), 3.56 (qd, 2H, *CH2*NH, J=4Hz), 6.90 (m, 1H, *NH*Boc), 7.54 (m, 3H, CH 7, CH 8, CH 9), 7.73 (s, 1H, CH 2, J=4Hz), 7.97 (m, 1H, NH), 9.04 (d, 1H, CH 6, J=8Hz). 13C-RMN δ (ppm, 100 MHz, DMSO d6) 28.71 (CH3 tBu), 29.59 (*CH2*CH2NHBoc), 37.93 (*CH2*NH, *CH2*NHBoc), 77.95 (Cq tBu), 100.04 (Cq 1), 117.42 (CH 6), 126.66 (CH 7), 127.42 (Cq 5'), 128.59 (CH 8), 130.02 (CH 9), 134.03 (Cq 3'), 134.73 (CH 2), 137.24 (Cq 9'), 147.45 (Cq 4), 156.11 (C=O carbamate). MS (ESI +, QTof, m/z) : 420.10 [M+H]⁺

### 1-bromo-N-méthyl-7-(trifluorométhyl)imidazo[1,2-a]quinoxalin-4-amine

Mw : 345.12 g/mol. 1H-RMN δ (ppm, 400 MHz, DMSO d6) 3.04 (d, 3H, *CH3*NH, J=8 Hz), 7.65 (m, 2H, CH 8), 7.78 (s, 1H, CH 2), 7.88 (s, 1H, CH 6), 8.11 (m, 1H, *NH*CH3), 9.12 (d, 1H, CH 9, J=8 Hz). 19F-RMN δ (ppm, 400 MHz, DMSO d6) -60.42.

### 1-bromo-N-méthyl-7-(trifluorométhoxy)imidazo[1,2-a]quinoxalin-4-amine

Mw : 361.12 g/mol. 1H-RMN δ (ppm, 400 MHz, DMSO d6) 3.02 (d, 3H, *CH3*NH, J=8 Hz), 7.30 (d, 1H, CH 8, J=8Hz), 7.51 (s, 1H, CH 6), 7.73 (s, 1H, CH 2), 8.05 (m, 1H, *NH*CH3), 9.02 (d, 1H, CH 9, J=8Hz). 13C-RMN δ (ppm, 100 MHz, DMSO d6) 27.70 (*CH3*NH), 99.85 (Cq 1), 114.79 (CH 8), 116.88 (CH 9), 119.34 (CH 6), 124.80 (Cq 5'), 134.19 (CH 2), 134.71 (Cq 3'), 139.55 (Cq 9'), 146.82 (Cq 7), 148.03 (Cq 4). 19F-RMN δ (ppm, 400 MHz, DMSO d6) -56.77. MS (ESI +, QTof, m/z) : 360.99 [M+H]⁺

### 1-bromo-N-méthyl-8-(trifluorométhyl)imidazo[1,2-a]quinoxalin-4-amine

Mw: 345.12 g/mol. 1H-RMN δ (ppm, 400 MHz, DMSO d6) 3.04 (d, 3H, *CH3*NH, J=8Hz), 7.75 (m, 3H, CH 2, CH 6, CH 7), 8.21 (m, 1H, NH), 9.24 (s, 1H, CH 9). 13C-RMN δ (ppm, 100 MHz, DMSO d6) 27.73 (*CH3*NH), 100.32 (Cq 1), 112.26 (CH 9), 123.63 (CH 7), 125.58 (Cq 5'), 127.81 (CH 6), 134.25 (Cq 3'), 134.83 (CH 2), 141.44 (Cq 9'), 149.02 (Cq 4). 19F-RMN δ (ppm, 100 MHz, DMSO d6) -59.89 (CF3).

### tert-butyl 2-((1-bromoimidazo[1,2-a]quinoxalin-4-yl)amino)acétate

Mw : 377.24 g/mol. 1H-RMN δ (ppm, 400 MHz, DMSO d6) 1.37 (s, 9H, 3 x CH3 tBu), 4.11 (d, 2H, CH2 Gly, J=8Hz), 7.34 (t, 1H, CH 7, J=8Hz), 7.48 (t, 1H, CH 8, J=8Hz), 7.61 (t, 1H, CH 9, J=8Hz), 7.76 (s, 1H, CH 2), 8.02 (m, 1H, NH), 8.96 (d, 1H, CH 6, J=8Hz). MS (ESI +, QTof, m/z): 377.0 [M+H]⁺

### Procédure générale pour la réaction de couplage croisé de Suzuki

La réaction de couplage croisé de l'intermédiaire bromé avec l'acide arylboronique correspondant (1,1 éq.) en présence de catalyseur de palladium tétrakistriphénylphosphine est effectuée sous conditions basiques Na₂CO₃ (2 éq.), dans un mélange DME (10 mL) - H₂O (5 mL) et sous assistance micro-ondes (140°C, 20 min). Le milieu réactionnel est filtré sur célite et lavé par de l'éthanol. Le filtrat est concentré puis purifié par chromatographie liquide sur gel de silice permettant l'obtention des composés désirés purs.

### 1-(3,4-diméthoxyphényl)-N-méthylimidazo[1,2-a]quinoxalin-4-amine (EAPB 02203) composé de référence

Couplage de la 1-bromo-*N*-méthylimidazo[1,2-*a*]quinoxalin-4-amine avec l'acide 3,4-diméthoxyphényl boronique (218 mg, 1.2 mmol). Rendement 30%. ¹H NMR (300 MHz, CDCl₃) *δ* : 7.78 (d, 1H), 7.38-7.29 (m, 3H), 7.10-6.91 (m, 4H), 6.28 (br s, 1H), 3.96 (s, 3H), 3.85 (s, 3H), 3.29 (s, 3H). ¹³C NMR (300 MHz, CDCl₃) *δ* : 152.61, 148.06, 142.74, 139.57, 131.53, 131.13, 129.56, 129.02, 127.60, 124.90, 124.47, 124.02, 122.1, 119.29, 116.74, 114.96, 110.49, 55.87, 29.20.

### 1-(3,5-diméthoxyphényl)-N-méthylimidazo[1,2-a]quinoxalin-4-amine (EAPB 01803) composé de référence

Couplage de la 1-bromo-*N*-méthylimidazo[1,2-*a*]quinoxalin-4-amine avec l'acide 3,5-diméthoxyphényl boronique (218 mg, 1.2 mmol). Rendement 40%. ¹H NMR (300 MHz, CDCl₃) *δ* : 7.79 (d, 1H), 7.42-7.39 (m, 2H), 7.32 (td, 1H), 6.97 (td, 1H), 6.64-6.60 (m, 3H), 6.29 (br s, 1H), 3.29 (s, 3H). ¹³C NMR (300 MHz, CDCl₃) *δ* : 163.62, 142.86, 139.57, 131.13, 130.54, 130.05, 129.02, 124.90, 124.47, 123.08, 114.96, 101.33, 98.38, 55.33, 29.26.

### 1-(3,4-diméthoxyphényl)imidazo[1,2-a]quinoxalin-4-amine (EAPB 02202) composé de référence

Couplage de la 1-bromoimidazo[1,2-*a*]quinoxalin-4-amine (262 mg, 0.997 mmol) avec l'acide 3,4-diméthoxyphényl boronique (199 mg, 1.096 mmol). Rendement 15%. ¹H NMR (300 MHz, CDCl₃) *δ* : 7.45-7.35 (m, 3H), 7.28 (d, 1H), 7.09 (d, 1H), 7.05-7.01 (m, 1H), 6.85-6.79 (m, 1H), 3.97 (s, 3H), 3.89 (s, 3H), 3.60 (s, 3H), 1.24 (s, 3H).

### N-méthyl-1-(3,4,5-triméthoxyphényl)imidazo[1,2-a]quinoxalin-4-amine (EAPB 02703) composé de référence

Couplage de la 1-bromo-*N*-méthylimidazo[1,2-*a*]quinoxalin-4-amine avec l'acide 3,4,5-triméthoxyphényl boronique (365 mg, 2.17 mmol). Rendement 28%. ¹H NMR (300 MHz, CDCl₃) *δ* : 7.93-7.88 (m, 1H), 7.56-7.34 (m, 3H), 7.01 (t, 1H), 6.70 (s, 2H), 3.96 (s, 3H), 3.84 (s, 6H), 3.37 (br s, 3H). ¹³C NMR (300 MHz, CDCl₃) *δ* : 155.74, 142.86, 139.57, 136.68, 131.13, 129.38, 129.24, 129.02, 124.90, 124.75, 124.47, 123.08, 114.96, 103.91, 60.55, 56.22, 29.23.

### tert-butyl (2-((1-(3,4-diméthoxyphényl)imidazo[1,2-a]quinoxalin-4-yl)amino)éthyl)carbamate (EAPB 02211) composé de référence

Couplage du tert-butyl (2-((1-bromoimidazo[1,2-a]quinoxalin-4-yl)amino)éthyl)carbamate avec l'acide 3,4-diméthoxyphényl boronique. Rdt : 57%. Mw : 463.53 g/mol. 1H-RMN δ (ppm, 400 MHz, DMSO d6) 1.38 (s, 9H, 3 x CH3 tBu), 3.27 (qd, 2H, CH2NHBoc, J=8Hz), 3.63 (qd, 2H, CH2NH, J=8Hz), 3.74 (s, 3H, OCH3), 3.87 (s, 3H, OCH3), 6.98 (m, 2H, *NH*Boc, CH 7), 7.16 (m, 3H, CH 2', CH 5', CH 6'), 7.30 (m, 2H, CH 8, CH 6), 7.49 (s, 1H, CH 2), 7.58 (d, 2H, CH 9, J=8Hz), 7.71 (t, 1H, NH, J=4Hz). 13C-RMN δ (ppm, 100 MHz, DMSO d6) 28.70 (CH3 tBu), 40.00 (*CH2*NHBoc, *CH2*NH), 56.05 (OCH3), 56.15 (OCH3), 78.10 (Cq tBu), 112.30 (CH 2'), 114.19 (CH 5'), 115.88 (CH 6), 122.35 (Cq 1' phényl), 122.76 (CH 7), 123.26 (CH 6'), 125.84 (Cq 5'), 126.49 (CH 8), 127.09 (CH 9), 130.85 (Cq 1), 132.42 (CH 2), 133.30 (Cq 3' quinoxaline), 138.06 (Cq 9'), 148.00 (Cq 3' phényl), 149.25 (Cq 4' phényl), 150.10 (Cq 4), 156.25 (C=O carbamate). MS (ESI +, QTof, m/z) : 464.23 [M+H]⁺

### tert-butyl (6-((1-(3,4-diméthoxyphényl)imidazo[1,2-a]quinoxalin-4-yl)amino)hexyl)carbamate (EAPB 02212) composé de référence

Couplage du tert-butyl (6-((1-bromoimidazo[1,2-a]quinoxalin-4-yl)amino)hexyl)carbamate avec l'acide 3,4-diméthoxyphényl boronique. Rdt : 61%. Mw : 519.64 g/mol. 1H-RMN δ (ppm, 400 MHz, DMSO d6) 1.31 to 1.40 (m, 15H, 3 x CH3 tBu, *CH2*CH2CH2NH, *CH2*CH2CH2NHBoc, *CH2*CH2NHBoc), 1.66 (qt, 2H, *CH2*CH2NH, J=8Hz), 2.91 (qd, 2H, *CH2*NHBoc, J=4Hz), 3.53 (qd, 2H, *CH2*NH, J=4Hz), 3.74 (s, 3H, OCH3), 3.87 (s, 3H, OCH3), 6.77 (t, 1H, *NH*Boc, J=4Hz), 6.95 (t, 1H, CH 7, J=8Hz), 7.10 (m, 3H, CH 2', CH 5', CH 6'), 7.28 (m, 2H, CH 6, CH8), 7.47 (s, 1H, CH 2), 7.57 (d, 1H, CH 9, J=8Hz), 7.67 (t, 1H, NH, J=4Hz). 13C-RMN δ (ppm, 100 MHz, DMSO d6) 26.5 and 26.72 (*CH2*CH2CH2NH, *CH2*CH2CH2NHBoc), 28.74 (CH3 tBu), 29.30 (*CH2*CH2NH), 29.97 (*CH2*CH2NHBoc), 40.00 (CH2NHBoc, *CH2*NH), 56.04 (OCH3), 56.14 (OCH3) 77.74 (Cq tBu), 112.28 (CH 2'), 114.23 (CH 5'), 115.85 (CH 6), 122.05 (CH 7), 122.84 (Cq 1'), 123.27 (CH 6'), 125.73 (Cq 5' quinoxaline), 126.44 (CH 8), 127.05 (CH 9), 130.79 (Cq 1), 132.31 (CH 2), 133.35 (Cq 3' quinoxaline), 138.31 (Cq 9'), 147.96 (Cq 3' phényl), 149.24 (Cq 4' phényl), 150.08 (Cq 4), 156.05 (C=O carbamate). MS (ESI +, QTof, m/z) : 520.29 [M+H]⁺

### tert-butyl 4-(1-(3,4-diméthoxyphényl)imidazo[1,2-a]quinoxalin-4-yl)pipérazine-1-carboxylate (EAPB 02213) composé de référence

Couplage du tert-butyl 4-(1-bromoimidazo[1,2-a]quinoxalin-4-yl)pipérazine-1-carboxylate avec l'acide 3,4-diméthoxyphényl boronique. Rdt : 85%. Mw : 489.57 g/mol. 1H-RMN δ (ppm, 400 MHz, DMSO d6) 1.45 (s, 9H, 3 x CH3 tBu), 3.53 (m, 4H, 2 x *CH2*NBoc), 3.73 (s, 3H, *OCH3*), 3.87 (s, 3H, *OCH3),* 4.29 (m, 4H, 2 x *CH2*NC=N), 7.05 (t, 1H, CH 7, J=8Hz), 7.10 (d, 1H, CH 6'), 7.15 (m, 2H, CH 2', CH 5'), 7.30 (d, 1H, CH 6, J=8Hz), 7.35 (t, 1H, CH 8, J=8Hz), 7.56 (s, 1H, CH 2), 7.63 (d, 1H, CH 9, J=8Hz). 13C-RMN δ (ppm, 100 MHz, DMSO d6) 28.56 (CH3 tBu), 43.00 (*CH2*NBoc), 46.40 (*CH2*NC=N), 56.07 (OCH3), 56.16 (OCH3), 79.54 (Cq tBu), 112.36 (CH 5'), 114.12 (CH 2'), 115.81 (CH 6), 122.79 (CH 7), 123.23 (CH 6', Cq 1'), 125.73 (Cq 5' quinoxaline), 126.44 (CH 8), 127.35 (CH 9), 130.36 (Cq 1), 132.51 (CH 2), 133.35 (Cq 3' quinoxaline), 136.91 (Cq 9'), 147.94 (Cq 3' phényl), 149.33 (Cq 4' phényl), 150.18 (Cq 4), 154.46 (C=O carbamate). MS (ESI +, QTof, m/z) : 490.25 [M+H]⁺

### tert-butyl (3-((1-(3,4-diméthoxyphényl)imidazo[1,2-a]quinoxalin-4-yl)amino)propyl)carbamate (EAPB 02214) composé de référence

Couplage du tert-butyl (3-((1-bromoimidazo[1,2-a]quinoxalin-4-yl)amino)propyl)carbamate avec l'acide 3,4-diméthoxyphényl boronique. Rdt : 69%. Mw : 477.56 g/mol. 1H-RMN δ (ppm, 400 MHz, DMSO d6) 1.36 (s, 9H, 3 x CH3 tBu), 1.77 (m, 1H, CH2CH2NHBoc), 3.02 (qd, 2H, CH2NHBoc, J=4Hz), 3.56 (qd, 2H, *CH2*NH, J=4Hz), 3.74 (s, 3H, *OCH3),* 3.89 (s, 3H, *OCH3),* 6.97 (m, 2H, CH 7, NHBoc), 7.17 (m, 3H, CH 6', CH 2', CH 5'), 7.29 (m, 2H, CH 6, CH 8), 7.49 (s, 1H, CH 2), 7.61 (m, 1H, CH 9). 13C-RMN δ (ppm, 100 MHz, DMSO d6) 28.73 (CH3 tBu), 29.80 (CH2CH2NHBoc), 37.91 (CH2NHBoc, *CH2*NH), 56.05 (OCH3), 56.15 (OCH3), 77.95 (Cq tBu), 112.30 (CH 5'), 114.22 (CH 2'), 115.92 (CH 6), 122.27 (Cq 1'), 123.28 (CH 7, CH 6'), 125.73 à 127.93 (CH 8, CH 9, Cq 5' quinoxaline), 130.85 (Cq 1), 132.43 (CH 2), 133.13 (Cq 3' quinoxaline), 139.56 (Cq 9'), 147.95 (Cq 3' phényl), 149.25 (Cq 4' phényl), 150.11 (Cq 4), 156.12 (C=O carbamate). MS (ESI +, QTof, m/z) : 478.25 [M+H]⁺

### N-(1,3-dihydroxy-2-(hydroxyméthyl)propan-2-yl)-2-((1-(3,4-diméthoxyphényl)imidazo[1,2-a]quinoxalin-4-yl)amino)acétamide (EAPB 02210) composé de référence

Couplage du 2-(acétoxyméthyl)-2-(2-((1-bromoimidazo[1,2-a]quinoxalin-4-yl)amino)acétamido)propane-1,3-diyl diacétate avec l'acide 3,4-diméthoxyphényl boronique. Rdt : 16%. Mw : 481.50 g/mol. MS (ESI +, QTof, m/z) : 482.20 [M+H]⁺

### 1-(3,4-diméthoxyphényl)-N-méthyl-7-(trifluorométhyl)imidazo[1,2-a]quinoxalin-4-amine (EAPB 02203-7a) composé de référence

Couplage du 1-bromo-N-méthyl-7-(trifluorométhyl)imidazo[1,2-a]quinoxalin-4-amine avec l'acide 3,4-diméthoxyphényl boronique. Rdt: 13%. Mw : 402.37 g/mol. 1H-RMN δ (ppm, 400 MHz, DMSO d6) 3.06 (d, 3H, *CH3*NH, J=8 Hz), 7.15 (m, 2H, CH 5', CH 6'), 7.20 (s, 1H, CH 2'), 7.33 (d, 1H, CH 8, J=8Hz), 7.45 (d, 1H, CH 9, J=8 Hz), 7.54 (s, 1H, CH 2), 7.85 (s, 1H, CH 6), 8.05 (m, 1H, *NH*CH3). 13C-RMN δ (ppm, 100 MHz, DMSO d6) 27.72 (*CH3*NH), 56.03 (OCH3), 56.16 (OCH3), 112.37 (CH 5'), 114.01 (CH 2'), 117.09 (CH 9), 118.97 (CH 8), 122.18 (Cq 1'), 122.88 (CH 6, CH 6'), 125.85 (Cq 5'), 131.29 (Cq 1), 132.82 (CH 2), 133.01 (Cq 3'), 137.53 (Cq 9'), 138.47 (Cq 7), 149.27 (Cq 3', Cq 4'), 150.22 (Cq 4). 19F-RMN δ (ppm, 400 MHz, DMSO d6). MS (ESI +, QTof, m/z) : 403.00 [M+H]⁺

### 1-(3,4-diméthoxyphényl)-N-méthyl-7-(trifluorométhoxy)imidazo[1,2-a]quinoxalin-4-amine (EAPB 02203-7b) composé de référence

Couplage du 1-bromo-N-méthyl-7-(trifluorométhoxy)imidazo[1,2-a]quinoxalin-4-amine avec l'acide 3,4-diméthoxyphényl boronique. Rdt : 67%. Mw : 418.37 g/mol. 1H-RMN δ (ppm, 400 MHz, DMSO d6) 3.08 (d, 3H, *CH3*NH, J=8 Hz), 3.77 (s, 3H, OCH3), 3.88 (s, 3H, OCH3), 7.01 (d, 1H, CH 8, J=8Hz), 7.12 (d, 1H, CH 6', J=8Hz), 7.17 (d, 1H, CH 5', J=8Hz), 7.20 (s, 1H, CH 2'), 7.36 (d, 1H, CH 9), 7.48 (s, 1H, CH 6), 7.51 (d, 1H, CH 2, J=4Hz), 8.02 (d, 1H, *NH*CH3, J=4Hz). 13C-RMN δ (ppm, 100 MHz, DMSO d6) 27.75 (*CH3*NH), 56.01 (OCH3), 56.14 (OCH3), 112.34 (CH 5'), 114.14 (CH 2'), 114.59 (CH 8), 117.23 (CH 9), 117.97 (CH 6), 122.35 (Cq 1'), 123.29 (CH 6'), 124.01 (Cq 5'), 131.01 (Cq 1), 132.56 (CH 2), 133.24 (Cq 3'), 139.82 (Cq 9'), 146.29 (Cq 7), 149.27 et 149.36 (Cq 4' phényl, Cq 3' phényl), 150.20 (Cq 4). 19F-RMN δ (ppm, 100 MHz, DMSO d6) -56.86 (OCF3). MS (ESI +, QTof, m/z) : 419.20 [M+H]⁺

### 1-(3,4-diméthoxyphényl)-N-méthyl-7-(carbonitrile)imidazo[1,2-a]quinoxalin-4-amine (EAPB 02203-7c) composé de référence

Couplage du 1-bromo-N-méthyl-7-(carbonitrile)imidazo[1,2-a]quinoxalin-4-amine avec l'acide 3,4-diméthoxyphényl boronique. Rdt < 10 %. Mw : 359.38 g/mol. 1H-RMN δ (ppm, 400 MHz, DMSO d6) 3.08 (d, 3H, *CH3*NH, J=8 Hz), 3.77 (s, 3H, OCH3), 3.88 (s, 3H, OCH3), 7.01 (d, 1H, CH 8, J=8Hz), 7.12 (d, 1H, CH 6', J=8Hz), 7.17 (d, 1H, CH 5', J=8Hz), 7.20 (s, 1H, CH 2'), 7.36 (d, 1H, CH 9), 7.48 (s, 1H, CH 6), 7.51 (d, 1H, CH 2, J=4Hz), 8.02 (d, 1H, *NH*CH3, J=4Hz). MS (ESI +, QTof, m/z) : 360.15 [M+H]⁺

### 1-(3,4-diméthoxyphényl)-N-méthyl-8-(trifluorométhyl)imidazo[1,2-a]quinoxalin-4-amine (EAPB 02203-8a) composé de référence

Couplage du 1-bromo-N-méthyl-8-(trifluorométhyl)imidazo[1,2-a]quinoxalin-4-amine avec l'acide 3,4-diméthoxyphényl boronique. Rdt : 21%. Mw: 402.37 g/mol. 1H-RMN δ (ppm, 400 MHz, DMSO d6) 2.94 (d, 3H, *CH3*NH), 3.75 (s, 3H, OCH3), 3.86 (s, 3H, OCH3), 7.16 (m, 2H, CH 5', CH 6'), 7.23 (s, 1H, CH 2'), 7.55 (s, 1H, CH 2), 7.59 (m, 2H, CH 7, CH 9), 7.72 (d, 1H, CH 6, J=8Hz), 8.16 (m, 1H, NH). 13C-RMN δ (ppm, 100 MHz, DMSO d6) 29.42 (CH3NH), 56.18 (OCH3), 56.24 (OCH3), 112.55 (CH 5'), 113.31 (CH 9), 114.22 (CH 2'), 122.12 (Cq 1'), 122.85 (CH 7), 123.46 (CH 6'), 125.11 (Cq 5'),127.53 (CH 6), 131.21 (Cq 1), 132.53 (CH 2), 133.36 (Cq 3' imidazole), 141.46 (Cq 9'), 142.5 (Cq 8), 149.55 (Cq 3' phényl, Cq 4' phényl), 150.47 (Cq 4). 19F-RMN δ (ppm, 100 MHz, DMSO d6) - 60.28. MS (ESI +, QTof, m/z) : 403.00 [M+H]⁺

### tert-butyl 2-((1-(3,4-diméthoxyphényl)imidazo[1,2-a]quinoxalin-4-yl)amino)acétate (EAPB 02216) composé de référence

Couplage du tert-butyl 2-((1-bromoimidazo[1,2-a]quinoxalin-4-yl)amino)acétate avec l'acide 3,4-diméthoxyphényl boronique. Rdt : 19%. Mw: 434.49 g/mol. 1H-RMN δ (ppm, 400 MHz, DMSO d6) 1.44 (s, 9H, 3 x *CH3 tbu*), 3.74 (s, 3H, OCH3), 3.87 (s, 3H, OCH3), 4.13 (d, 2H, CH2 Gly, J=8Hz), 7.01 (t, 1H, CH 7), 7.14 (m, 3H, CH 2', CH 5', CH 6'), 7.31 (m, 2H, CH 6, CH 8), 7.52 (s, 1H, CH 2), 7.54 (m, 1H, CH 9), 7.98 (m, 1H, NH). MS (ESI +, QTof, m/z) : 435.20 [M+H]⁺

### Procédure générale pour la réaction de déprotection

Le composé protégé est solubilisé dans du dichlorométhane. Sous atmosphère inerte à 0°C et sous forte agitation, le tribromure de bore est additionné (2 éq. par fonction à déprotéger). Une fois l'addition terminée, le milieu réactionnel est agité 1h30 à température ambiante. Il est ensuite hydrolysé par une solution saturée de bicarbonate de sodium à 0°C. La phase aqueuse est extraite trois fois avec du dichlorométhane, séchée sur sulfate de sodium, filtrée et concentrée. Le brut réactionnel est purifié par chromatographie sur colonne de silice permettant d'accéder aux produits attendus.

### 1-(3,4-dihydroxyphényl)imidazo[1,2-a]quinoxalin-4-amine (EAPB 02302)

Rdt 25 %. ¹H NMR (300 MHz, DMSO-d₆) *δ* : 9.41 (s, 1H), 9.31 (s, 1H), 7.53 (dd, 1H), 7.44 (s, 1H), 7.37-7.28 (m, 2H), 7.21 (m, 2H), 7.04-6.98 (m, 1H), 6.92-6.89 (m, 2H), 6.82 (dd, 1H). ¹³C NMR (300 MHz, DMSO-d₆) *δ* : 149.02, 146.58, 145.54, 137.52, 132.45, 131.85, 130.87, 125.93, 125.86, 125.61, 121.80, 121.55, 120.78, 117.39, 115.98, 115.40.

### 1-(3,4-dihydroxyphényl)-N-méthylimidazo[1,2-a]quinoxalin-4-amine (EAPB 02303)

Rdt 17%. ¹H NMR (300 MHz, DMSO-d6) *δ* : 7.65 (q, 1H), 7.58 (dd, 1H), 7.38-7.29 (m, 3H), 6.97 (m, 1H), 6.90-6.86 (m, 2H), 6.80 (dd, 1H), 3.04 (d, 3H). ¹³C NMR (300 MHz, DMSO-d6) *δ* : 148.03, 146.51, 145.48, 137.76, 132.76, 131.45, 130.59, 126.47, 125.84, 125.26, 121.55, 121.49, 120.78, 117.36, 115.92, 115.33, 27.22.

### 1-(3,5-dihydroxyphényl)-N-méthylimidazo[1,2-a]quinoxalin-4-amine (EAPB 01903) composé de référence

Rdt 33%. ¹H NMR (300 MHz, MeOD) *δ* : 7.71 (dd, 1H), 7.52 (dd, 1H), 7.41 (s, 1H), 7.36 (td, 1H), 7.04 (td, 1H), 6.46-6,40 (m, 3H), 3.22 (s, 3H).

### 4-(4-((2-aminoéthyl)amino)imidazo[1,2-a]quinoxalin-1-yl)benzène-1,2-diol (EAPB 02306)

Rdt : 94%. Mw : 335.36 g/mol. 1H-RMN δ (ppm, 400 MHz, DMSO d6) 3.14 (m, 2H, *CH2*NH2), 3.81 (m, 2H, *CH2*NH), 6.79 (d, 1H, CH 6', J=8Hz), 6.90 (s, 1H, CH 2'), 6.94 (d, 1H, CH 5', J=8Hz), 7.01 (t, 1H, CH 7, J=8Hz), 7.31 (m, 2H, CH 8, CH 6), 7.43 (s, 1H, CH 2), 7.60 (d, 1H, CH 9, J=8Hz), 7.87 (t, 1H, NH, J=4Hz). 13C-RMN δ (ppm, 100 MHz, DMSO d6) 40.00 (*CH2*NH2, *CH2*NH), 115.83 (CH 6), 116.52 (CH 5'), 117.86 (CH 2'), 121.05 (Cq 1'), 121.96 (CH 6'), 122.71 (CH 7), 125.95 (Cq 5'), 126.52 (CH 8), 127.13 (CH 9), 131.34 (Cq 1), 132.13 (CH 2), 133.02 (Cq 3' quinoxaline), 137.65 (Cq 9'), 146.04 (Cq 3' phényl), 147.10 (Cq 4' phényl), 148.13 (Cq 4). MS (ESI +, QTof, m/z) : 336.15 [M+H]⁺

### Chlorure de 2-((1-(3,4-dihydroxyphényl)imidazo[1,2-a]quinoxalin-4-yl)amino)éthylammonium (EAPB 02306s)

Mw : 371.82 g/mol. 1H-RMN δ (ppm, 400 MHz, DMSO d6) 3.18 (m, 2H, *CH2*NH3), 4.05 (m, 2H, *CH2*NH), 6.80 (d, 1H, CH 6', J=8Hz), 6.94 (s, 1H, CH 2'), 6.99 (d, 1H, CH 5', J=8Hz), 7.14 (t, 1H, CH 7, J=8Hz), 7.33 (m, 1H, CH 6), 7.39 (m, 1H, CH 8), 7.52 (s, 1H, CH 2), 7.60 (d, 1H, CH 9, J=8Hz), 8.05 (m, 1H, CH 9), 8.27 (m, 3H, NH3). 13C-RMN δ (ppm, 100 MHz, DMSO d6) 40.00 (*CH2*NH3, *CH2*NH), 116.22 (CH 6), 116.69 (CH 5'), 117.73 (CH 2'), 120.06 (Cq 1'), 122.03 (CH 6'), 124.34 (CH 7), 125.75 (Cq 5'), 127.19 (CH 8, CH 9), 132.23 (CH 2), 132.93 to 133.22 (Cq 1, Cq 3' quinoxaline, Cq 9'), 146.18 (Cq 3' phényl), 146.68 (Cq 4' phényl), 147.46 (Cq 4). MS (ESI +, QTof, m/z) : 336.15 [M+H]⁺

### 4-(4-((6-aminohexyl)amino)imidazo[1,2-a]quinoxalin-1-yl)benzène-1,2-diol (EAPB 02307)

Rdt : 81%. Mw: 391.47 g/mol. 1H-RMN δ (ppm, 400 MHz, DMSO d6) 1.39 (m, 4H, *CH2*CH2CH2NH, *CH2*CH2CH2NH2), 1.54 (m, 2H, *CH2*CH2NH2), 1.69 (m, 2H, *CH2*CH2NH), 2.76 (m, 2H, *CH2*NH2), 3.57 (m, 2H, *CH2*NH), 6.79 (d, 1H, CH 6', J=8Hz), 6.88 (s, 1H, CH 2'), 6.93 (d, 1H, CH 5', J=8Hz), 6.97 (t, 1H, CH 7), 7.29 (m, 2H, CH 6, CH8), 7.39 (s, 1H, CH 2), 7.54 (d, 1H, CH 9, J=8Hz), 7.64 (t, 1H, NH, J=4Hz). 13C-RMN δ (ppm, 100 MHz, DMSO d6) 26.08 and 26.53 (*CH2*CH2CH2NH, *CH2*CH2CH2NH2), 27.47 (*CH2*CH2NH2), 29.16 (*CH2*CH2NH), 40.00 (*CH2*NH2, *CH2*NH), 115.85 (CH 6), 116.46 (CH 5'), 117.89 (CH 2'), 121.26 (Cq 1'), 122.09 (CH 7, CH 6'), 125.75 (Cq 5'), 126.41 (CH 8), 126.99 (CH 9), 131.22 (Cq 1), 131.96 (CH 2), 133.14 (Cq 3'), 138.24 (Cq 9'), 146.02 (Cq 3' phényl), 147.05 (Cq 4' phényl), 147.97 (Cq 4). MS (ESI +, QTof, m/z) : 392.21 [M+H]⁺

### Chlorure de 6-((1-(3,4-dihydroxyphényl)imidazo[1,2-a]quinoxalin-4-yl)amino)hexan-1-ammonium (EAPB 02307s)

Mw: 427.93 g/mol. 1H-RMN δ (ppm, 400 MHz, DMSO d6) 1.39 (m, 4H, *CH2*CH2CH2NH, *CH2*CH2CH2NH2), 1.59 (m, 2H, *CH2*CH2NH2), 1.73 (m, 2H, *CH2*CH2NH), 2.77 (m, 2H, *CH2*NH2), 3.85 (m, 2H, *CH2*NH), 6.81 (d, 1H, CH 6', J=8Hz), 6.95 (s, 1H, CH 2'), 6.99 (d, 1H, CH 5', J=8Hz), 7.22 (t, 1H, CH 7, J=8Hz), 7.34 (d, 1H, CH 6, J=8Hz), 7.45 (t, 1H, CH 8 , J=8Hz), 7.68 (s, 1H, CH 2), 7.97 (m, 3H, NH3), 8.35 (m, 1H, CH 9). 13C-RMN δ (ppm, 100 MHz, DMSO d6) 25.95 and 26.03 (*CH2*CH2CH2NH, *CH2*CH2CH2NH2), 27.23 (*CH2*CH2NH2), 28.44 (*CH2*CH2NH), 40.00 (*CH2*NH2, *CH2*NH), 116.57 (CH 6), 116.71 (CH 5'), 117.61 (CH 2'), 119.60 (CH 9), 120.16 (Cq 1'), 121.82 (CH 6'), 124.19 (CH 7), 125.21 (Cq 5'), 127.52 (CH 8), 131.73 (Cq 1), 134.10 to 134.20 (CH 2, Cq 3' quinoxaline, Cq 9'), 144.88 (Cq 3' phényl), 146.27 (Cq 4' phényl), 147.69 (Cq 4). MS (ESI +, QTof, m/z) : 392.21 [M+H]⁺

### 4-(4-(pipérazin-1-yl)imidazo[1,2-a]quinoxalin-1-yl)benzène-1,2-diol (EAPB 02308)

Rdt : 54%. Mw : 361.40 g/mol. 1H-RMN δ (ppm, 400 MHz, DMSO d6) 3.09 (m, 4H, 2 x *CH2*NH), 4.40 (m, 2H, 2 x *CH2*NC=N), 6.79 (d, 1H, CH 6', J=8Hz), 6.88 (s, 1H, CH 2'), 6.92 (d, 1H, CH 5', J=8Hz), 7.06 (t, 1H, CH 7, J=8Hz), 7.35 (m, 3H, CH 6, CH 8, NH), 7.49 (s, 1H, CH 2), 7.60 (d, 1H, CH 9, J=8Hz). MS (ESI +, QTof, m/z) : 362.16 [M+H]⁺

### 4-(4-((3-aminopropyl)amino)imidazo[1,2-a]quinoxalin-1-yl)benzène-1,2-diol (EAPB 02309)

Rdt : 80%. Mw : 349.39 g/mol. 1H-RMN δ (ppm, 400 MHz, DMSO d6) 2.02 (m, 2H, CH2), 2.90 (m, 2H, CH2), 3.64 (m, 2H, CH2), 6.80 (d, 1H, CH 6', J=8Hz), 6.91 (m, 2H, CH 2', CH 5'), 6.99 (t, 1H, CH 7, J=8Hz), 7.30 (m, 3H, CH 6, CH 8, NH), 7.43 (s, 1H, CH 2), 7.60 (d, 1H, CH 9, J=8Hz), 7.90 (m, 2H, NH2), 9.31 (m, 1H, NH). MS (ESI +, QTof, m/z) : 350.16 [M+H]⁺

### 4-(4-(méthylamino)-7-(trifluorométhyl)imidazo[1,2-a]quinoxalin-1-yl)benzène-1,2-diol (EAPB 02303-7a)

Rdt : 28%. Mw : 374.32 g/mol. 1H-RMN δ (ppm, 400 MHz, DMSO d6) 3.06 (d, 3, CH3NH, J=8Hz), 6.81 (d, 1H, CH 6', J=8Hz), 6.89 (s, 1H, CH 2'), 6.92 (d, 1H, CH 5', J=8Hz), 7.34 (d, 1H, CH 8, J=8Hz), 7.47 (s, 1H, CH 2), 7.51 (d, 1H, CH 9, J=8Hz), 7.82 (s, 1H, CH 6), 8.03 (m, 1H, *NH*CH3). MS (ESI +, QTof, m/z) : 375.10 [M+H]⁺

### 4-(4-(méthylamino)-7-(trifluorométhoxy)imidazo[1,2-a]quinoxalin-1-yl)benzène-1,2-diol (EAPB 02303-7b)

Rdt : 67%. Mw : 390.32 g/mol. 1H-RMN δ (ppm, 400 MHz, DMSO d6) 3.08 (d, 3H, CH3NH, J=8 Hz), 6.84 (d, 1H, CH 6', J=8Hz), 6.94 (s, 1H, CH 2'), 6.97 (d, 1H, CH 5', J=8Hz), 7.03 (d, 1H, CH 8, J=8Hz), 7.40 (d, 1H, CH 9, J=8Hz), 7.42 (s, 1H, CH 2), 7.45 (s, 1H, CH 6), 8.01 (d, 1H, *NH*CH3, J=4Hz). 13C-RMN δ (ppm, 100 MHz, DMSO d6) 27.74 (*CH3*NH), 114.58 (CH8), 116.56 (CH 5'), 117.15 (CH 9), 117.72 (CH 2'), 118.12 (CH 6), 120.80 (Cq 1'), 121.99 (CH 6'), 124.76 (Cq 5'), 131.38 (Cq 1), 132.20 (CH 2), 133.04 (Cq 3'), 139.79 (Cq 9'), 146.12 (Cq 3' phényl), 146.25 (Cq 7), 147.18 (Cq 4' phényl), 149.27 (Cq 4). 19F-RMN δ (ppm, 100 MHz, DMSO d6) -56.83 (OCF3). MS (ESI +, QTof, m/z) : 391.10 [M+H]⁺

### 4-(4-(méthylamino)-8-(trifluorométhyl)imidazo[1,2-a]quinoxalin-1-yl)benzène-1,2-diol (EAPB 02303-8a)

Rdt : 55%. Mw: 374.32 g/mol. 1H-RMN δ (ppm, 400 MHz, DMSO d6) 3.12 (d, 3H, *CH3*NH, J=8Hz), 6.83 (d, 1H, CH 6', J=8Hz), 6.91 (s, 1H, CH 2'), 6.96 (d, 1H, CH 5'), 7.55 (s, 1H, CH 2), 7.60 (s, 1H, CH 9), 7.67 (d, 1H, CH 7, J=8Hz), 7.82 (d, 1H, CH 6). 13C-RMN δ (ppm, 100 MHz, DMSO d6) 28.38 (*CH3*NH), 113.45 (CH 9), 116.52 (CH 5'), 118.50 (CH 2'), 120.08 (Cq 1'), 121.93 (CH 1'), 123.11 (CH 7), 125.08 (Cq 5'), 132.68 to 132.75 (CH 2, Cq 1, Cq 3', Cq 9'), 146.36 (Cq 3' phényl, Cq 4' phényl), 147.49 (Cq 4). 19F-RMN δ (ppm, 100 MHz, DMSO d6) -60.44 (CF3). MS (ESI +, QTof, m/z): 375.20 [M+H]⁺

### Acide (2S)-2-((1-(3,4-diméthoxyphényl)imidazo[1,2-a]quinoxalin-4-yl)amino)propanoïque (EAPB 02219) composé de référence

Rdt : 11%. Mw :392.41 g/mol. MS (ESI +, QTof, m/z) : 393.0 [M+H]⁺

### Acide (2S)-2-((1-(3,4-diméthoxyphényl)imidazo[1,2-a]quinoxalin-4-yl)amino)-3-méthylbutanoïque (EAPB 02221) composé de référence

Rdt : 13%. Mw :420.46 g/mol. MS (ESI +, QTof, m/z) : 421.1 [M+H]⁺

### Acide (2S)-2-amino-5-((1-(3,4-dihydroxyphényl)imidazo[1,2-a]quinoxalin-4-yl)amino)pentanoïque (EAPB 02325)

Rdt : 20%. Mw : 407.42 g/mol. MS (ESI +, QTof, m/z): 408.1 [M+H]⁺

### Biologie: Etude de l'activité des composés sur les 15 lignées tumorales, Etude de cytotoxicité in vitro

Le but de ce protocole est d'étudier les effets des composés sur la prolifération de différentes lignées cellulaires cancéreuses humaines afin d'évaluer la CE₅₀ et leur activité *in cellulo.*

### Protocole

Les cellules sont ensemencées la veille du traitement en plaque de 96 puits. Chaque concentration de composé est testée en triple exemplaire. Une plaque de 96 puits peut contenir 3 composés.

Les suspensions de cellules pour chaque lignée cellulaire cancéreuse sont préparées dans le milieu de culture à une densité spécifique de la cellule spécifique (voir le tableau suivant). Ensuite, 100 µL de suspension de cellules sont étalées par puits (plaque par une lignée cellulaire).

**Tableau 1 : Les lignées cellulaires cancéreuses testées**

| **Cell line** | **Organism** | **Tissue origin** | **Cell type** | **Cell nb / well** | **Culture medium** |
|---|---|---|---|---|---|
| **A375** | Homo sapiens, humain | peau | Mélanome malin | 5000 | DMEM 10% |
| **CFPAC-1** | Homo sapiens, humain | côlon; provenant région métastatique sus-claviculaire | Adénocarcinome | 13 000 | RPMI 10% |
| **PC-3** | Homo sapiens, humain | Prostate (à partir du site métastatique: os) | Adénocarcinome, grade IV | 10 000 | RPMI 10% |
| **AsPC-1** | Homo sapiens, humain | Pancréas; ascites | Adénocarcinome | 13 000 | RPMI 10% |
| **BxPC3** | Homo sapiens, humain | Pancréas | Carcinome | 5000 | RPMI 10% |
| **A2058** | Homo sapiens, humain | Mélanome | Mélanome malin | 7500 | DMEM 10% |
| **Calu-1** | Homo sapiens, humain | poumon; à partir du site métastatique: plèvre | Carcinome | 7500 | DMEM 10% |
| **LoVo** | Homo sapiens, humain | **côlon, région métastatique: région sus-claviculaire** | Adénocarcinome | 5000 | DMEM 10% |
| **MEWO** | Homo sapiens, humain | Peau | Mélanome malin | 7500 | DMEM 10% |
| **T-47D** | Homo sapiens, humain | **sein; glande mammaire (de l'épanchement pleural)** | Carcinome canalaire | 10 000 | DMEM 10% |
| **Capan-1** | Homo sapiens, humain | Pancréas (à partir du site métastatique : foie) | Adénocarcinome | 10 000 | RPMI 10% |
| **HepG2** | Homo sapiens, humain | Foie | Hépatoblastome (carcinome hépatocellulaire) | 13 000 | RPMI 10% |
| **IPC298** | Homo sapiens, humain | Mélanome | Mélanome malin | 5000 | RPMI 10% |
| **PF382** | Homo sapiens, humain | Hématopoïétique et le tissu lymphoïde | Néoplasme lymphoïde | 100 000 | RPMI 10% (SUSPENSION) |
| **COLO-205** | *Homo sapiens,* humain | côlon; ascite: à partir du site métastatique | adénocarcinome colorectal Dukes type D | 7500 | RPMI 10% (1/2 SUSPENSION) |

### Préparations des composés

Les solutions mères des composés testés sont préparées à 10 mM dans 100% de DMSO et stockées à -20 ° C.

Les études d'activité *in vitro* des composés ont été évaluées sur différents types tumoraux et ont montré une activité du même ordre de grandeur entre les composés de l'invention EAPB02303 et EAP02302 et systématiquement supérieure par rapport à l'activité de EAPB0503 [i.e. (1-(3-méthoxyphényl)-N-méthylimidazo[1,2-a]quinoxalin-4-amine)].

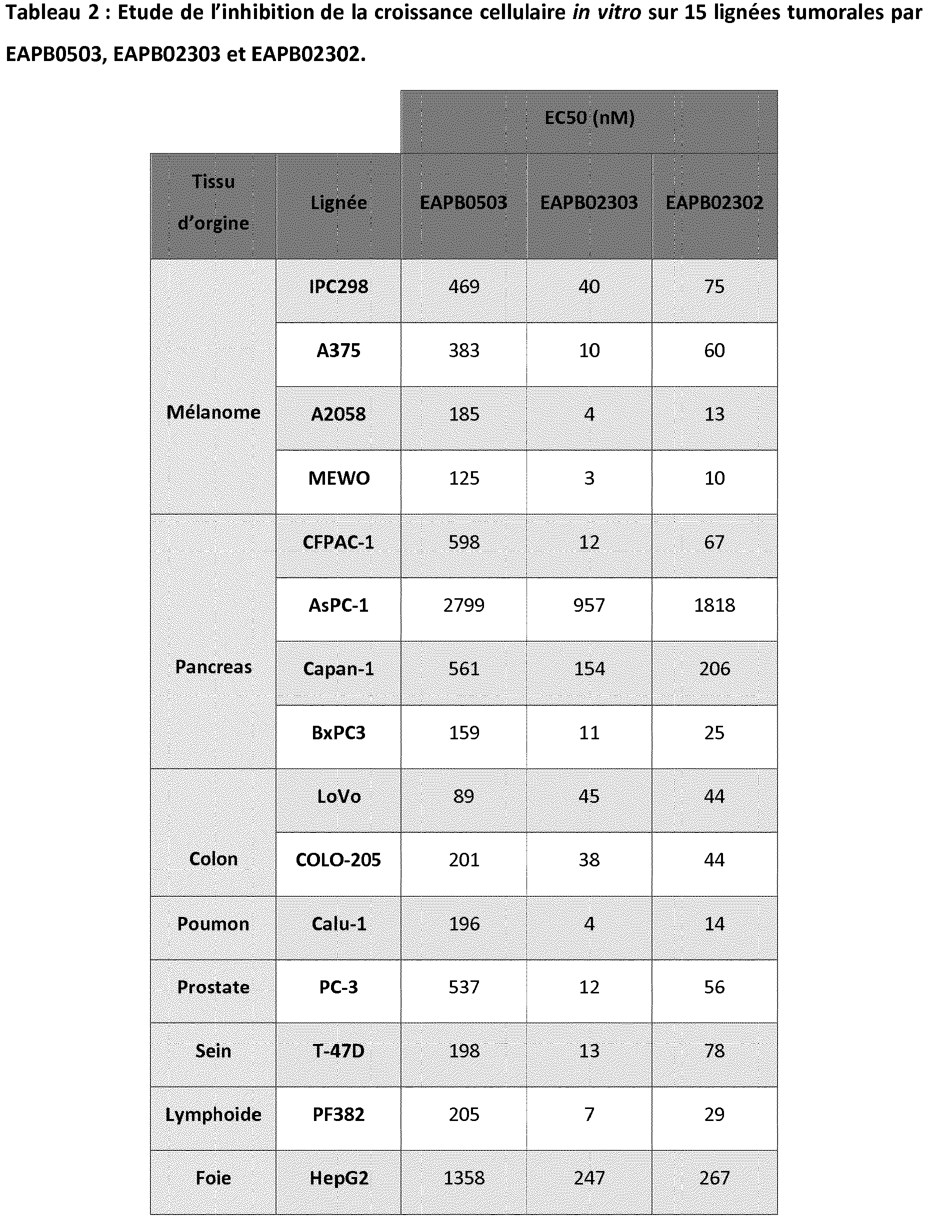

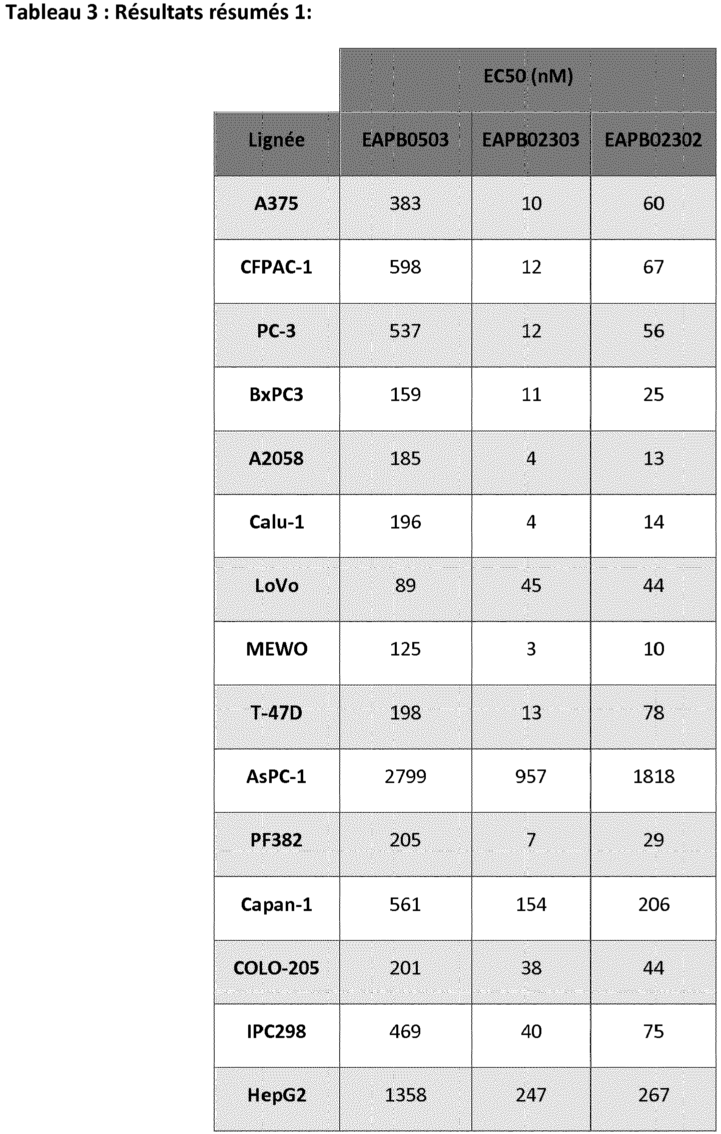

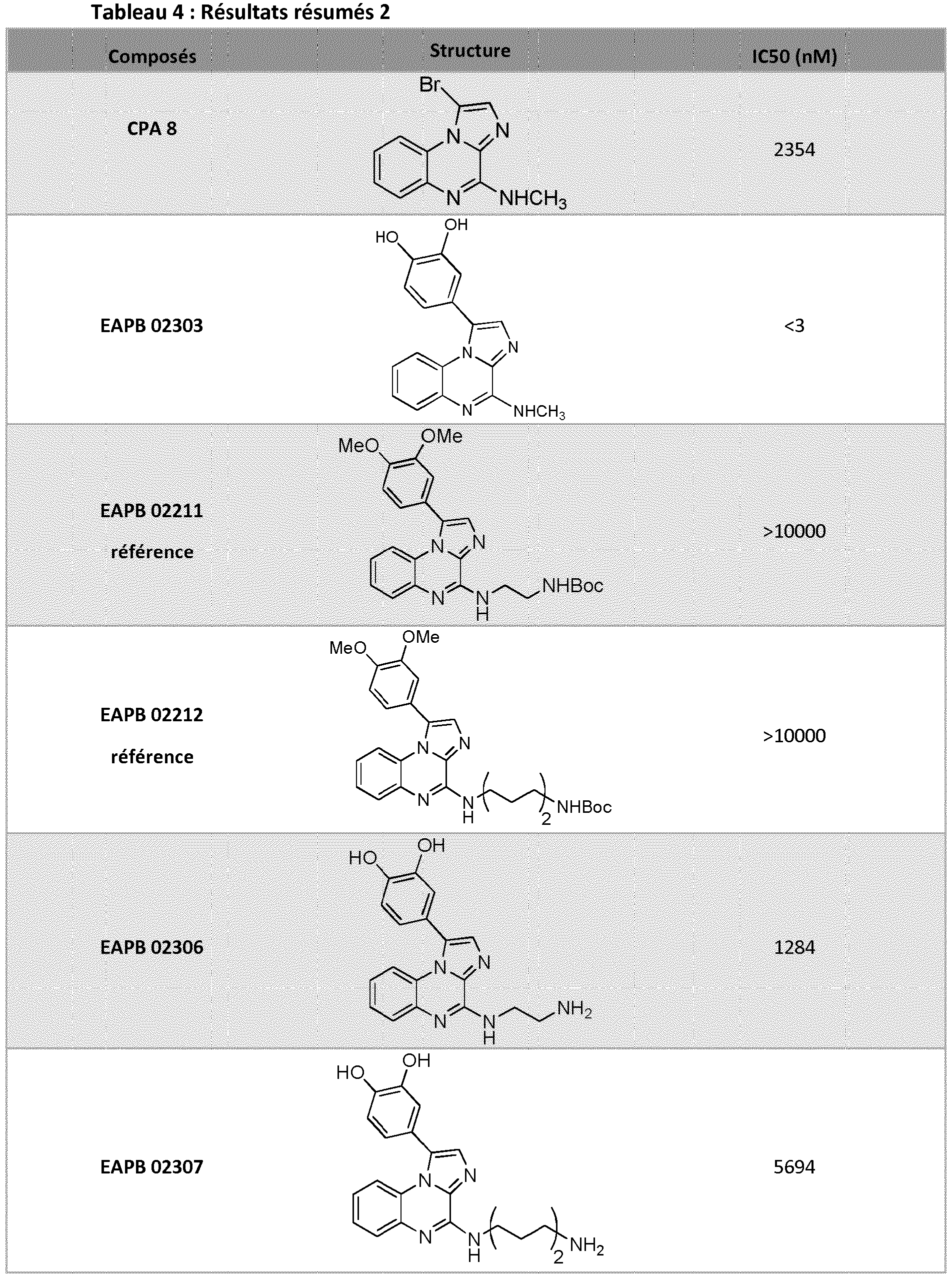

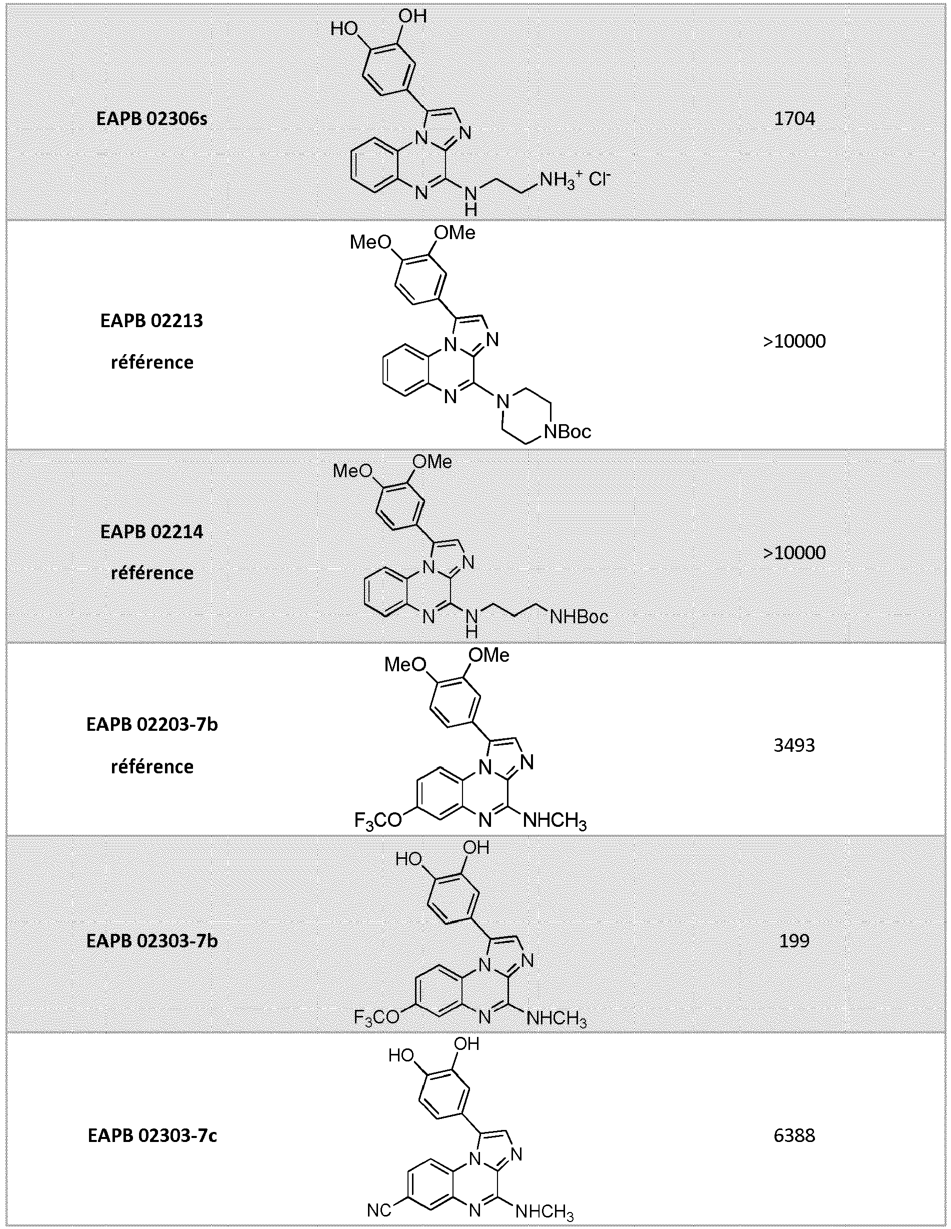

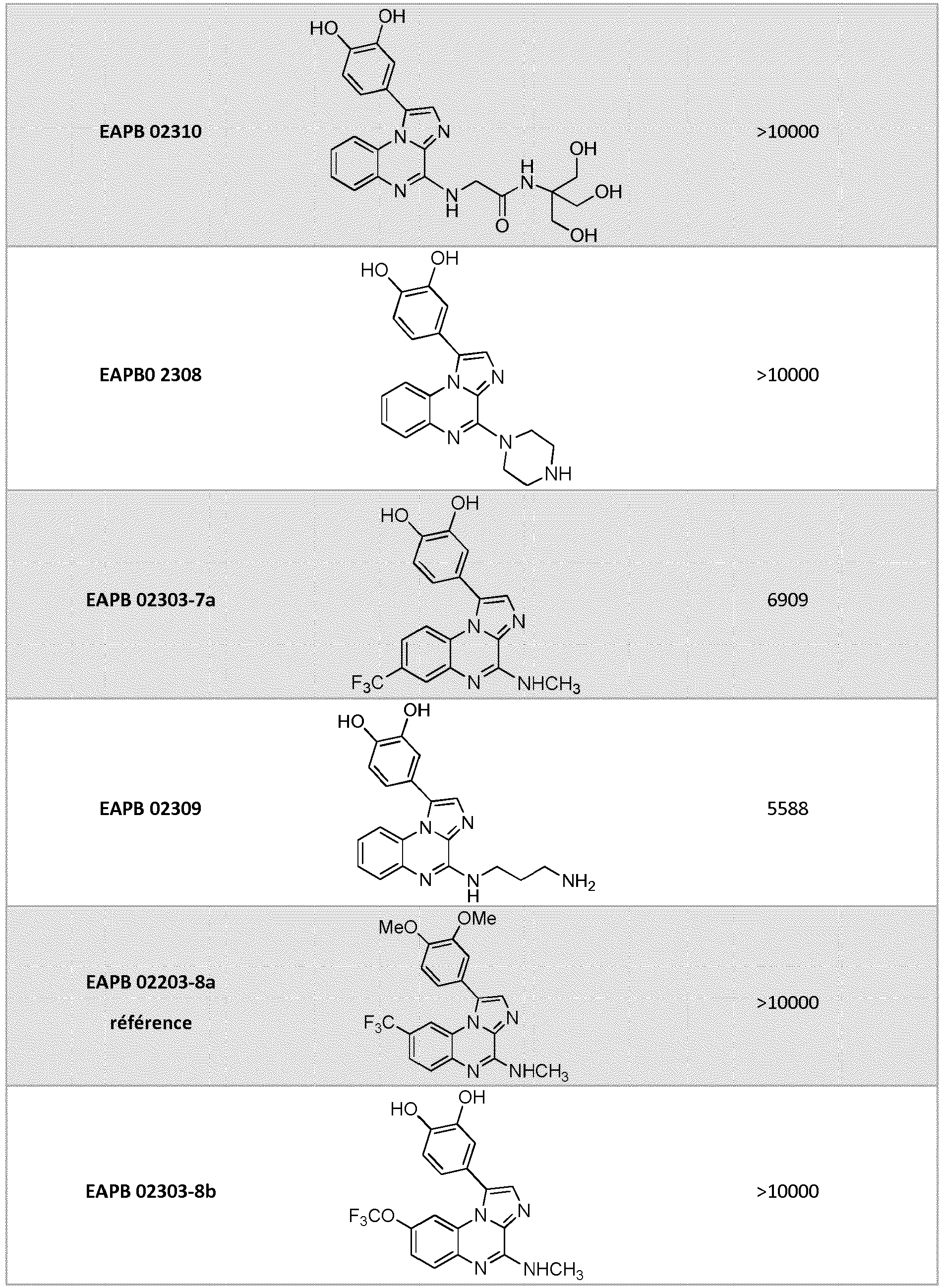

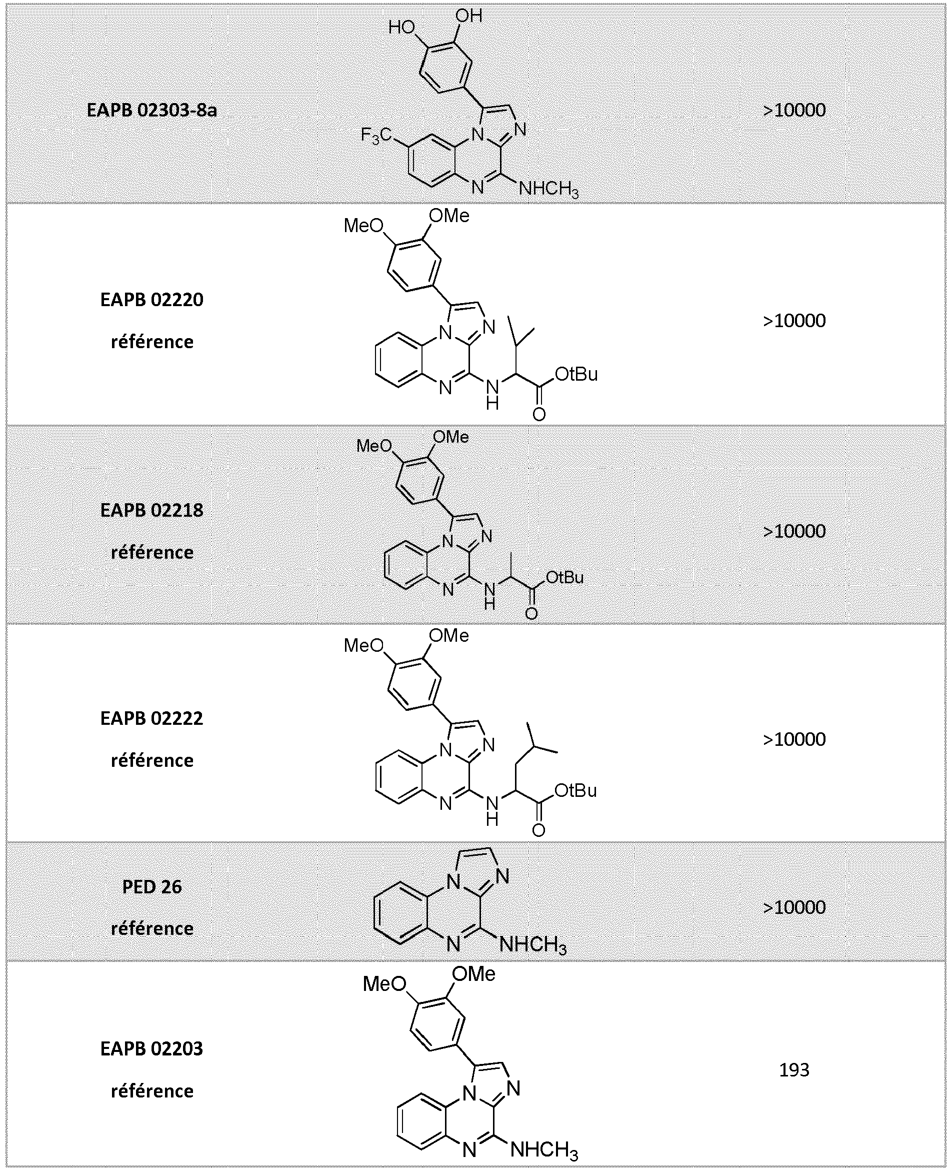

Les valeurs de CE₅₀ (concentration provoquant 50% d'inhibition de l'activité spécifique du contrôle) sont déterminées par l'analyse de la régression non linéaire de la courbe d'inhibition produite par la moyenne des valeurs répliquées (utilisation d'une courbe sigmoïde dose-réponse avec la pente de Hill comme variable et les valeurs constantes de 0,0 pour le bas et de 100,0 pour le sommet comme contraintes). L'analyse est effectuée en utilisant le logiciel GraphPad Prism 5.0.

### Matériels et méthodes

Jour 1: les cellules sont ensemencées un jour avant le traitement par les composés dans des plaques à 96 puits. Les suspensions cellulaires pour chaque lignée cellulaire cancéreuse sont préparées à une densité spécifique dans un milieu de culture spécifique (voir tableau précédent). Ensuite, 100 µl de suspension de cellules sont distribuées par puits (1x plaque à 96 puits par lignée cellulaire).

Jour 2: 24 heures plus tard, les composés ou témoins négatifs (DMSO) sont ajoutés aux cellules (100 µL d'une solution de composé dans son milieu spécifique avec une concentration finale de DMSO de 0,15%).

Chaque concentration de composé est testée en triple exemplaire (6 concentrations par composé comprises entre 10⁻⁹ M et 10⁻⁵ M). Les cellules sont incubées à 37°C pendant 72 heures sans nouveau traitement.

Jour 5: 15 minutes avant l'incubation des cellules avec le MTT (3-[4,5-diméthylthiazol-2-yl] -2,5-diphényltétrazolium bromure), le SDS à 10% est ajouté dans les puits témoins pour obtenir une concentration finale de 1%. Le SDS 1% représente le contrôle positif de l'inhibition de la prolifération.

Une fois le milieu éliminé, 100 µl d'une solution de MTT dans du milieu frais sont ajoutés à chaque puits (10 µl d'une solution 10X MTT à 5 mg / mL dans du PBS + 90 µl de milieu frais) et on incube pendant 4 h à 37 ° C.

La réaction au MTT est arrêtée et le milieu est homogénéisé par ajout de 100 µl d'une solution de SDS à 10% / HCl 0,01 M placés dans chaque puits. Le milieu est incubé pendant 2 heures à 37 ° C avant de mesurer son absorbance. L'absorbance est mesurée à 570 nm.

### Etude de l'effet des composés sur la polymérisation de la tubuline purifiée.

La tubuline est préparée à partir de cerveaux de porc selon le procédé de purification décrit par Williams et Lee (1982). La polymérisation de la tubuline est suivie par turbidimétrie à 350 nm avec un spectrophotomètre MC2 (Safas, Monaco) équipé d'un porte cuve à chemise thermique. Le mélange réactionnel a été préparé à 0 ° C, et contient du tampon PEM, 25% de glycérol (v / v), GTP 1 mM, et 2,4 µM de tubuline. Le GTP et la tubuline sont ajoutés à la dernière minute. Les solutions mères des composés testés et de la colchicine sont réalisées dans du DMSO à la concentration désirée, et 1 µL de la solution de composé est ajoutée au milieu réactionnel. Le même volume de DMSO seul a été utilisé pour le contrôle négatif. Le volume final de l'échantillon est de 200 µL. La réaction est déclenchée en plaçant la cuve dans le compartiment de cellule du spectrophotomètre thermostaté à 37 °C. De la glace est ajoutée 45 minutes plus tard pour lancer la dépolymérisation afin de vérifier la spécificité du signal.

## Revendications

1. Composé de formule générale (I) : 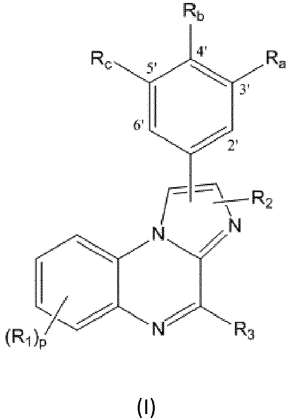 dans laquelle :
- les différents R₁ représentent indépendamment les uns des autres un atome d'hydrogène, d'halogène ou un groupe choisi parmi les groupes hydroxy, C₁-C₄ alkyle, C₁-C₄ alcoxy, amino, C₁-C₄ alkylamino, C₁-C₄ dialkylamino, cyano, CF₃, OCF₃, -(CH₂)ₙNR₄R₅,-NH-(CH₂)ₙNR₄R₅, -(CH₂)ₙCOR₄, -(CH₂)ₙCO-NR₄R₅, -(CH₂)ₙSO₂-NR₄R₅, et -(CH₂)ₙCO₂R₄ ,;
- R₂ représente un atome d'hydrogène, ou un groupe choisi parmi les groupes hydroxy et C₁-C₂ alkyle, préférentiellement un atome d'hydrogène;
- R₃ représente un halogène ou un groupe choisi parmi les groupes amino, C₁-C₃ alkylamino, C₁-C₃ dialkylamino, amine-(C₁-C₆ alkyl)-amino, N-(tert-butyloxycarbonyl)amino-(C₁-C₆ alkyl)-amino, pipéridyle, N-(tert-butyloxycarbonyl)pipéridyle, un groupe aminé d'un acide α-aminé ou un groupe aminé de la chaîne latérale d'un acide α-aminé,
- Rₐ, R_{b} sont des groupes hydroxy et R_{c} représente indépendamment un atome d'hydrogène ou un groupe hydroxy;
- n étant 0, 1, 2, 3 ou 4 ;
- p étant 1, 2, 3 ou 4 ;
- R₄ et R₅ représentent indépendamment l'un de l'autre un atome d'hydrogène ou un groupe C₁-C₄ alkyle, préférentiellement linéaire ou ramifié ;
ou un sel pharmaceutiquement acceptable de celui-ci.

2. Composé selon la revendication 1, **caractérisé en ce qu'**il réponde à la formule (II) : 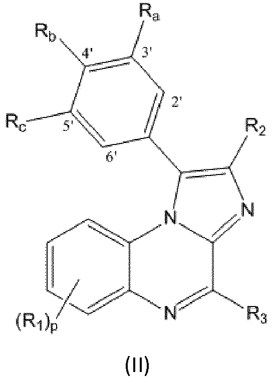
- dans laquelle les différents groupements R₁, R₂, R₃, Rₐ, R_{b}, R_{c} et p sont tels que définis dans la revendication 1,
ou un sel pharmaceutiquement acceptable de celui-ci.

3. Composé selon l'une quelconque des revendications 1 ou 2 **caractérisé en ce qu'**il réponde à la formule (III) : 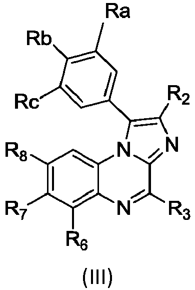
- dans laquelle les différents groupements R₂, R₃, Rₐ, R_{b} et R_{c} sont tels que définis dans l'une quelconque des revendications précédentes,
- R₆, R₇ et R₈ représentent indépendamment l'un de l'autre un atome d'hydrogène, d'halogène ou un groupe choisi parmi les groupes hydroxy, C₁-C₄ alkyle, C₁-C₄ alcoxy, amino, C₁-C₄ alkylamino, C₁-C₄ dialkylamino, cyano, CF₃, OCF₃, -(CH₂)ₙNR₄R₅,-NH-(CH₂)ₙNR₄R₅, -(CH₂)ₙCOR₄, -(CH₂)ₙCO-NR₄R₅, -(CH₂)ₙSO₂-NR₄R₅, et -(CH₂)nCO₂R₄ ;
- les différents R₄ et R₅ représentent indépendamment les uns des autres un atome d'hydrogène ou un groupe C₁-C₄ alkyle, préférentiellement linéaire ou ramifié ; et
- n étant 0, 1, 2, 3 ou 4 ;
ou un sel pharmaceutiquement acceptable de celui-ci.

4. Composé selon la revendication 3 **caractérisé en ce que** :
- R₆, R₇ et R₈ représentent indépendamment l'un de l'autre un atome d'hydrogène, d'halogène ou un groupe choisi parmi les groupes hydroxy, C₁-C₄ alkyle, C₁-C₄ alcoxy, OCF₃, préférentiellement hydrogène, méthyle, méthoxy ou OCF₃;
ou un sel pharmaceutiquement acceptable de celui-ci.

5. Composé selon l'une quelconque des revendications 1 à 4 **caractérisé en ce qu'**il réponde à la formule (IV) : 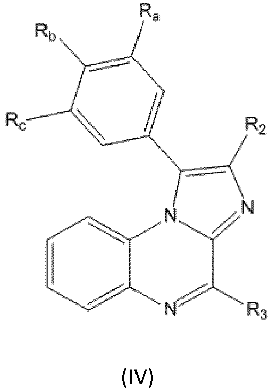
- dans laquelle les différents groupements R₂, R₃, Rₐ, R_{b} et R_{c} sont tels que définis dans l'une quelconque des revendications précédentes;
ou un sel pharmaceutiquement acceptable de celui-ci.

6. Composé selon l'une quelconque des revendications 1 à 5, **caractérisé en ce que** lesdits composés sont choisis parmi:
1. 1-(3,4-dihydroxyphényl)imidazo[1,2-a]quinoxalin-4-amine
2. 1-(3,4-dihydroxyphényl)-N-méthylimidazo[1,2-a]quinoxalin-4-amine
3. 4-(4-amino-6-(trifluorométhyl)imidazo[1,2-a]quinoxalin-1-yl)benzène-1,2-diol
4. 4-(4-(méthylamino)-6-(trifluorométhyl)imidazo[1,2-a]quinoxalin-1-yl)benzène-1,2-diol
5. 4-(4-((2-aminoéthyl)amino)-6-(trifluorométhyl)imidazo[1,2-a]quinoxalin-1-yl)benzène-1,2-diol
6. 4-(4-((3-aminopropyl)amino)-6-(trifluorométhyl)imidazo[1,2-a]quinoxalin-1-yl)benzène-1,2-diol
7. 4-(4-((6-aminohexyl)amino)-6-(trifluorométhyl)imidazo[1,2-a]quinoxalin-1-yl)benzène-1,2-diol
8. 4-amino-1-(3,4-dihydroxyphényl)imidazo[1,2-a]quinoxaline-6-carbonitrile
9. 1-(3,4-dihydroxyphényl)-4-(méthylamino)imidazo[1,2-a]quinoxaline-6-carbonitrile
10. 4-((2-aminoéthyl)amino)-1-(3,4-dihydroxyphényl)imidazo[1,2-a]quinoxaline-6-carbonitrile
11. 4-((3-aminopropyl)amino)-1-(3,4-dihydroxyphényl)imidazo[1,2-a]quinoxaline-6-carbonitrile
12. 4-((6-aminohexyl)amino)-1-(3,4-dihydroxyphényl)imidazo[1,2-a]quinoxaline-6-carbonitrile
13. 4-amino-1-(3,4-dihydroxyphényl)imidazo[1,2-a]quinoxaline-6-carboxylate de méthyle
14. 1-(3,4-dihydroxyphényl)-4-(méthylamino)imidazo[1,2-a]quinoxaline-6-carboxylate de méthyle
15. 4-((3-aminopropyl)amino)-1-(3,4-dihydroxyphényl)imidazo[1,2-a]quinoxaline-6-carboxylate de méthyle
16. 4-((6-aminohexyl)amino)-1-(3,4-dihydroxyphényl)imidazo[1,2-a]quinoxaline-6-carboxylate de méthyle
17. Acide 4-amino-1-(3,4-dihydroxyphényl)imidazo[1,2-a]quinoxaline-6-carboxylique
18. Acide 1-(3,4-dihydroxyphényl)-4-(méthylamino)imidazo[1,2-a]quinoxaline-6-carboxylique
19. Acide 4-((2-aminoéthyl)amino)-1-(3,4-dihydroxyphényl)imidazo[1,2-a]quinoxaline-6-carboxylique
20. Acide 4-((3-aminopropyl)amino)-1-(3,4-dihydroxyphényl)imidazo[1,2-a]quinoxaline-6-carboxylique
21. Acide 4-((6-aminohexyl)amino)-1-(3,4-dihydroxyphényl)imidazo[1,2-a]quinoxaline-6-carboxylique
22. 4-(4,6-diaminoimidazo[1,2-a]quinoxalin-1-yl)benzène-1,2-diol
23. 4-(6-amino-4-(méthylamino)imidazo[1,2-a]quinoxalin-1-yl)benzène-1,2-diol
24. 4-(6-amino-4-((2-aminoéthyl)amino)imidazo[1,2-a]quinoxalin-1-yl)benzène-1,2-diol
25. 4-(6-amino-4-((3-aminopropyl)amino)imidazo[1,2-a]quinoxalin-1-yl)benzène-1,2-diol
26. 4-(6-amino-4-((6-aminohexyl)amino)imidazo[1,2-a]quinoxalin-1-yl)benzène-1,2-diol
27. 4-(4-amino-7-(trifluorométhyl)imidazo[1,2-a]quinoxalin-1-yl)benzène-1,2-diol
28. 4-(4-(méthylamino)-7-(trifluorométhyl)imidazo[1,2-a]quinoxalin-1-yl)benzène-1,2-diol
29. 4-(4-((2-aminoéthyl)amino)-7-(trifluorométhyl)imidazo[1,2-a]quinoxalin-1-yl)benzène-1,2-diol
30. 4-(4-((3-aminopropyl)amino)-7-(trifluorométhyl)imidazo[1,2-a]quinoxalin-1-yl)benzène-1,2-diol
31. 4-(4-((6-aminohexyl)amino)-7-(trifluorométhyl)imidazo[1,2-a]quinoxalin-1-yl)benzène-1,2-diol
32. 4-amino-1-(3,4-dihydroxyphényl)imidazo[1,2-a]quinoxaline-7-carbonitrile
33. 1-(3,4-dihydroxyphényl)-4-(méthylamino)imidazo[1,2-a]quinoxaline-7-carbonitrile
34. 4-((2-aminoéthyl)amino)-1-(3,4-dihydroxyphényl)imidazo[1,2-a]quinoxaline-7-carbonitrile
35. 4-((3-aminopropyl)amino)-1-(3,4-dihydroxyphényl)imidazo[1,2-a]quinoxaline-7-carbonitrile
36. 4-((6-aminohexyl)amino)-1-(3,4-dihydroxyphényl)imidazo[1,2-a]quinoxaline-7-carbonitrile
37. Acide 4-amino-1-(3,4-dihydroxyphényl)imidazo[1,2-a]quinoxaline-7-carboxylique
38. Acide 1-(3,4-dihydroxyphényl)-4-(méthylamino)imidazo[1,2-a]quinoxaline-7-carboxylique
39. Acide 4-((2-aminoéthyl)amino)-1-(3,4-dihydroxyphényl)imidazo[1,2-a]quinoxaline-7-carboxylique
40. Acide 4-((3-aminopropyl)amino)-1-(3,4-dihydroxyphényl)imidazo[1,2-a]quinoxaline-7-carboxylique
41. Acide 4-((6-aminohexyl)amino)-1-(3,4-dihydroxyphényl)imidazo[1,2-a]quinoxaline-7-carboxylique
42. 4-(4,7-diaminoimidazo[1,2-a]quinoxalin-1-yl)benzène-1,2-diol
43. 4-(7-amino-4-(méthylamino)imidazo[1,2-a]quinoxalin-1-yl)benzène-1,2-diol
44. 4-(7-amino-4-((2-aminoéthyl)amino)imidazo[1,2-a]quinoxalin-1-yl)benzène-1,2-diol
45. 4-(7-amino-4-((3-aminopropyl)amino)imidazo[1,2-a]quinoxalin-1-yl)benzène-1,2-diol
46. 4-(7-amino-4-((6-aminohexyl)amino)imidazo[1,2-a]quinoxalin-1-yl)benzène-1,2-diol
47. 4-(4-amino-8-(trifluorométhyl)imidazo[1,2-a]quinoxalin-1-yl)benzène-1,2-diol
48. 4-(4-(méthylamino)-8-(trifluorométhyl)imidazo[1,2-a]quinoxalin-1-yl)benzène-1,2-diol
49. 4-(4-((2-aminoéthyl)amino)-8-(trifluorométhyl)imidazo[1,2-a]quinoxalin-1-yl)benzène-1,2-diol
50. 4-(4-((3-aminopropyl)amino)-8-(trifluorométhyl)imidazo[1,2-a]quinoxalin-1-yl)benzène-1,2-diol
51. 4-(4-((6-aminohexyl)amino)-8-(trifluorométhyl)imidazo[1,2-a]quinoxalin-1-yl)benzène-1,2-diol
52. 4-amino-1-(3,4-dihydroxyphényl)imidazo[1,2-a]quinoxaline-8-carbonitrile
53. 1-(3,4-dihydroxyphényl)-4-(méthylamino)imidazo[1,2-a]quinoxaline-8-carbonitrile
54. 4-((2-aminoéthyl)amino)-1-(3,4-dihydroxyphényl)imidazo[1,2-a]quinoxaline-8-carbonitrile
55. 4-((3-aminopropyl)amino)-1-(3,4-dihydroxyphényl)imidazo[1,2-a]quinoxaline-8-carbonitrile
56. 4-((6-aminohexyl)amino)-1-(3,4-dihydroxyphényl)imidazo[1,2-a]quinoxaline-8-carbonitrile
57. 4-amino-1-(3,4-dihydroxyphényl)imidazo[1,2-a]quinoxaline-8-carboxylate de méthyle
58. 1-(3,4-dihydroxyphényl)-4-(méthylamino)imidazo[1,2-a]quinoxaline-8-carboxylate de méthyle
59. 4-((2-aminoéthyl)amino)-1-(3,4-dihydroxyphényl)imidazo[1,2-a]quinoxaline-8-carboxylate de méthyle
60. 4-((3-aminopropyl)amino)-1-(3,4-dihydroxyphényl)imidazo[1,2-a]quinoxaline-8-carboxylate de méthyle
61. 4-((6-aminohexyl)amino)-1-(3,4-dihydroxyphényl)imidazo[1,2-a]quinoxaline-8-carboxylate de méthyle
62. Acide 4-amino-1-(3,4-dihydroxyphényl)imidazo[1,2-a]quinoxaline-8-carboxylique
63. Acide 1-(3,4-dihydroxyphényl)-4-(méthylamino)imidazo[1,2-a]quinoxaline-8-carboxylique
64. Acide 4-((2-aminoéthyl)amino)-1-(3,4-dihydroxyphényl)imidazo[1,2-a]quinoxaline-8-carboxylique
65. Acide 4-((3-aminopropyl)amino)-1-(3,4-dihydroxyphényl)imidazo[1,2-a]quinoxaline-8-carboxylique
66. Acide 4-((6-aminohexyl)amino)-1-(3,4-dihydroxyphényl)imidazo[1,2-a]quinoxaline-8-carboxylique
67. 4-(4,8-diaminoimidazo[1,2-a]quinoxalin-1-yl)benzène-1,2-diol
68. 4-(8-amino-4-(méthylamino)imidazo[1,2-a]quinoxalin-1-yl)benzène-1,2-diol
69. 4-(8-amino-4-((2-aminoéthyl)amino)imidazo[1,2-a]quinoxalin-1-yl)benzène-1,2-diol
70. 4-(8-amino-4-((3-aminopropyl)amino)imidazo[1,2-a]quinoxalin-1-yl)benzène-1,2-diol
71. 4-(8-amino-4-((6-aminohexyl)amino)imidazo[1,2-a]quinoxalin-1-yl)benzène-1,2-diol.
72. 4-(4-((2-aminoéthyl)amino)imidazo[1,2-a]quinoxalin-1-yl)benzène-1,2-diol
73. Chlorure de 2-((1-(3,4-dihydroxyphényl)imidazo[1,2-a]quinoxalin-4-yl)amino)éthylammonium
74. 4-(4-((6-aminohexyl)amino)imidazo[1,2-a]quinoxalin-1-yl)benzène-1,2-diol
75. Chlorure de 6-((1-(3,4-dihydroxyphényl)imidazo[1,2-a]quinoxalin-4-yl)amino)hexan-1-ammonium
76. 4-(4-(pipérazin-1-yl)imidazo[1,2-a]quinoxalin-1-yl)benzène-1,2-diol
77. 4-(4-((3-aminopropyl)amino)imidazo[1,2-a]quinoxalin-1-yl)benzène-1,2-diol
78. N-(1,3-dihydroxy-2-(hydroxyméthyl)propan-2-yl)-2-((1-(3,4-dihydroxyphényl)imidazo[1,2-a]quinoxalin-4-yl)amino)acétamide
79. 4-(4-(méthylamino)-7-(trifluorométhoxy)imidazo[1,2-a]quinoxalin-1-yl)benzène-1,2-diol
80. Acide (2S)-2-amino-5-((1-(3,4-dihydroxyphényl)imidazo[1,2-a]quinoxalin-4-yl)amino)pentanoïque

7. Composé selon l'une quelconque des revendications 1 à 6 **caractérisé en ce que** :
R₂ et R_{c} sont des atomes d'hydrogène ;
R₃ est un groupe méthylamino ou amine; et
Rₐ et R_{b} sont des groupes hydroxy ;
ou un sel pharmaceutiquement acceptable de celui-ci.

8. Procédé de fabrication d'un composé selon l'une quelconque des revendications 1 à 7 **caractérisé en ce que** ledit procédé comprend :
- une étape de couplage du composé de formule (V) : 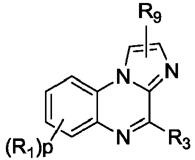 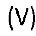
dans laquelle R₁, R₃ et p sont tels que définis dans l'une quelconque des revendications 1 à 6 ; et
R₉ est un atome d'halogène ;
avec un acide arylboronique approprié, dans les conditions de Suzuki; et
- récupération, extraction et/ou purification du composé obtenu selon l'une quelconque des revendications 1 à 7.

9. Composition pharmaceutique comprenant au moins un composé selon l'une quelconque des revendications 1 à 7 et optionnellement un véhicule pharmaceutiquement approprié.

10. Composé selon l'une quelconque des revendications 1 à 7 pour son utilisation en tant que médicament.

11. Composé selon l'une quelconque des revendications 1 à 7 pour son utilisation dans le traitement d'au moins un cancer.

12. Composé pour son utilisation selon la revendication 11 **caractérisé en ce que** le cancer est solide, liquide, éventuellement métastatique ou secondaire.

13. Composé pour son utilisation selon la revendication 11 ou 12 **caractérisé en ce que** le cancer est un mélanome, un cancer du pancréas, du colon ou de la prostate, un lymphome, une leucémie ou un myélome.

14. Composé pour son utilisation selon l'une quelconque des revendications 11 à 12 **caractérisé en ce que** le cancer est un cancer du sein, de la prostate, des poumons, de l'œsophage, de la peau, de la vessie, de l'estomac, du foie, de l'utérus, du côlon ou du rectum.

15. Composé pour son utilisation selon l'une quelconque des revendications 11 à 12 **caractérisé en ce que** le cancer est un cancer du sang ou de certaines cellules du sang telles que lymphocytes ou leucocytes.

## Patentansprüche

1. Verbindung mit der allgemeinen Formel (I): 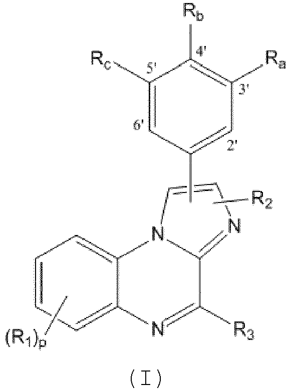 wobei:
- die verschiedenen R₁ unabhängig voneinander ein Wasserstoffatom, ein Halogenatom oder eine Gruppe, die unter den Hydroxy-, C₁-C₄-Alkyl-, C₁-C₄-Alkoxy-, Amino-, C₁-C₄-Alkylamino-, C₁-C₄-Dialkylamino-, Cyano-, CF₃-, OCF₃-, -(CH₂)ₙNR₄R₅-, -NH-(CH₂)ₙNR₄R₅-, -(CH₂)ₙCOR₄-,-(CH₂)ₙCO-NR₄R₅-, -(CH₂)ₙSO₂-NR₄R₅- und -(CH₂)ₙCO₂R₄-Gruppen ausgewählt sind;
- R₂ ein Wasserstoffatom oder eine Gruppe, die unter den Hydroxy- und C₁-C₂-Alkyl-Gruppen ausgewählt ist, vorzugsweise ein Wasserstoffatom, darstellt;
- R₃ ein Halogen oder eine Gruppe darstellt, die unter den Amino-, C₁-C₃-Alkylamino-, C₁-C₃-Dialkylamino-, Amino-(C₁-C₆-alkyl)-amino-, N-(tert-Butyloxycarbonyl)amino-(C₁-C₆-alkyl)-amino-, Piperidyl-, N-(tert-butyloxycarbonyl)piperidyl-Gruppen, einer Aminogruppe einer α-Aminosäure oder einer Aminogruppe der Seitenkette einer α-Aminosäure ausgewählt ist,
- Rₐ, R_{b} Hydroxygruppen sind und R_{c} unabhängig ein Wasserstoffatom oder eine Hydroxygruppe darstellt;
- n 0, 1, 2, 3 oder 4 ist;
- p 1, 2, 3 oder 4 ist;
- R₄ und R₅ unabhängig voneinander ein Wasserstoffatom oder eine, vorzugsweise lineare oder verzweigte, C₁-C₄-Alkylgruppe darstellen;
oder ein pharmazeutisch annehmbares Salz davon.

2. Verbindung nach Anspruch 1, **dadurch gekennzeichnet, dass** sie der Formel (II) entspricht: 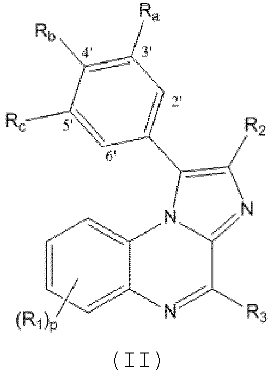
- wobei die verschiedenen Gruppen R₁, R₂, R₃, Rₐ, R_{b}, R_{c} und p nach Anspruch 1 sind,
oder ein pharmazeutisch annehmbares Salz davon.

3. Verbindung nach einem der Ansprüche 1 oder 2, **dadurch gekennzeichnet, dass** sie der folgenden Formel (III) entspricht: 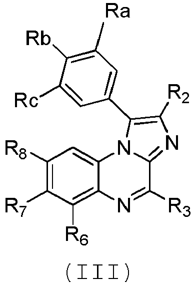
- wobei die verschiedenen Gruppen R₂, R₃, Rₐ, R_{b} und R_{c} nach einem der vorhergehenden Ansprüche sind,
- R₆, R₇ und R₈ unabhängig voneinander ein Wasserstoffatom, ein Halogenatom oder eine Gruppe darstellen, die unter den Hydroxy-, C₁-C₄-Alkyl-, C₁-C₄-Alkoxy-, Amino-, C₁-C₄-Alkylamino-, C₁-C₄-Dialkylamino-, Cyano-, CF₃-, OCF₃-, -(CH₂)ₙNR₄R₅-, -NH-(CH₂)ₙNR₄R₅-,-(CH₂)ₙCOR₄-, -(CH₂)ₙCO-NR₄R₅-, -(CH₂)ₙSO₂-NR₄R₅-, und-(CH₂)nCO₂R₄-Gruppen gewählt sind;
- die verschiedenen R₄ und R₅ unabhängig voneinander ein Wasserstoffatom oder eine, vorzugsweise lineare oder verzweigte, C₁-C₄-Alkylgruppe darstellen; und
- n 0, 1, 2, 3 oder 4 ist;
oder ein pharmazeutisch annehmbares Salz davon.

4. Verbindung nach Anspruch 3, **dadurch gekennzeichnet, dass**:
- R₆, R₇ et R₈ unabhängig voneinander ein Wasserstoffatom, ein Halogenatom oder eine Gruppe darstellen, die unter den Hydroxy-, C₁-C₄-Alkyl-, C₁-C₄-Alkoxy-, OCF₃-, vorzugsweise Wasserstoff-, Methyl-, Methoxy- oder OCF₃-Gruppen, gewählt ist;
oder ein pharmazeutisch annehmbares Salz davon.

5. Verbindung nach einem der Ansprüche 1 bis 4, **dadurch gekennzeichnet, dass** sie der folgenden Formel (IV) entspricht: 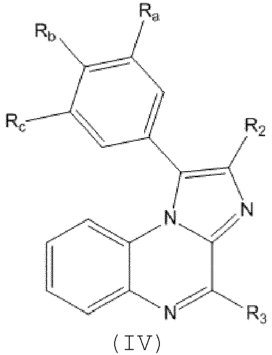
- wobei die verschiedenen Gruppen R₂, R₃, Rₐ, R_{b} und R_{c} nach einem der vorhergehenden Ansprüche sind;
oder ein pharmazeutisch annehmbares Salz davon.

6. Verbindung nach einem der Ansprüche 1 bis 5, **dadurch gekennzeichnet, dass** die Verbindungen ausgewählt sind unter:
1. 1-(3,4-Dihydroxyphenyl)imidazo[1,2-a]chinoxalin-4-amin
2. 1-(3,4-Dihydroxyphenyl)-N-methylimidazo[1,2-a]chinoxalin-4-amin
3. 4-(4-Amino-6-(trifluoromethyl)imidazo[1,2-a]chinoxalin-1-yl)benzol-1,2-diol
4. 4-(4-(Methylamino)-6-(trifluoromethyl)imidazo[1,2-a]chinoxalin-1-yl)benzol-1,2-diol
5. 4-(4-((2-Aminoethyl)amino)-6-(trifluoromethyl)imidazo[1,2-a]chinoxalin-1-yl)benzol-1,2-diol
6. 4-(4-((3-Aminopropyl)amino)-6-(trifluoromethyl)imidazo[1,2-a]chinoxalin-1-yl)benzol-1,2-diol
7. 4-(4-((6-Aminohexyl)amino)-6-(trifluoromethyl)imidazo[1,2-a]chinoxalin-1-yl)benzol-1,2-diol
8. 4-Amino-1-(3,4-dihydroxyphenyl)imidazo[1,2-a]chinoxalin-6-carbonitril
9. 1-(3,4-Dihydroxyphenyl)-4-(methylamino)imidazo[1,2-a]chinoxalin-6-carbonitril
10. 4-((2-Aminoethyl)amino)-1-(3,4-dihydroxyphenyl)imidazo[1,2-a]chinoxalin-6-carbonitril
11. 4-((3-Aminopropyl)amino)-1-(3,4-dihydroxyphenyl)imidazo[1,2-a]chinoxalin-6-carbonitril
12. 4-((6-Aminohexyl)amino)-1-(3,4-dihydroxyphenyl)imidazo[1,2-a]chinoxalin-6-carbonitril
13. 4-Amino-1-(3,4-dihydroxyphenyl)imidazo[1,2-a]chinoxalin-6-Methylcarboxylat
14. 1-(3,4-Dihydroxyphenyl)-4-(methylamino)imidazo[1,2-a]chinoxalin-6-Methylcarboxylat
15. 4-((3-Aminopropyl)amino)-1-(3,4-Dihydroxyphenyl)imidazo[1,2-a]chinoxalin-6-Methylcarboxylat
16. 4-((6-Aminohexyl)amino)-1-(3,4-dihydroxyphenyl)imidazo[1,2-a]chinoxalin-6-Methylcarboxylat
17. 4-Amino-1-(3,4-dihydroxyphenyl)imidazo[1,2-a]chinoxalin-6-Carbonsäure
18. 1-(3,4-Dihydroxyphenyl)-4-(methylamino)imidazo[1,2-a]chinoxalin-6-Carbonsäure
19. 4-((2-Aminoethyl)amino)-1-(3,4-dihydroxyphenyl)imidazo[1,2-a]chinoxalin-6-Carbonsäure
20. 4-((3-Aminopropyl)amino)-1-(3,4-dihydroxyphenyl)imidazo[1,2-a]chinoxalin-6-Carbonsäure
21. 4-((6-Aminohexyl)amino)-1-(3,4-dihydroxyphenyl)imidazo[1,2-a]chinoxalin-6-Carbonsäure
22. 4-(4,6-Diaminoimidazo[1,2-a]chinoxalin-1-yl)benzol-1,2-diol
23. 4-(6-Amino-4-(methylamino)imidazo[1,2-a]chinoxalin-1-yl)benzol-1,2-diol
24. 4-(6-Amino-4-((2-aminoethyl)amino)imidazo[1,2-a]chinoxalin-1-yl)benzol-1,2-diol
25. 4-(6-Amino-4-((3-aminopropyl)amino)imidazo[1,2-a]chinoxalin-1-yl)benzol -1,2-diol
26. 4-(6-Amino-4-((6-aminohexyl)amino)imidazo[1,2-a]chinoxalin-1-yl)benzol-1,2-diol
27. 4-(4-Amino-7-(trifluoromethyl)imidazo[1,2-a]chinoxalin-1-yl)benzol-1,2-diol
28. 4-(4-(Methylamino)-7-(trifluoromethyl)imidazo[1,2-a]chinoxalin-1-yl)benzol-1,2-diol
29. 4-(4-((2-Aminoethyl)amino)-7-(trifluoromethyl)imidazo[1,2-a]chinoxalin-1-yl)benzol-1,2-diol
30. 4-(4-((3-Aminopropyl)amino)-7-(trifluoromethyl)imidazo[1,2-a]chinoxalin-1-yl)benzol-1,2-diol
31. 4-(4-((6-Aminohexyl)amino)-7-(trifluoromethyl)imidazo[1,2-a]chinoxalin-1-yl)benzol-1,2-diol
32. 4-Amino-1-(3,4-dihydroxyphenyl)imidazo[1,2-a]chinoxalin-7-carbonitril
33. 1-(3,4-Dihydroxyphenyl)-4-(methylamino)imidazo[1,2-a]chinoxalin-7-carbonitril
34. 4-((2-Aminoethyl)amino)-1-(3,4-dihydroxyphenyl)imidazo[1,2-a]chinoxalin-7-carbonitril
35. 4-((3-Aminopropyl)amino)-1-(3,4-dihydroxyphenyl)imidazo[1,2-a]chinoxalin-7-carbonitril
36. 4-((6-Aminohexyl)amino)-1-(3,4-dihydroxyphenyl)imidazo[1,2-a]chinoxalin-7-carbonitril
37. 4-Amino-1-(3,4-dihydroxyphenyl)imidazo[1,2-a]chinoxalin-7-Carboxylsäure
38. 1-(3,4-Dihydroxyphenyl)-4-(methylamino)imidazo[1,2-a]chinoxalin-7-Carboxylsäure
39. 4-((2-Aminoethyl)amino)-1-(3,4-dihydroxyphenyl)imidazo[1,2-a]chinoxalin-7-Carboxylsäure
40. 4-((3-Aminopropyl)amino)-1-(3,4-dihydroxyphenyl)imidazo[1,2-a]chinoxalin-7-Carboxylsäure
41. 4-((6-Aminohexyl)amino)-1-(3,4-dihydroxyphenyl)imidazo[1,2-a]chinoxalin-7-Carboxylsäure
42. 4-(4,7-Diaminoimidazo[1,2-a]chinoxalin-1-yl)benzol-1,2-diol
43. 4-(7-Amino-4-(methylamino)imidazo[1,2-a]chinoxalin-1-yl)benzol-1,2-diol
44. 4-(7-Amino-4-((2-aminoethyl)amino)imidazo[1,2-a]chinoxalin-1-yl)benzol-1,2-diol
45. 4-(7-Amino-4-((3-aminopropyl)amino)imidazo[1,2-a]chinoxalin-1-yl)benzol -1,2-diol
46. 4-(7-Amino-4-((6-aminohexyl)amino)imidazo[1,2-a]chinoxalin-1-yl)benzol-1,2-diol
47. 4-(4-Amino-8-(trifluoromethyl)imidazo[1,2-a]chinoxalin-1-yl)benzol-1,2-diol
48. 4-(4-(Methylamino)-8-(trifluoromethyl)imidazo[1,2-a]chinoxalin-1-yl)benzol-1,2-diol
49. 4-(4-((2-Aminoethyl)amino)-8-(trifluoromethyl)imidazo[1,2-a]chinoxalin-1-yl)benzol-1,2-diol
50. 4-(4-((3-Aminopropyl)amino)-8-(trifluoromethyl)imidazo[1,2-a]chinoxalin-1-yl)benzol-1,2-diol
51. 4-(4-((6-Aminohexyl)amino)-8-(trifluoromethyl)imidazo[1,2-a]chinoxalin-1-yl)benzol-1,2-diol
52. 4-Amino-1-(3,4-dihydroxyphenyl)imidazo[1,2-a]chinoxalin-8-carbonitril
53. 1-(3,4-Dihydroxyphenyl)-4-(methylamino)imidazo[1,2-a]chinoxalin-8-carbonitril
54. 4-((2-Aminoethyl)amino)-1-(3,4-dihydroxyphenyl)imidazo[1,2-a]chinoxalin-8-carbonitril
55. 4-((3-Aminopropyl)amino)-1-(3,4-dihydroxyphenyl)imidazo[1,2-a]chinoxalin-8-carbonitril
56. 4-((6-Aminohexyl)amino)-1-(3,4-dihydroxyphenyl)imidazo[1,2-a]chinoxalin-8-carbonitril
57. 4-Amino-1-(3,4-dihydroxyphenyl)imidazo[1,2-a]chinoxalin-8-Methylcarboxylat
58. 1-(3,4-Dihydroxyphenyl)-4-(methylamino)imidazo[1,2-a]chinoxalin-8-Methylcarboxylat
59. 4-((2-Aminoethyl)amino)-1-(3,4-dihydroxyphenyl)imidazo[1,2-a]chinoxalin-8-Methylcarboxylat
60. 4-((3-Aminopropyl)amino)-1-(3,4-dihydroxyphenyl)imidazo[1,2-a]chinoxalin-8-Methylcarboxylat
61. 4-((6-Aminohexyl)amino)-1-(3,4-dihydroxyphenyl)imidazo[1,2-a]chinoxalin-8-Methylcarboxylat
62. 4-Amino-1-(3,4-dihydroxyphenyl)imidazo[1,2-a]chinoxalin-8-Carboxylsäure
63. 1-(3,4-Dihydroxyphenyl)-4-(methylamino)imidazo[1,2-a]chinoxalin-8-Carboxylsäure
64. 4-((2-Aminoethyl)amino)-1-(3,4-dihydroxyphenyl)imidazo[1,2-a]chinoxalin-8-Carboxylsäure
65. 4-((3-Aminopropyl)amino)-1-(3,4-dihydroxyphenyl)imidazo[1,2-a]chinoxalin-8-Carboxylsäure
66. 4-((6-Aminohexyl)amino)-1-(3,4-dihydroxyphenyl)imidazo[1,2-a]chinoxalin-8-Carboxylsäure
67. 4-(4,8-Diaminoimidazo[1,2-a]chinoxalin-1-yl)benzol-1,2-diol
68. 4-(8-Amino-4-(methylamino)imidazo[1,2-a]chinoxalin-1-yl)benzol-1,2-diol
69. 4-(8-Amino-4-((2-aminoethyl)amino)imidazo[1,2-a]chinoxalin-1-yl)benzol-1,2-diol
70. 4-(8-Amino-4-((3-aminopropyl)amino)imidazo[1,2-a]chinoxalin-1-yl)benzol-1,2-diol
71. 4-(8-Amino-4-((6-aminohexyl)amino)imidazo[1,2-a]chinoxalin-1-yl)benzol-1,2-diol
72. 4-(4-((2-Aminoethyl)amino)imidazo[1,2-a]chinoxalin-1-yl)benzol-1,2-diol
73. 2-((1-(3,4-Dihydroxyphenyl)imidazo[1,2-a]chinoxalin-4-yl)amino)ethylammoniumchlorid
74. 4-(4-((6-Aminohexyl)amino)imidazo[1,2-a]chinoxalin-1-yl)benzol-1,2-diol
75. 6-((1-(3,4-Dihydroxyphenyl)imidazo[1,2-a]chinoxalin-4-yl)amino)hexan-1-ammoniumchlorid
76. 4-(4-(Piperazin-1-yl)imidazo[1,2-a]chinoxalin-1-yl)benzol-1,2-diol
77. 4-(4-((3-Aminopropyl)amino)imidazo[1,2-a]chinoxalin-1-yl)benzol-1,2-diol
78. N-(1,3-Dihydroxy-2-(hydroxymethyl)propan-2-yl)-2-((1-(3,4-dihydroxyphenyl)imidazo[1,2-a]chinoxalin-4-yl)amino)acetamid
79. 4-(4-(Methylamino)-7-(trifluoromethoxy)imidazo[1,2-a]chinoxalin-1-yl)benzol-1,2-diol
80. (2S)-2-Amino-5-((1-(3,4-dihydroxyphenyl)imidazo[1,2-a]chinoxalin-4-yl)amino)valeriansäure.

7. Verbindung nach einem der Ansprüche 1 bis 6, **dadurch gekennzeichnet, dass**:
R₂ und R_{c} Wasserstoffatome sind;
R₃ eine Methylamino- oder Amingruppe ist; und
Rₐ und R_{b} Hydroxygruppen sind;
oder ein pharmazeutisch annehmbares Salz davon.

8. Verfahren zur Herstellung einer Verbindung nach einem der Ansprüche 1 bis 7, **dadurch gekennzeichnet, dass** das Verfahren umfasst:
- einen Schritt der Kopplung der Verbindung mit der Formel (V): 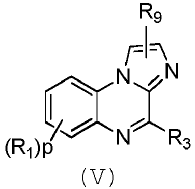
wobei R₁, R₃ und p nach einem der Ansprüche 1 bis 6 sind; und *
Rg ein Halogenatom ist;
mit einer geeigneten Arylboronsäure unter Suzuki-Bedingungen; und
- Rückgewinnung, Extraktion und/oder Reinigung der erhaltenen Verbindung nach einem Ansprüche 1 bis 7.

9. Pharmazeutische Zusammensetzung, die mindestens eine Verbindung nach einem der Ansprüche 1 bis 7 und wahlweise einen pharmazeutisch geeigneten Träger umfasst.

10. Verbindung nach einem der Ansprüche 1 bis 7 zur Verwendung als Medikament.

11. Verbindung nach einem der Ansprüche 1 bis 7 zur Verwendung bei der Behandlung von mindestens einem Krebs.

12. Verbindung zur Verwendung nach Anspruch 11, **dadurch gekennzeichnet, dass** der Krebs ein solider, flüssiger, gegebenenfalls metastatischer oder sekundärer Krebs ist.

13. Verbindung zur Verwendung nach Anspruch 11 oder 12, **dadurch gekennzeichnet, dass** der Krebs ein Melanom, ein Bauchspeicheldrüsen-, Dickdarm- oder Prostatakrebs, ein Lymphom, eine Leukämie oder ein Myelom ist.

14. Verbindung zur Verwendung nach Anspruch 11 bis 12, **dadurch gekennzeichnet, dass** der Krebs ein Brust-, Prostata- Lungen-, Speiseröhren-, Haut-, Blasen-, Magen-, Leber-, Gebärmutter-, Dickdarm- oder Mastdarmkrebs ist.

15. Verbindung zur Verwendung nach einem der Ansprüche 11 bis 12, **dadurch gekennzeichnet, dass** der Krebs ein Blutkrebs oder ein Krebs bestimmter Blutzellen, wie beispielsweise Lymphozyten oder Leukozyten, ist.

## Claims

1. A compound of the general formula (I): 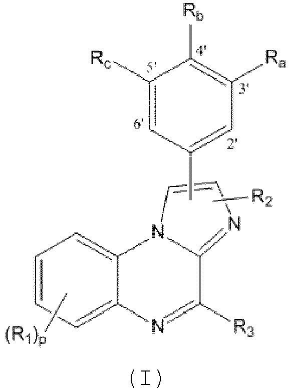 wherein:
- the various R₁ independently represent a hydrogen or halogen atom or a group selected from hydroxy, C₁-C₄ alkyl, C₁-C₄ alkoxy, amino, C₁-C₄ alkylamino, C₁-C₄ dialkylamino, cyano, CF₃, OCF₃, -(CH₂)ₙNR₄R₅, -NH-(CH₂)ₙNR₄R₅, -(CH₂)ₙCOR₄, -(CH₂)ₙCO-NR₄R₅, -(CH₂)ₙSO₂-NR₄R₅, and -(CH₂)ₙCO₂R₄ groups;
- R₂ represents a hydrogen atom or a group selected from hydroxy and C₁-C₂ alkyl groups, preferably a hydrogen atom;
- R₃ represents a halogen or a group selected from amino, C₁-C₃ alkylamino, C₁-C₃ dialkylamino, amine-(C₁-C₆ alkyl)-amino, N-(*tert*-butyloxycarbonyl)amino-(C₁-C₆ alkyl)-amino, piperidyl, N-(tert-butyloxycarbonyl)piperidyl groups, an amino group of an α-amino acid or an amino group of an α-amino acid side chain,
- Rₐ, R_{b} are hydroxy groups and R_{c} independently represents a hydrogen atom or a hydroxy group;
- n being 0, 1, 2, 3 or 4;
- p being 1, 2, 3 or 4;
- R₄ and R₅ independently represent a hydrogen atom or a C₁-C₄ alkyl group, preferably linear or branched;
or a pharmaceutically acceptable salt thereof.

2. The compound as claimed in claim 1, **characterized in that** it has the formula (II): 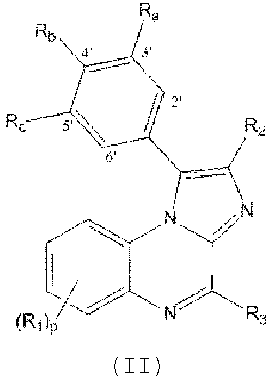
- wherein the various R₁, R₂, R₃, Rₐ, R_{b}, R_{c} and p groups are as defined in claim 1,
or a pharmaceutically acceptable salt thereof.

3. A compound as claimed in any one of claims 1 or 2 **characterized in that** it has the formula (III): 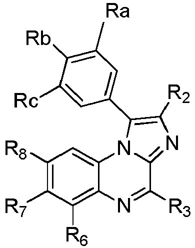 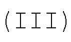
- wherein the various R₂, R₃, Rₐ, R_{b} and R_{c} groups are as defined in any one of the preceding claims,
- R₆, R₇ and R₈ independently represent a hydrogen or halogen atom or a group selected from hydroxy, C₁-C₄ alkyl, C₁-C₄ alkoxy, amino, C₁-C₄ alkylamino, C₁-C₄ dialkylamino, cyano, CF₃, OCF₃, -(CH₂)ₙNR₄R₅, -NH-(CH₂)ₙNR₄R₅, -(CH₂)ₙCOR₄, -(CH₂)ₙCO-NR₄R₅, -(CH₂)ₙSO₂-NR₄R₅, and -(CH₂)nCO₂R₄ groups;
- the various R₄ and R₅ independently represent a hydrogen atom or a C₁-C₄ alkyl group, preferably linear or branched; and
- n being 0, 1, 2, 3 or 4;
or a pharmaceutically acceptable salt thereof.

4. The compound as claimed in claim 3 **characterized in that**:
- R₆, R₇ and R₈ independently represent a hydrogen or halogen atom or a group selected from hydroxy, C₁-C₄ alkyl, C₁-C₄ alkoxy, OCF₃, preferably hydrogen, methyl, methoxy or OCF₃ groups;
or a pharmaceutically acceptable salt thereof.

5. The compound as claimed in any one of claims 1 to 4 **characterized in that** it has the formula (IV): 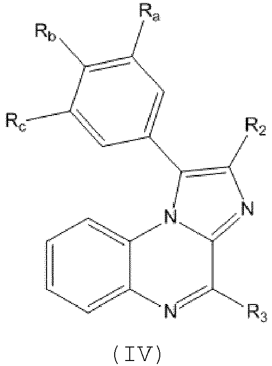
- wherein the various R₂, R₃, Rₐ, R_{b} and R_{c} groups are as defined in any one of the preceding claims;
or a pharmaceutically acceptable salt thereof.

6. The compound as claimed in any one of claims 1 to 6, **characterized in that** said compounds are selected from:
1. 1-(3,4-Dihydroxyphenyl)imidazo[1,2-a]quinoxalin-4-amine
2. 1-(3,4-Dihydroxyphenyl)-N-methylimidazo[1,2-a]quinoxalin-4-amine
3. 4-(4-Amino-6-(trifluoromethyl)imidazo[1,2-a]quinoxalin-1-yl)benzene-1,2-diol
4. 4-(4-(Methylamino)-6-(trifluoromethyl)imidazo[1,2-a]quinoxalin-1-yl)benzene-1,2-diol
5. 4-(4-((2-Aminoethyl)amino)-6-(trifluoromethyl)imidazo[1,2-a]quinoxalin-1-yl)benzene-1,2-diol
6. 4-(4-((3-Aminopropyl)amino)-6-(trifluoromethyl)imidazo[1,2-a]quinoxalin-1-yl)benzene-1,2-diol
7. 4-(4-((6-Aminohexyl)amino)-6-(trifluoromethyl)imidazo[1,2-a]quinoxalin-1-yl)benzene-1,2-diol
8. 4-Amino-1-(3,4-dihydroxyphenyl)imidazo[1,2-a]quinoxaline-6-carbonitrile
9. 1-(3,4-Dihydroxyphenyl)-4-(methylamino)imidazo[1,2-a]quinoxaline-6-carbonitrile
10. 4-((2-Aminoethyl)amino)-1-(3,4-dihydroxyphenyl)imidazo[1,2-a]quinoxaline-6-carbonitrile
11. 4-((3-Aminopropyl)amino)-1-(3,4-dihydroxyphenyl)imidazo[1,2-a]quinoxaline-6-carbonitrile
12. 4-((6-Aminohexyl)amino)-1-(3,4-dihydroxyphenyl)imidazo[1,2-a]quinoxaline-6-carbonitrile
13. Methyl 4-amino-1-(3,4-dihydroxyphenyl)imidazo[1,2-a]quinoxaline-6-carboxylate
14. Methyl 1-(3,4-dihydroxyphenyl)-4-(methylamino)imidazo[1,2-a]quinoxaline-6-carboxylate
15. Methyl 4-((3-aminopropyl)amino)-1-(3,4-dihydroxyphenyl)imidazo[1,2-a]quinoxaline-6-carboxylate
16. Methyl 4-((6-aminohexyl)amino)-1-(3,4-dihydroxyphenyl)imidazo[1,2-a]quinoxaline-6-carboxylate
17. 4-Amino-1-(3,4-dihydroxyphenyl)imidazo[1,2-a]quinoxaline-6-carboxylic acid
18. 1-(3,4-Dihydroxyphenyl)-4-(methylamino)imidazo[1,2-a]quinoxaline-6-carboxylic acid
19. 4-((2-Aminoethyl)amino)-1-(3,4-dihydroxyphenyl)imidazo[1,2-a]quinoxaline-6-carboxylic acid
20. 4-((3-Aminopropyl)amino)-1-(3,4-dihydroxyphenyl)imidazo[1,2-a]quinoxaline-6-carboxylic acid
21. 4-((6-Aminohexyl)amino)-1-(3,4-dihydroxyphenyl)imidazo[1,2-a]quinoxaline-6-carboxylic acid
22. 4-(4,6-Diaminoimidazo[1,2-a]quinoxalin-1-yl)benzene-1,2-diol
23. 4-(6-Amino-4-(methylamino)imidazo[1,2-a]quinoxalin-1-yl)benzene-1,2-diol
24. 4-(6-Amino-4-((2-aminoethyl)amino)imidazo[1,2-a]quinoxalin-1-yl)benzene-1,2-diol
25. 4-(6-Amino-4-((3-aminopropyl)amino)imidazo[1,2-a]quinoxalin-1-yl)benzene-1,2-diol
26. 4-(6-Amino-4-((6-aminohexyl)amino)imidazo[1,2-a]quinoxalin-1-yl)benzene-1,2-diol
27. 4-(4-Amino-7-(trifluoromethyl)imidazo[1,2-a]quinoxalin-1-yl)benzene-1,2-diol
28. 4-(4-(Methylamino)-7-(trifluoromethyl)imidazo[1,2-a]quinoxalin-1-yl)benzene-1,2-diol
29. 4-(4-((2-Aminoethyl)amino)-7-(trifluoromethyl)imidazo[1,2-a]quinoxalin-1-yl)benzene-1,2-diol
30. 4-(4-((3-Aminopropyl)amino)-7-(trifluoromethyl)imidazo[1,2-a]quinoxalin-1-yl)benzene-1,2-diol
31. 4-(4-((6-Aminohexyl)amino)-7-(trifluoromethyl)imidazo[1,2-a]quinoxalin-1-yl)benzene-1,2 diol
32. 4-Amino-1-(3,4-dihydroxyphenyl)imidazo[1,2-a]quinoxaline-7-carbonitrile
33. 1-(3,4-Dihydroxyphenyl)-4-(methylamino)imidazo[1,2-a]quinoxaline-7-carbonitrile
34. 4-((2-Aminoethyl)amino)-1-(3,4-dihydroxyphenyl)imidazo[1,2-a]quinoxaline-7-carbonitrile
35. 4-((3-Aminopropyl)amino)-1-(3,4-dihydroxyphenyl)imidazo[1,2-a]quinoxaline-7-carbonitrile
36. 4-((6-Aminohexyl)amino)-1-(3,4-dihydroxyphenyl)imidazo[1,2-a]quinoxaline-7-carbonitrile
37. 4-Amino-1-(3,4-dihydroxyphenyl)imidazo[1,2-a]quinoxaline-7-carboxylic acid
38. 1-(3,4-Dihydroxyphenyl)-4-(methylamino)imidazo[1,2-a]quinoxaline-7-carboxylic acid
39. 4-((2-Aminoethyl)amino)-1-(3,4-dihydroxyphenyl)imidazo[1,2-a]quinoxaline-7-carboxylic acid
40. 4-((3-Amlnopropyl)amino)-1-(3,4-dihydroxyphenyl)imidazo[1,2-a]quinoxaline-7-carboxylic acid
41. 4-((6-Aminohexyl)amino)-1-(3,4-dihydroxyphenyl)imidazo[1,2-a]quinoxaline-7-carboxylic acid
42. 4-(4,7-Diaminoimidazo[1,2-a]quinoxalin-1-yl)benzene-1,2-diol
43. 4-(7-Amino-4-(methylamino)imidazo[1,2-a]quinoxalin-1-yl)benzene-1,2-diol
44. 4-(7-Amino-4-((2-aminoethyl)amino)imidazo[1,2-a]quinoxalin-1-yl)benzene-1,2-diol
45. 4-(7-Amino-4-((3-aminopropyl)amino)imidazo[1,2-a]quinoxalin-1-yl)benzene-1,2-diol
46. 4-(7-Amino-4-((6-aminohexyl)amino)imidazo[1,2-a]quinoxalin-1-yl)benzene-1,2-diol
47. 4-(4-Amino-8-(trifluoromethyl)imidazo[1,2-a]quinoxalin-1-yl)benzene-1,2-diol
48. 4-(4-(Methylamino)-8-(trifluoromethyl)imidazo[1,2-a]quinoxalin-1-yl)benzene-1,2-diol
49. 4-(4-((2-Aminoethyl)amino)-8-(trifluoromethyl)imidazo[1,2-a]quinoxalin-1-yl)benzene-1,2-diol
50. 4-(4-((3-Aminopropyl)amino)-8-(trifluoromethyl)imidazo[1,2-a]quinoxalin-1-yl)benzene-1,2-diol
51. 4-(4-((6-Aminohexyl)amino)-8-(trifluoromethyl)imidazo[1,2-a]quinoxalin-1-yl)benzene-1,2-diol
52. 4-Amino-1-(3,4-dihydroxyphenyl)imidazo[1,2-a]quinoxaline-8-carbonitrile
53. 1-(3,4-Dihydroxyphenyl)-4-(methylamino)imidazo[1,2-a]quinoxaline-8-carbonitrile
54. 4-((2-Aminoethyl)amino)-1-(3,4-dihydroxyphenyl)imidazo[1,2-a]quinoxaline-8-carbonitrile
55. 4-((3-Aminopropyl)amino)-1-(3,4-dihydroxyphenyl)imidazo[1,2-a]quinoxaline-8-carbonitrile
56. 4-((6-Aminohexyl)amino)-1-(3,4-dihydroxyphenyl)imidazo[1,2-a]quinoxaline-8-carbonitrile
57. Methyl 4-amino-1-(3,4-dihydroxyphenyl)imidazo[1,2-a]quinoxaline-8-carboxylate
58. Methyl 1-(3,4-dihydroxyphenyl)-4-(methylamino)imidazo[1,2-a]quinoxaline-8-carboxylate
59. Methyl 4-((2-aminoethyl)amino)-1-(3,4-dihydroxyphenyl)imidazo[1,2-a]quinoxaline-8-carboxylate
60. Methyl 4-((3-aminopropyl)amino)-1-(3,4-dihydroxyphenyl)imidazo[1,2-a]quinoxaline-8-carboxylate
61. Methyl 4-((6-aminohexyl)amino)-1-(3,4-dihydroxyphenyl)imidazo[1,2-a]quinoxaline-8-carboxylate
62. 4-Amino-1-(3,4-dihydroxyphenyl)imidazo[1,2-a]quinoxaline-8-carboxylic acid
63. 1-(3,4-Dihydroxyphenyl)-4-(methylamino)imidazo[1,2-a]quinoxaline-8-carboxylic acid
64. 4-((2-Aminoethyl)amino)-1-(3,4-dihydroxyphenyl)imidazo[1,2-a]quinoxaline-8-carboxylic acid
65. 4-((3-Aminopropyl)amino)-1-(3,4-dihydroxyphenyl)imidazo[1,2-a]quinoxaline-8-carboxylic acid
66. 4-((6-Aminohexyl)amino)-1-(3,4-dihydroxyphenyl)imidazo[1,2-a]quinoxaline-8-carboxylic acid
67. 4-(4,8-Diaminoimidazo[1,2-a]quinoxalin-1-yl)benzene-1,2-diol
68. 4-(8-Amino-4-(methylamino)imidazo[1,2-a]quinoxalin-1-yl)benzene-1,2-diol
69. 4-(8-Amino-4-((2-aminoethyl)amino)imidazo[1,2-a]quinoxalin-1-yl)benzene-1,2-diol
70. 4-(8-Amino-4-((3-aminopropyl)amino)imidazo[1,2-a]quinoxalin-1-yl)benzene-1,2-diol
71. 4-(8-Amino-4-((6-aminohexyl)amino)imidazo[1,2-a]quinoxalin-1-yl)benzene-1,2-diol
72. 4-(4-((2-Aminoethyl)amino)imidazo[1,2-a]quinoxalin-1-yl)benzene-1,2-diol
73. 2-((1-(3,4-Dihydroxyphenyl)imidazo[1,2-a]quinoxalin-4-yl)amino)ethylammonium chloride
74. 4-(4-((6-Aminohexyl)amino)imidazo[1,2-a]quinoxalin-1-yl)benzene-1,2-diol
75. 6-((1-(3,4-Dihydroxyphenyl)imidazo[1,2-a]quinoxalin-4-yl)amino)hexan-1-ammonium chloride
76. 4-(4-Piperazin-1-yl)imidazo[1,2-a]quinoxalin-1-yl)benzene-1,2-diol
77. 4-(4-((3-Aminopropyl)amino)imidazo[1,2-a]quinoxalin-1-yl)benzene-1,2-diol
78. N-(1,3-Dihydroxy-2-(hydroxymethyl)propan-2-yl)-2-((1-(3,4-dihydroxyphenyl)imidazo[1,2-a]quinoxalin-4-yl)amino)acetamide
79. 4-(4-(Methylamino)-7-(trifluoromethoxy)imidazo[1,2-a]quinoxalin-1-yl)benzene-1,2-diol
80. (2S)-2-Amino-5-((1-(3,4-dihydroxyphenyl)imidazo[1,2-a]quinoxalin-4-yl)amino)pentanoic acid.

7. The compound as claimed in any one of claims 1 to 6 **characterized in that**:
R₂ and R_{c} are hydrogen atoms;
R₃ is a methylamino or amine group; and
Rₐ and R_{b} are hydroxy groups;
or a pharmaceutically acceptable salt thereof.

8. Method for manufacturing a compound as claimed in any one of claims 1 to 7 **characterized in that** said method comprises:
- a step of coupling the compound of the formula (V) : 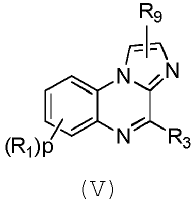
wherein R₁, R₃ and p are as defined in any one of claims 1 to 6; and
R₉ is a halogen atom;
with a suitable arylboronic acid, under Suzuki conditions; and
- recovering, extracting and/or purifying the compound obtained according to any one of claims 1 to 7.

9. A pharmaceutical composition comprising at least one compound as claimed in any one of claims 1 to 7 and optionally a pharmaceutically acceptable carrier.

10. The compound as claimed in any one of claims 1 to 7 for use as a medicinal product.

11. The compound as claimed in any one of claims 1 to 7 for use in the treatment of at least one cancer.

12. The compound for use as claimed in claim 11 **characterized in that** the cancer is solid or liquid, optionally metastatic or secondary.

13. The compound for use as claimed in claim 11 or 12 **characterized in that** the cancer is a melanoma, a cancer of the pancreas, colon or prostate, a lymphoma, a leukemia or a myeloma.

14. The compound for use as claimed in any one of claims 11 to 12 **characterized in that** the cancer is cancer of the breast, prostate, lungs, esophagus, skin, bladder, stomach, liver, uterus, colon or rectum.

15. The compound for use as claimed in any one of claims 11 to 12 **characterized in that** the cancer is cancer of the blood or of certain blood cells such as lymphocytes or leukocytes.
